# EUROPEAN PATENT APPLICATION

(11) **EP 4 768 506 A1**
(43) Date of publication of application: **01.07.2026**
(21) Application number: 24855815.7
(22) Date of filing: 20.08.2024
(51) Int. Cl.: C07K 16/30, A61K 103/32, A61K 103/10, A61K 103/20, A61K 103/30, G01N 33/574, G01N 33/60, G01N 33/534, A61K 51/10, A61K 51/04, A61K 47/68, A61K 45/00, A61P 35/00

(54) **MOLECULAR PROBES TARGETING GPC3 AND USE THEREOF**

(30) Priority: 22.08.2023 CN 202311071351
(71) Applicant: Wuhan Raydif Biotechnology Co., Ltd., Wuhan, Hubei 430073 (CN); Jecho Institute, Co., Ltd., Shanghai 200241 (CN)
(72) Inventor: LAN, Xiaoli, Wuhan, Hubei 430022 (CN); GAI, Yongkang, Wuhan, Hubei 430022 (CN); PAN, Zhidi, Shanghai 200241 (CN); ZHANG, Xiao, Wuhan, Hubei 430022 (CN); ZHU, Jianwei Sean, Shanghai 200241 (CN); JIANG, Dawei, Wuhan, Hubei 430022 (CN); LIN, Zhaoguo, Wuhan, Hubei 430022 (CN)
(74) Representative: Huang, Liwei
(86) International application number: PCT/CN2024/113446
(87) International publication number: WO 2025/040093

(57) **Abstract**

The present invention relates to the field of molecular imaging, and more particularly, to a molecular probe targeting GPC3. The present invention sets forth a molecular probe targeting GPC3. The molecular probe is a compound having the following sequence, or a pharmaceutically acceptable salt or ester thereof: (GPC3 antibodies, comprising antibody fragments of GPC3 antibodies, or single domain antibodies thereof)m-Rn, wherein R is selected from a group consisting of a linking group, a chelating agent, a radionuclide, another targeting polypeptide or monoclonal antibody, a monoclonal antibody fragment, a single domain antibody, and a small molecule inhibitor, wherein n represents the number of R, n = 0-4, and m is not 0. The probe has good stability in vitro, clear imaging in tumors, and good specificity.

## Description

### TECHNICAL FIELD

The present invention relates to the field of molecular imaging, in particular to a molecular probe targeting GPC3. More particularly, it relates to a molecular probe including anti-GPC3 antibody or single-domain antibodies thereof.

### BACKGROUND

Glypican-3 (GPC3) is a heparan sulfate proteoglycan anchored to a cell surface via glycosylphosphatidylinositol (GPI). It is highly expressed in normal embryonic tissues (including liver and placenta), but is rarely or not expressed in normal adult tissues. GPC3 interacts with heparin-binding proteins such as extracellular matrix components, cell adhesion molecules, growth factors, enzymes, and enzyme inhibitors, and participates in the regulation of physiological processes such as cell differentiation, proliferation, adhesion, and migration. It was found by numerous studies that GPC3 is specifically and highly expressed in liver cancer tissues and is closely associated with the prognosis of liver cancer. GPC3 plays a crucial role in regulating malignant transformation and promoting the growth of liver cancer by stimulating the canonical Wnt signaling pathway. It was shown by genetic analysis of liver cancer that most cases are associated with up-regulation of GPC3 expression. Extensive data indicate that for liver cancer, GPC3-positive patients exhibit lower five-year survival rates compared to GPC3-negative patients. Therefore, GPC3 represents a promising and reliable biomarker for liver cancer, serving as a highly prospective target for precision diagnosis and treatment. In addition, it was found by further studies that GPC3 is also associated with a variety of cancers and is expressed in non-small cell lung cancer, melanoma, ovarian yolk sac tumors, ovarian clear cell carcinoma, and colorectal cancer. It was suggested that GPC3 may participate in various signaling pathways and play important biological roles in the growth of tumor cells.

Radioimmunoimaging (RII) is used for early tumor diagnosis through combining the high specificity of antigen-antibody recognition with the high sensitivity of radioactive detection by utilizing radionuclide-labeled monoclonal antibodies. RII provides a non-invasive approach for studding the distribution (localization), quantity (density), and function (affinity) of antigens *in vivo.* Compared to whole IgG antibodies, Fab fragments exhibit faster blood clearance and superior tumor tissue penetration and can significantly reduce non-specific binding caused by interactions with native Fc receptors, thereby improving image quality and enhancing detection efficiency at target sites. Furthermore, radio-immunotherapy (RIT) is used for specific internal irradiating tumor cells by utilizing monoclonal antibodies or fragments thereof labeled with therapeutic nuclides emitting alpha or beta particles. Compared to chemotherapy drugs, which caused severe toxic and side effects by indiscriminately killing normal tissue cells, and radiotherapy, which limits to the irradiated area only, radio-immunotherapy combines "systematicness" and "targeting" in the treatment, achieving rapid development in recent years. Currently, most RII and RIT related probes are mouse-derived antibodies or humanized human-mouse chimeric antibodies. However, during clinical transformation, their heterogeneity often triggers immune side effects in the body, creating barriers to clinical application. Fab fragments derived from fully human antibodies, produced by using antibody library technology, can effectively reduce the occurrence of immune side effects and offer higher safety, thereby enabling clinical transformation.

Therefore, developing non-invasive molecular probes based on Fab fragments of fully human antibodies targeting GPC3 for GPC3 tracing *in vivo* facilitates early, real-time dynamic detection and monitoring of tissues with high GPC3 expression, such as malignant tumors like hepatocellular carcinoma (HCC). This holds significant clinical value for diagnosis, staging, and efficacy assessment in diseases with high GPC3 expression.

Consequently, there remains a need in this field for a molecular probe targeting GPC3 with high specificity and superior pharmacokinetic performance *in vivo.* Such a probe could be utilized for the diagnosis and treatment of diseases characterized by high GPC3 expression, particularly solid tumors with high GPC3 expression.

### SUMMARY

In the first aspect, the present disclosure provides a molecular probe targeting GPC3. The molecular probe is a compound with following structure, or a pharmaceutically acceptable salt or ester thereof:
(anti-GPC3 antibody, including a fragment of the anti-GPC3 antibody, or a single-domain antibody thereof)ₘ-Rₙ,
where R is selected from a group consisting of a linking group, a chelating agent, a radionuclide, another targeting peptide or monoclonal antibody, a monoclonal antibody fragment, a single-domain antibody, and a small-molecule inhibitor;
n represents a number of R, and n=0-4;
m is not 0.

In some embodiments, the molecular probe is a compound with a structure represented by following general formula (1), or a pharmaceutically acceptable salt or ester thereof:

Abₘ-(L-Mz)ₙ (I)

where Ab is the GPC3 anti-antibody or an antigen binding fragment thereof; L is the linking group. M is the radionuclide; z is 0 or 1; n is an integer from 1 to 4, preferably 1 or 2; and m is an integer from 1 to 4, preferably 1 or 2.

In some embodiments, L is represented by following general formula (II):

Y-Lk-T (II)

where Y is a linker, Lk is a coupling group, T is a chelating moiety;
preferably, Y includes a reactive moiety and a spacer group, in which the spacer group is independently selected from a single bond, an optionally substituted C₁-C₂₀ alkylene group, an optionally substituted C₁-C₂₀ alkenylene group, an optionally substituted C₁-C₂₀ alkynylene group, an optionally substituted C₁-C₂₀ heteroalkylene group, an optionally substituted C₃-C₂₀ cycloalkylene group, an optionally substituted C₃-C₂₀ heterocycloalkylene group, an optionally substituted C₅-C₂₀ arylene group, an optionally substituted C₅-C₂₀ heteroarylene group, ketone group, -S-, -(C=S)-, -O-, -NH-, -NHCO-, polypeptides (e.g., valine-citrulline (Val-Cit), valine-alanine (Val-Ala), glycine-glycine-phenylalanine-glycine (Gly-Gly-Phe-Gly), phenylalanine-lysine (Phe-Lys), alanine-alanine-asparagine (Ala-Ala-Asn), polyglycine (Gly)ᵣ, r is an integer from 1 to 10), polyethylene glycol (such as (PEG)ᵣ, in which r is an integer from 1 to 10), polypropylene glycol, polyoxyalkylene, copolymerization groups of polyethylene glycol and polypropylene glycol, or a combination thereof; the reactive moiety is linked to Ab and is independently selected from a single bond, p-isothiocyanate phenyl, N-hydroxysuccinimide ester, maleimidyl, 2,3,5,6-tetrafluorophenol ester, N-succinimidyl-S-acetylthioacetate, amido group, or a combination thereof;
preferably, Lk is independently selected from a single bond, p-isothiocyanate phenyl, N-hydroxysuccinimide ester, maleimidyl, 2,3,5,6-tetrafluorophenol ester, N-succinimidyl-S-acetylthioacetate, amido group, or a combination thereof;
preferably, T is independently selected from a single bond or a ligand derived from any of following structures: deferoxamine (DFO), 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), 6-hydrazinonicotinic acid (HYNIC), 4-[[[(1R,2R)-2-[bis(carboxymethyl)amino]cyclohexyl] (carboxymethyl)amino]methyl]-phenylacetic acid (H3RESCA), ethylenediaminetetraacetic acid (EDTA), 1,4,7,10-tetraazacyclododecane-1,4,7,10-tetrakis (methylene phosphonic acid) (DOTP), 1,4,7- triazacyclononane-1,4,7-tris (methylene phosphonic acid) (NOTP), 1,4,8,11-tetraazacyclotetradecane-1,4,8,11-tetraacetic acid (TETA), 1,4,7,10-tetrakis(carbamoylmethyl)-1,4,7,10-tetraazacyclododecane (DOTAM), triacetylfusarine C (TAFC), ferrochloroquine E (FOXE), ferrochloroquine B (FOXB), Ferrichrome A (FCHA), or a combination thereof.
optionally, a spacer group is further included between Lk and T, in which the spacer group is independently selected from a single bond, an optionally substituted C₁-C₂₀ alkylene group, an optionally substituted C₁-C₂₀ alkenylene group, an optionally substituted C₁-C₂₀ alkynylene group, an optionally substituted C₁-C₂₀ heteroalkylene group, an optionally substituted C₃-C₂₀ cycloalkylene group, an optionally substituted C₃-C₂₀ heterocycloalkylene group, an optionally substituted C₅-C₂₀ arylene group, an optionally substituted C₅-C₂₀ heteroarylene group, ketone group, -S-, -(C=S)-, -O-, -NH-, -NHCO-, polypeptides (e.g., valine-citrulline (Val-Cit), valine-alanine (Val-Ala), glycine-glycine-phenylalanine-glycine (Gly-Gly-Phe-Gly), phenylalanine-lysine (Phe-Lys), alanine-alanine-asparagine (Ala-Ala-Asn), polyglycine (Gly)ᵣ, r is an integer from 1 to 10), polyethylene glycol ((PEG)ᵣ, in which r is an integer from 1 to 10), polypropylene glycol, polyoxyalkylene, copolymerization groups of polyethylene glycol and polypropylene glycol, or a combination thereof.

In some embodiments, Y includes a reactive moiety and a spacer group, in which the spacer group is independently selected from a single bond, an optionally substituted C₁-C₁₀ alkylene group, an optionally substituted C₁-C₁₀ alkenylene group, an optionally substituted C₁-C₁₀ alkynylene group, an optionally substituted C₁-C₁₀ heteroalkylene group, an optionally substituted C₃-C₁₀ cycloalkylene group, an optionally substituted C₃-C₁₀ heterocycloalkylene group, an optionally substituted C₃-C₁₀ arylene group, an optionally substituted C₃-C₁₀ heteroarylene group, ketone group, -S-, -(C=S)-, -O-, -NH-, -NHCO-, -(PEG)ᵣ-, valine-citrulline (Val-Cit), valine-alanine (Val-Ala), glycine-glycine-phenylalanine-glycine (Gly-Gly-Phe-Gly), phenylalanine-lysine (Phe-Lys), alanine-alanine-asparagine (Ala-Ala-Asn), -(Gly)ₙ-, or a combination thereof, in which n is an integer from 1 to 10; the reactive moiety is linked to Ab and is independently selected from a single bond, p-isothiocyanate phenyl, maleimidyl, amido group, or a combination thereof;
Lk is independently selected from a single bond, p-isothiocyanate phenyl, amido group or maleimidyl, and
T is independently selected from a single bond or a ligand derived from any of following structures: deferoxamine (DFO), 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), 6-hydrazinonicotinic acid (HYNIC), 4-[[[(1R,2R)-2-[bis(carboxymethyl)amino]cyclohexyl] (carboxymethyl)amino]methyl]-phenylacetic acid (H3RESCA), 1,4,7,10-tetraazacyclododecane-1,4,7,10-tetrakis (methylene phosphonic acid) (DOTP), or 1,4,7- triazacyclononane-1,4,7-tris (methylene phosphonic acid) (NOTP);
optionally, a spacer group is further included between Lk and T, in which the spacer group is independently selected from a single bond, an optionally substituted C₁-C₁₀ alkylene group, an optionally substituted C₁-C₁₀ alkenylene group, an optionally substituted C₁-C₁₀ alkynylene group, an optionally substituted C₁-C₁₀ heteroalkylene group, an optionally substituted C₃-C₁₀ cycloalkylene group, an optionally substituted C₃-C₁₀ heterocycloalkylene group, an optionally substituted C₃-C₁₀ arylene group, an optionally substituted C₃-C₁₀ heteroarylene group, ketone group, -S-, -(C=S)-, -O-, -NH-, -NHCO-, -(PEG)ᵣ-, valine-citrulline (Val-Cit), valine-alanine (Val-Ala), glycine-glycine-phenylalanine-glycine (Gly-Gly-Phe-Gly), phenylalanine-lysine (Phe-Lys), alanine-alanine-asparagine (Ala-Ala-Asn), -(Gly)n, or a combination thereof, in which n is an integer from 1 to 10.

In some embodiments, Y includes a reactive moiety and a spacer group, -(PEG)ᵣ-, -(Gly)ᵣ-, or a combination thereof, in which r is an integer from 1 to 10; the reactive moiety is linked to Ab and is independently selected from a single bond, p-isothiocyanate phenyl, maleimidyl, amido group, or a combination thereof;
Lk is independently selected from a single bond, p-isothiocyanate phenyl, or amido group, and
T is independently selected from a single bond or a ligand derived from any of following structures: 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), 6-hydrazinonicotinic acid (HYNIC), or 4-[[[(1R,2R)-2-[bis(carboxymethyl)amino]cyclohexyl] (carboxymethyl)amino]methyl]-phenylacetic acid (H3RESCA);
optionally, a spacer group is further included between Lk and T, in which the spacer group is independently selected from a single bond, -(PEG)ᵣ-, -(Gly)ᵣ-, or a combination thereof, in which r is an integer from 1 to 10.

In some embodiments, Lk is independently selected from p-isothiocyanate phenyl, or amido group, Y is a single bond, and T is independently selected from a ligand derived from any of following structures: 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), 6-hydrazinonicotinic acid (HYNIC), or 4-[[[(1R,2R)-2-[bis(carboxymethyl)amino]cyclohexyl] (carboxymethyl)amino]methyl]-phenylacetic acid (H3RESCA).

In some embodiments, Lk is p-isothiocyanate phenyl, or amido group, Y includes a reactive moiety and a spacer group, in which the spacer group is independently selected from a single bond, -(PEG)ᵣ-, -(Gly)ᵣ-, or a combination thereof, in which r is an integer from 1 to 10; the reactive moiety is linked to Ab and is independently selected from a single bond, p-isothiocyanate phenyl, maleimidyl, amido group, or a combination thereof; and T is independently selected from a ligand derived from any of following structures: 1,4,7,10-tetraazacyclododecane-1,4,7,10-tetracarboxylic acid (DOTA), 1,4,7-triazacyclononane-1,4,7-tricarboxylic acid (NOTA), or diethylenetriamine pentaacetic acid (DTPA).

In some embodiments, Lk is p-isothiocyanate phenyl, Y is a single bond, and T is independently selected from a ligand derived from any of following structures: 1,4,7,10-tetraazacyclododecane-1,4,7,10-tetracarboxylic acid (DOTA), 1,4,7-triazacyclononane-1,4,7-tricarboxylic acid (NOTA), or diethylenetriamine pentaacetic acid (DTPA).

In some embodiments, Lk is amido group, Y includes a reactive moiety and a spacer group, in which the spacer group is independently selected from a single bond, -(PEG)ᵣ-, -(Gly)ᵣ-, or a combination thereof, in which r is an integer from 1 to 10; the reactive moiety is linked to Ab and is independently selected from a single bond, p-isothiocyanate phenyl, maleimidyl, amido group, or a combination thereof; and T is independently selected from a ligand derived from any of following structures: mercaptoacetyl triglycine (MAG3), 6-hydrazinonicotinic acid (HYNIC), or 4-[[[(1R,2R)-2-[bis(carboxymethyl)amino]cyclohexyl] (carboxymethyl)amino]methyl]-phenylacetic acid (H3RESCA).

In some embodiments, Lk is amido group, Y is a single bond; and T is independently selected from a ligand derived from any of following structures: mercaptoacetyl triglycine (MAG3), 6-hydrazinonicotinic acid (HYNIC), or 4-[[[(1R,2R)-2-[bis(carboxymethyl)amino]cyclohexyl] (carboxymethyl)amino]methyl]-phenylacetic acid (H3RESCA).

In some embodiments, Lk is amido group, Y is a single bond; and T is independently selected from a ligand derived from any of following structures: 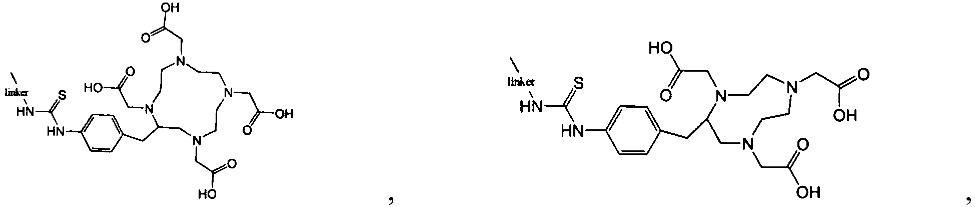 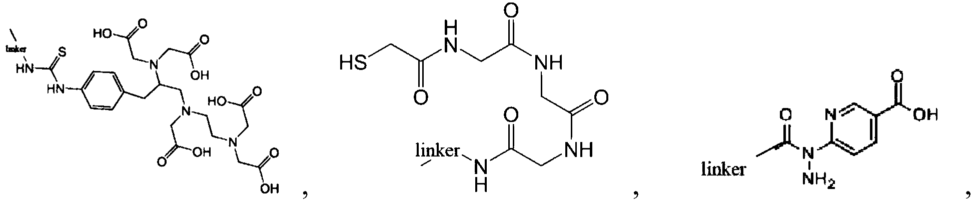 or 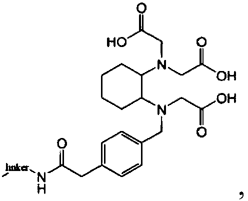 where, the linker includes a reactive moiety and a spacer group, in which the spacer group is independently selected from a single bond, -(PEG)ᵣ-, -(Gly)ᵣ-, or a combination thereof, in which r is an integer from 1 to 10; the reactive moiety is linked to Ab and is independently selected from a single bond, p-isothiocyanate phenyl, maleimidyl, amido group, or a combination thereof. Preferably, the linker is a single bond.

In some embodiments, M is a nuclide that emits β or α particles, a positron-emitting nuclide, a single-photon-emitting nuclide, or a paramagnetic metal. Preferably, M is selected from a diagnostic radionuclide or a therapeutic radionuclide.

Preferably, the diagnostic radionuclide is selected from ⁶⁸Ga, ¹⁸F-Al, ⁶⁴Cu, ^{99m}Tc, ⁸⁹Zr, ⁴⁴Sc, ¹¹¹In, ⁴⁵Ti, ⁵²Mn, ⁵⁹Fe, ^{94m}Tc, ⁶⁷Ga, ⁷¹As, ⁷²As, ^{82m}Rb, ⁸⁶Y, ¹³¹I, Gd, Mn, or a combination thereof.

Preferably, the therapeutic radionuclide is selected from ¹⁷⁷Lu, ⁹⁰Y, ¹⁵³Sm, ⁶⁷Cu, ⁸⁹Sr, ¹⁶⁶Ho, ¹⁷⁷Yb, ⁴⁷Sc, ¹⁸⁶Re, ¹⁸⁸Re, ²¹²Bi, ²¹³Bi, ¹⁴⁹Pm, ²¹²Pb, ²¹¹At, ²²³Ra, ²²⁵Ac, ²²⁷Th, or a combination thereof.

More preferably, M is selected from ⁶⁸Ga, ¹⁸F-Al, ^{99m}Tc, ¹⁷⁷Lu, ¹³¹I, or a combination thereof.

In some embodiments, L-M is selected from the following structures: 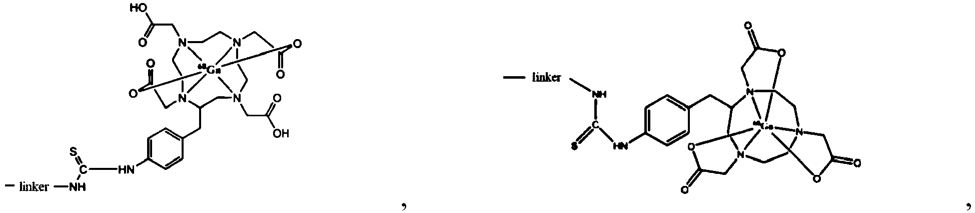 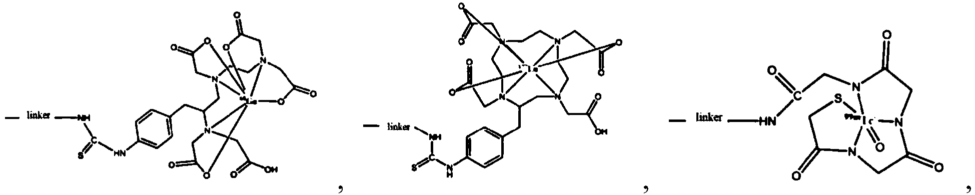 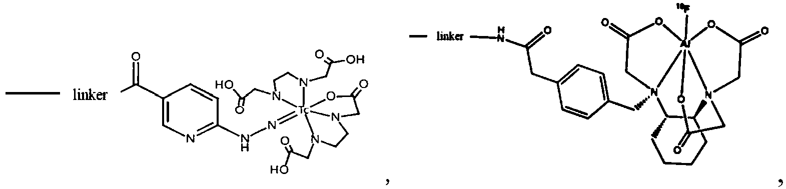 or -linker-¹³¹I,
where the linker includes a reactive moiety and a spacer group, in which the spacer group is independently selected from a single bond, -(PEG)ᵣ-, -(Gly)ᵣ-, or a combination thereof, in which r is an integer from 1 to 10; the reactive moiety is linked to Ab and is independently selected from a single bond, p-isothiocyanate phenyl, maleimidyl, amido group, or a combination thereof. Preferably, the linker is a single bond.

In some embodiments, Ab is anti-GPC3 antibody or antigen binding fragment thereof. Preferably, Ab is a monoclonal antibody, polyclonal antibody, single-chain antibody, chimeric antibody, humanized antibody, or fully human antibody. Preferably, Ab is selected from a monoclonal antibody (mAb), Fab, Fab', F(ab')₂, Fv, scFv, scFv-Fc, or single-domain antibody.

In some embodiments, Ab contains a heavy chain or a fragment thereof, while the heavy chain or fragment thereof contains HCDR1, HCDR2, and HCDR3.

HCDR1 contains an amino acid sequence having at least 80% sequence identity to the amino acid sequence shown as SEQ ID NO: 1. Preferably the amino acid sequence of HCDR1 is an amino acid sequence having at least 90% sequence identity to the amino acid sequence shown as SEQ ID NO: 1, more preferably at least 95% sequence identity to the amino acid sequence shown as SEQ ID NO: 1, most preferably the amino acid sequence of HCDR1 is shown as SEQ ID NO: 1.

HCDR2 contains an amino acid sequence having at least 80% sequence identity to the amino acid sequence shown as SEQ ID NO: 2. Preferably the amino acid sequence of HCDR2 is an amino acid sequence having at least 90% sequence identity to the amino acid sequence shown as SEQ ID NO: 2, more preferably at least 95% sequence identity to the amino acid sequence shown as SEQ ID NO: 2, most preferably the amino acid sequence of HCDR2 is shown as SEQ ID NO: 2.

HCDR3 contains an amino acid sequence having at least 80% sequence identity to the amino acid sequence shown as SEQ ID NO: 3. Preferably the amino acid sequence of HCDR3 is an amino acid sequence having at least 90% sequence identity to the amino acid sequence shown as SEQ ID NO: 3, more preferably at least 95% sequence identity to the amino acid sequence shown as SEQ ID NO: 3, most preferably the amino acid sequence of HCDR3 is shown as SEQ ID NO: 3.

In some embodiments, Ab contains a heavy chain variable region (VH), and the VH contains an amino acid sequence having at least 80%, preferably at least 90%, more preferably at least 95% sequence identity to the amino acid sequence shown as SEQ ID NO: 7, most preferably the amino acid sequence of VH is shown as SEQ ID NO: 7.

In some embodiments, Ab contains a heavy chain, and the heavy chain contains an amino acid sequence having at least 80%, preferably at least 90%, more preferably at least 95% sequence identity to the amino acid sequence shown as SEQ ID NO: 9. Most preferably the amino acid sequence of the heavy chain is shown as SEQ ID NO: 9.

In some embodiments, Ab contains a light chain or a fragment thereof, while the light chain or fragment thereof contains LCDR1, LCDR2, and LCDR3.

LCDR1 contains an amino acid sequence having at least 80% sequence identity to the amino acid sequence shown as SEQ ID NO: 4. Preferably the amino acid sequence of LCDR1 is an amino acid sequence having at least 90% sequence identity to the amino acid sequence shown as SEQ ID NO: 4, more preferably at least 95% sequence identity to the amino acid sequence shown as SEQ ID NO: 4, most preferably the amino acid sequence of LCDR1 is shown as SEQ ID NO: 4.

LCDR2 contains an amino acid sequence having at least 80% sequence identity to the amino acid sequence shown as SEQ ID NO: 5. Preferably the amino acid sequence of LCDR2 is an amino acid sequence having at least 90% sequence identity to the amino acid sequence shown as SEQ ID NO: 5, more preferably at least 95% sequence identity to the amino acid sequence shown as SEQ ID NO: 5, most preferably the amino acid sequence of LCDR2 is shown as SEQ ID NO: 5.

LCDR3 contains an amino acid sequence having at least 80% sequence identity to the amino acid sequence shown as SEQ ID NO: 6. Preferably the amino acid sequence of LCDR3 is an amino acid sequence having at least 90% sequence identity to the amino acid sequence shown as SEQ ID NO: 6, more preferably at least 95% sequence identity to the amino acid sequence shown as SEQ ID NO: 6, most preferably the amino acid sequence of LCDR3 is shown as SEQ ID NO: 6.

In some embodiments, Ab contains a light chain variable region (VL), while the VL contains an amino acid sequence having at least 80%, preferably at least 90%, more preferably at least 95% sequence identity to the amino acid sequence shown as SEQ ID NO: 8. Most preferably, the amino acid sequence of VL is shown as SEQ ID NO: 8.

In some embodiments, Ab contains a light chain, while the light chain contains an amino acid sequence having at least 80%, preferably at least 90%, more preferably at least 95% sequence identity to the amino acid sequence shown as SEQ ID NO: 10. Most preferably, the amino acid sequence of the light chain is shown as SEQ ID NO: 10.

In some embodiments, Ab is Fab, Fab', or F(ab')₂ fragment, preferably Fab fragment.

Preferably, Ab contains a heavy chain with an amino acid sequence having at least 80%, preferably at least 90%, more preferably at least 95% to the amino acid sequence shown as SEQ ID NO: 11, most preferably the amino acid sequence shown as SEQ ID NO: 11; and
preferably, Ab contains a light chain with an amino acid sequence having at least 80%, preferably at least 90%, more preferably at least 95% to the amino acid sequence shown as SEQ ID NO: 10, most preferably the amino acid sequence shown as SEQ ID NO: 10.

In some embodiments, Ab is scFv fragment.

Preferably, Ab contains an amino acid sequence having at least 80%, preferably at least 90%, more preferably at least 95% sequence identity to the amino acid sequence shown as SEQ ID NO: 12. Most preferably, the amino acid sequence of Ab is shown as SEQ ID NO: 12.

In some embodiments, the molecular probe is selected from the following structures:

| | | |
|---|---|---|
| 1. ⁶⁸Ga-DOTA-aGPC3-mAb | 2. ⁶⁸Ga-DOTA-aGPC3-Fab | 3. ⁶⁸Ga-DOTA-aGPC3-scFv |
| 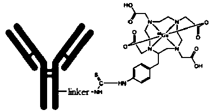 | 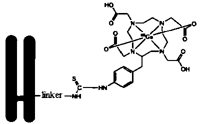 | 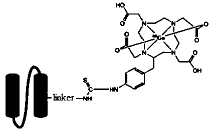 |
| 4. ⁶⁸Ga-NOTA-aGPC3-mAb | 5. ⁶⁸Ga-NOTA-aGPC3-Fab | 6. ⁶⁸Ga-NOTA-aGPC3-scFv |
| 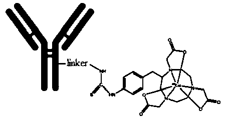 | 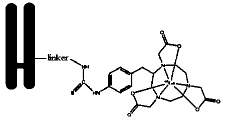 | 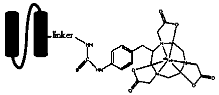 |
| 7. ⁶⁸Ga-DOTA-aGPC3-mAb | 8. ⁶⁸Ga-DOTA-aGPC3-Fab | 9. ⁶⁸Ga-DOTA-aGPC3-scFv |
| 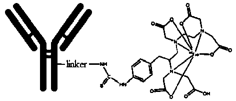 | 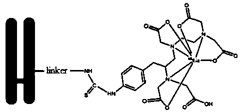 | 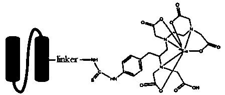 |
| 10. ¹⁷⁷Lu-DOTA-aGPC3-mAb | 11. ¹⁷⁷Lu-DOTA-aGPC3-Fab | 12. ¹⁷⁷Lu-DOTA-aGPC3-scFv |
| 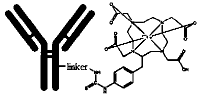 | 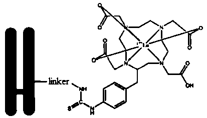 | 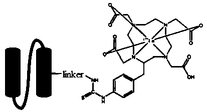 |
| 13. ^{99m}TC-MAG3-aGPC3-mAb | 14. ^{99m}TC-MAG3-aGPC3-Fab | 15. ^{99m}TC-MAG3-aGPC3-scFv |
| 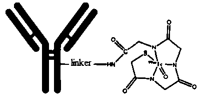 | 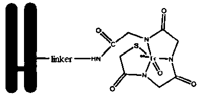 | 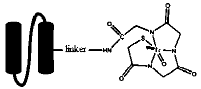 |
| 16. Al¹⁸F-RESCA-aGPC3-mAb | 17. Al¹⁸F-RESCA-aGPC3-Fab | 18. Al¹⁸F-RESCA-aGPC3-scFv |
| 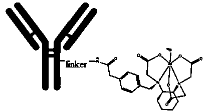 | 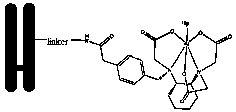 | 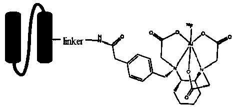 |
| 19. ¹³¹I-aGPC3-mAb | 20. ¹³¹I-aGPC3-Fab | 21. ¹³¹I-aGPC3-scFv |
| 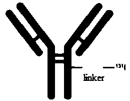 | 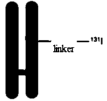 | 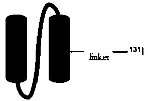 |
| 22. ^{99m}TC-HYNIC-aGPC3-mAb | 23. ^{99m}TC-HYNIC-aGPC3-Fab | 24. ^{99m}TC-HYNIC-aGPC3-scFv |
| 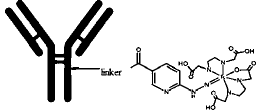 | 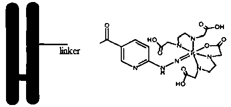 | 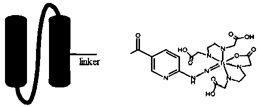 |

where the linker includes a reactive moiety and a spacer group, in which the spacer group is independently selected from a single bond, -(PEG)ᵣ-, -(Gly)ᵣ-, or a combination thereof, in which r is an integer from 1 to 10; the reactive moiety is linked to Ab and is independently selected from a single bond, p-isothiocyanate phenyl, maleimidyl, amido group, or a combination thereof. Preferably, the linker is a single bond.

In another aspect, the present disclosure provides an antibody conjugate having a structure represented by following general formula (III):

Abₘ-L'ₙ (III)

where L' is represented by following general formula (IV):

Y-Lk-T' (IV)

optionally, a spacer group is further included between Lk and T',
where Ab, Y, Lk, the spacer group, m, and n have the same definitions as previously defined, and T' is independently selected from a single bond, deferoxamine (DFO), 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), 6-hydrazinonicotinic acid (HYNIC), 4-[[[(1R,2R)-2-[bis(carboxymethyl)amino]cyclohexyl] (carboxymethyl)amino]methyl]-phenylacetic acid (H3RESCA), ethylenediaminetetraacetic acid (EDTA), 1,4,7,10-tetraazacyclododecane-1,4,7,10-tetrakis (methylene phosphonic acid) (DOTP), 1,4,7- triazacyclononane-1,4,7-tris (methylene phosphonic acid) (NOTP), 1,4,8,11-tetraazacyclotetradecane-1,4,8,11-tetraacetic acid (TETA), 1,4,7,10-tetrakis(carbamoylmethyl)-1,4,7,10-tetraazacyclododecane (DOTAM), triacetylfusarine C (TAFC), ferrochloroquine E (FOXE), ferrochloroquine B (FOXB), Ferrichrome A (FCHA), or a combination thereof. Preferably, T' is independently selected from a single bond, deferoxamine (DFO), 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), 6-hydrazinonicotinic acid (HYNIC), 4-[[[(1R,2R)-2-[bis(carboxymethyl)amino]cyclohexyl] (carboxymethyl)amino]methyl]-phenylacetic acid (H3RESCA), 1,4,7,10-tetraazacyclododecane-1,4,7,10-tetrakis (methylene phosphonic acid) (DOTP), or 1,4,7- triazacyclononane-1,4,7-tris (methylene phosphonic acid) (NOTP). More preferably, T' is independently selected from a single bond, 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), or 4-[[[(1R,2R)-2-[bis(carboxymethyl)amino]cyclohexyl] (carboxymethyl)amino]methyl]-phenylacetic acid (H3RESCA).

In another aspect, the present disclosure provides a pharmaceutical composition including the molecular probe or antibody conjugate mentioned above; and a pharmaceutically acceptable carrier.

In another aspect, the present disclosure provides a kit including the antibody conjugate mentioned above, and radionuclide M mentioned above.

In another aspect, the present disclosure provides a method for preparing the molecular probe targeting GPC3 mentioned above. The method includes reacting the antibody conjugate with radionuclide M.

In another aspect, the present disclosure provides use of the molecular probe targeting GPC3, antibody conjugate, pharmaceutical composition, or kit in the manufacture of a reagent for detecting of GPC3.

In another aspect, the present disclosure provides use of the molecular probe targeting GPC3, antibody conjugate, pharmaceutical composition, or kit in the manufacture of a kit for molecular imaging.

In another aspect, the present disclosure provides a method for imaging. The method includes: administering to a subject in need thereof the molecular probe targeting GPC3 or pharmaceutical composition; and detecting the presence of molecular probe targeting GPC3 *in vivo* through imaging.

In some embodiments, the presence of the molecular probe targeting GPC3 is detected through single photon emission computed tomography (SPECT), positron emission tomography (PET) imaging, or magnetic resonance imaging (MRI).

In another aspect, the present disclosure provides use of the molecular probe targeting GPC3, antibody conjugate, pharmaceutical composition, or kit in the manufacture of a kit for diagnosing cancer.

In another aspect, the present disclosure provides use of the molecular probe targeting GPC3, antibody conjugate, pharmaceutical composition, or kit in the manufacture of a reagent for treating cancer.

In another aspect, the present disclosure provides a method for treating cancer. The method includes: administering to a subject in need thereof the molecular probe targeting GPC3 or the pharmaceutical composition.

In some embodiments, the subject is GPC3-positive.

In some embodiments, the cancer is that with high GPC3 expression, preferably a solid tumor. More preferably, the cancer is selected from liver cancer, non-small cell lung cancer, melanoma, ovarian yolk sac tumor, ovarian clear cell carcinoma, and colorectal cancer, and most preferably liver cancer.

In some particular embodiments, the small molecule inhibitor may be a small molecule drug.

In some particular embodiments, the small molecule inhibitor may be a medicament for treating cancer.

In some particular embodiments, the small molecule inhibitor may be doxorubicin, cisplatin, vincristine, camptothecin, paclitaxel, etc.

In some particular embodiments, the small molecule inhibitor may be MK-0429, sorafenib, imatinib, gefitinib, etc.

The molecular probe targeting GPC3 in the present disclosure exhibits good stability *in vitro,* clear visualization within tumors, and excellent specificity.

### BRIEF DESCRIPTION OF THE DRAWINGS

FIG. 1 shows (A) SEC-HPLC profile of aGPC3-Fab antibody; (B) ITLC profile of ⁶⁸Ga-NOTA-aGPC3-Fab.
FIG. 2 shows ITLC profiles of ⁶⁸Ga-NOTA-aGPC3-scFv before and after PD-10 purification.
FIG. 3 shows ITLC profiles of ⁶⁸Ga-DOTA-aGPC3-scFv before and after PD-10 purification.
FIG. 4 shows ITLC profiles of ⁶⁸Ga-DOTA-aGPC3-Fab before and after PD-10 purification.
FIG. 5 shows (A) ITLC profile of ¹⁷⁷Lu-DOTA-aGPC3-Fab; (B) the radiochemical purity of ¹⁷⁷Lu-DOTA-aGPC3-Fab in PBS over time.
FIG. 6 shows ITLC profile of ¹⁷⁷Lu-DOTA-aGPC3-scFv.
FIG. 7 shows (A) ITLC profile of ^{I77}Lu-DOTA-aGPC3-mAb; (B) the radiochemical purity of ¹⁷⁷Lu-DOTA-aGPC3-mAb in PBS over time.
0FIG. 8 shows Western blot analysis of GPC3 expression in Huh7 and SK-HEP-1 cells (GAPDH as internal control).
FIG. 9 shows (A) PET/CT images of Huh7 tumor-bearing mouse at 30 min, 1 h, 2 h, and 4 h post-injection of ⁶⁸Ga-NOTA-aGPC3-Fab. (attenuation correction; fused images, coronal and transverse section images; arrows indicating Huh7 subcutaneous tumors); (B) ROI quantitative analysis of ⁶⁸Ga-NOTA-aGPC3-Fab uptake in blood, liver, kidney, muscle, and tumor from Huh7-bearing mouse; (C) Tumor/blood, tumor/liver, and tumor/muscle ratios at 30 min, 1 h, 2 h, and 4 h post-injection of ⁶⁸Ga-NOTA-aGPC3-Fab.
FIG. 10 shows (A) PET/CT images of SK-HEP-1 tumor-bearing mouse at 1 h, 2 h, and 4 h post-injection of ⁶⁸Ga-NOTA-aGPC3-Fab. (attenuation correction; fused images; white dotted circles indicating SK-HEP-1 subcutaneous tumor); (B) ROI quantitative analysis of ⁶⁸Ga-NOTA- aGPC3-Fab uptake in blood, liver, kidney, muscle, and tumor from SK-HEP-1-bearing mouse.
FIG. 11 shows (A) PET/CT images of PDX mouse model at 1 h, 2 h, and 4 h post-injection of ⁶⁸Ga-NOTA-aGPC3-Fab. (attenuation correction; fused images; white dotted circles indicating PDX transplant tumor); (B) ROI quantitative analysis of ⁶⁸Ga-NOTA-aGPC3-Fab uptake in blood, liver, kidney, muscle, and tumor from PDX mouse model.
FIG. 12 shows H&E staining and GPC3 immunohistochemical staining analysis of Huh7 and PDX tumor tissues.
FIG. 13 shows ⁶⁸Ga-NOTA-aGPC3-scFv PET/CT imaging of Hep3B tumor-bearing mouse. (A) Representative PET/CT images at different time points of the Hep3B subcutaneous tumor model; (B) Semi-quantitative analysis of mean PET signal intensity in blood pool, liver, kidney, muscle, and tumor regions of PET images from the Hep3B subcutaneous tumor model (n = 3); (C) Analysis of tumor/blood pool (T/B), tumor/liver (T/L), and tumor/muscle (T/M) ratios of Hep3B subcutaneous tumor model based on PET images (n = 3); white arrows indicating the tumor.

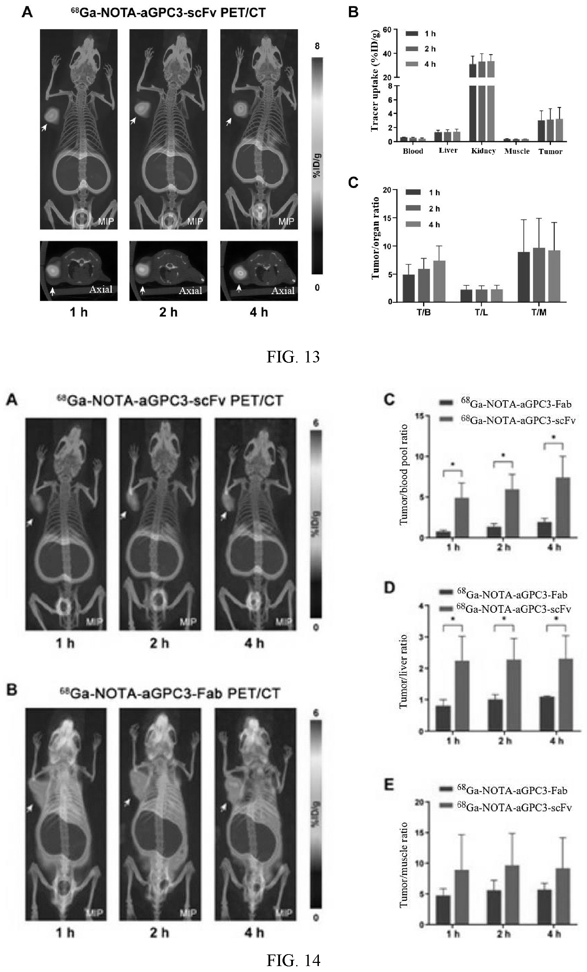

FIG. 14 shows a comparison of ⁶⁸Ga-NOTA-aGPC3-scFv PET/CT imaging and ⁶⁸Ga-NOTA-aGPC3-Fab PET/CT imaging of Hep3B tumor-bearing mouse. (A) Representative PET/CT images of Hep3B subcutaneous tumor model at 1 h, 2 h, and 4 h post-injection of ⁶⁸Ga-NOTA-aGPC3-scFv; (B) Representative PET/CT images of Hep3B subcutaneous tumor model at 1 h, 2 h, and 4 h post-injection of ⁶⁸Ga-NOTA-aGPC3-Fab; (C) Tumor/blood pool ratio analysis of Hep3B subcutaneous tumor model based on PET images (n = 3); (D) Tumor/liver ratio analysis of Hep3B subcutaneous tumor model based on PET images (n = 3); (E) Analysis of tumor/muscle ratios of Hep3B subcutaneous tumor model based on PET images (n = 3); white arrows indicating the tumor. *: P<0.05.
FIG. 15 shows a comparison of ⁶⁸Ga-NOTA-aGPC3-scFv, ⁶⁸Ga-NOTA-aGPC3-Fab, and ¹⁸F-FDG PET/CT PET/CT imaging of Hep3B liver orthotopic tumor mouse model. (A) Representative small animal PET static images at 1 h post-injection of ⁶⁸Ga-NOTA-aGPC3-scFv, ⁶⁸Ga-NOTA-aGPC3-Fab, and ¹⁸F-FDG of Hep3B liver orthotopic tumor mouse model. (B) Anatomical exploration for liver orthotopic tumor. Li: Liver; St: Stomach; Tu: Tumor; Sp: Spleen; SI: Small intestine; LI: Large intestine. White arrow (A) and yellow arrow (B) indicating tumor locations. Scale bar = 5 mm.
FIG. 16 shows (A) SPECT/CT images of Hep3B tumor-bearing mouse at 2 h, 6 h, 12 h, 24 h, and 48 h post-injection of ¹⁷⁷Lu-DOTA-aGPC3-Fab (white dotted circle indicating the tumor); (B) ROI quantitative analysis of ¹⁷⁷Lu-DOTA-aGPC3-Fab uptake in blood, liver, muscle, and tumor from Hep3B tumor-bearing mouse.
FIG. 17 shows (A) SPECT/CT images of Hep3B tumor-bearing mouse at 2 h, 6 h, 12 h, 24 h, and 48 h post-injection of ¹⁷⁷Lu-DOTA-aGPC3-scFv (white dotted circle indicating the tumor); (B) ROI semi-quantitative analysis of ¹⁷⁷Lu-DOTA-aGPC3-scFv uptake in blood, liver, muscle, and tumor from Hep3B tumor-bearing mouse.

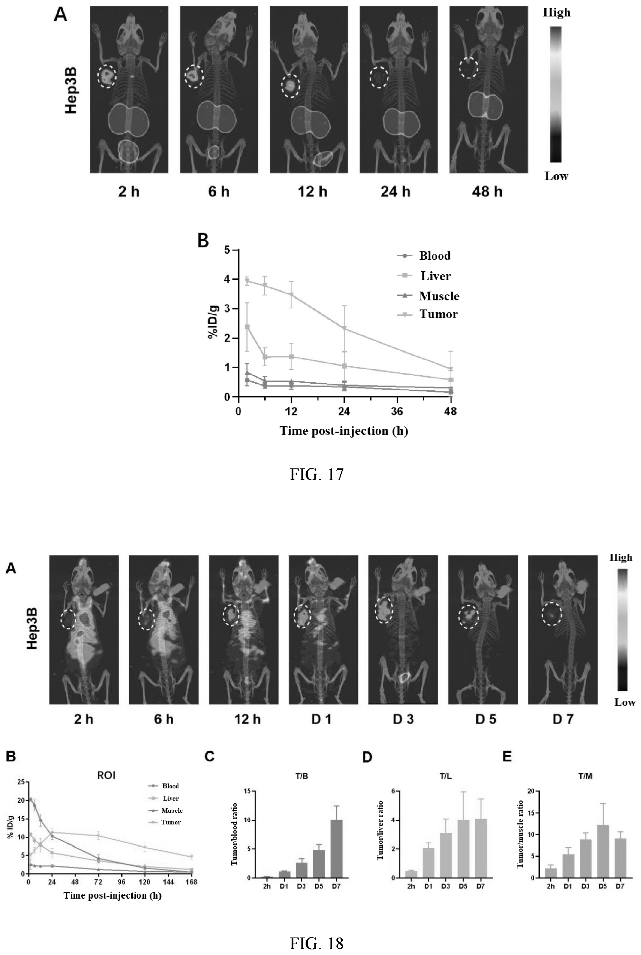

FIG. 18 shows (A) SPECT/CT images of Hep3B tumor-bearing mouse at 2 h, 6 h, 12 h, 1 day, 3 days, 5 days, and 7 days post-injection of ¹⁷⁷Lu-DOTA-aGPC3-mAb (white dotted circle indicating the tumor); (B) ROI semi-quantitative analysis of ¹⁷⁷Lu-DOTA-aGPC3-mAb uptake in blood, liver, muscle, and tumor from Hep3B tumor-bearing mouse; (C) The analysis of tumor/blood pool ratio over time of Hep3B subcutaneous tumor model based on SPECT images; (D) The analysis of tumor/liver ratio over time of Hep3B subcutaneous tumor model based on SPECT images; (E) The analysis of tumor/muscle ratio over time of Hep3B subcutaneous tumor model based on SPECT images.
FIG. 19 shows (A) changes in tumor volume over time and (B) changes in body weight over time of Hep3B tumor-bearing mouse post-injection of saline or ¹⁷⁷Lu-DOTA-aGPC3-Fab.
FIG. 20 shows (A) changes in tumor volume over time and (B) changes in body weight over time of Hep3B tumor-bearing mouse post-injection of saline or ¹⁷⁷Lu-DOTA-aGPC3-scFv.
FIG. 21 shows (A) changes in tumor volume over time and (B) changes in body weight over time of Hep3B tumor-bearing mouse post-injection of saline, aGPC3-mAb, 11.1 MBq ¹⁷⁷Lu-DOTA-aGPC3-mAb, or twice injection of 5.55 MBq ¹⁷⁷Lu-DOTA-aGPC3-mAb.

### DETAILED DESCRIPTION

### I. Definition

Unless defined otherwise, all scientific and technical terms used in the specification, figures and claims have their ordinary meaning as commonly understood by the person of ordinary skill in the art. All publications, patent applications, patents, and other references mentioned herein are incorporated by reference in their entirety. In the case of conflict, the present specification, including definitions, will control. If two or more documents incorporated by reference include conflicting and/or inconsistent disclosure with respect to each other, then the document having the later effective date shall control. The materials, methods, and examples are illustrative only and not intended to be limiting. Unless stated otherwise, the following terms used in this document, including the specification and claims, have the definitions given below.

Singular forms such as "a", "an" or "the" include plural references unless the context clearly indicates otherwise. Thus, for example, reference to an "antibody" includes a single antibody as well as a plurality of antibodies, either the same or different.

The word "about" as used herein refers to a value being within an acceptable error range for the particular value as determined by the person of ordinary skill in the art, which will depend in part on how the value is measured or determined, i.e., on the limitations of the measurement system.

For example, "about" can mean within 1 or more than 1 standard deviation per the practice in the art. The term "about" is also used to indicate that the amount or value in question may be the value designated or some other value that is approximately the same. The word is intended to convey that similar values promote equivalent results or effects as described herein. In this context "about" may refer to a range above and/or below of up to 20% or 10 %. Wherever the term "about" is specified for a certain assay or embodiment, that definition prevails for the particular context.

It is furthermore understood that slight variations above and below a stated range can be used to achieve substantially the same results as a value within the range. Also, unless indicated otherwise, the disclosure of ranges is intended as a continuous range including every value between the minimum and maximum values.

The terms "comprising", "including", "containing", "having" etc. shall be read expansively or open-ended and without limitation. The term "comprising" when used in the specification includes "consisting of" and "essentially consisting of".

Unless indicated otherwise, the term "at least" preceding a series of elements is to be understood to refer to every element in the series. The terms "at least one" and "at least one of" include for example, one, two, three, four, five or more elements.

The terms "peptide", "polypeptide", and "protein" are used interchangeably herein, and refer to a compound which comprises at least two amino acid residues covalently linked by at least one peptide bond. No limitation is placed on the maximum number of amino acids that can form a peptide's sequence. A "peptide" may include but is not limited to a modified amino acid, a non naturally-occuring amino acid and/or D-amino acid. Unless indicated otherwise, a particular peptide sequence also encompasses variants in which at least one amino acid has been replaced by an amino acid which is characterized by similar structural properties. A "peptide" may be a natural peptide, a recombinant peptide, a synthetic peptide, or a combination thereof. A "peptide" may be, for example, a biologically active fragment, an oligopeptide, a homodimer, a heterodimer, a peptide variant, a modified peptide, a peptide derivative, a peptide analog, and a fusion protein.

The term "amino acid" or "amino acid residue" ("aa") as used herein typically refers to a naturally-occuring amino acid but may also refer to a non naturally-occuring amino acid. The term typically refers to an L-amino acid but may also encompass a D-amino acid. An amino acid may or may not be modified as described elsewhere herein. The one letter code is used herein to refer to the respective amino acid. As used herein, a "charged amino acid" refers to an amino acid which is negatively charged or positively charged. The "negatively charged amino acid" refers to aspartic acid (D) or glutamic acid (E). The "positively charged amino acid" refers to arginine (R), lysine (K) or histidine (H). The "polar amino acid" refers to all amino acids that form hydrogen bonds as donors or acceptors, and thus includes all charged amino acids and asparagine (N), glutamine (Q), serine (S), threonine (T), tyrosine (Y) and cysteine (C). The "polar uncharged amino acid" refers to asparagine (N), glutamine (Q), serine (S), threonine (T), tyrosine (Y) and cysteine (C). The "amphiphatic amino acid" refers to tryptophan (W), tyrosine (Y) and methionine (M). The "aromatic amino acid" refers to phenylalanine (F), tyrosine (Y), and tryptophan (W). The "hydrophobic amino acids" refers to glycine (G), alanine (A), valine (V), leucine (L), isoleucine (I), proline (P), phenylalanine (F), methionine (M) and cysteine. The "small amino acid" refers to glycine (G), alanine (A), serine (S), proline (P), threonine (T), aspartic acid (D) and asparagine (N).

The term "sequence identity", "percent identity" or "percent (%) sequence identity" is a number that describes how similar a query sequence is to a target sequence, more precisely how many characters in each sequence are identical after alignment. The most popular tool to calculate sequence identity is BLAST (basic local alignment search tool, NCBI), which performs comparisons between pairs of sequences, searching for regions of local similarity. Suitable alignment methods are known in the art, e.g., Needleman-Wunsch algorithm for global-global alignment, using BLOSUM62 matrix, with gap opening penalty of 11 and a gap extension penalty of 1. Afterwards, the pairs of aligned identical residues can be counted and then divided by the total length of the alignment (including gaps, internal as well as external) to arrive at the percent identity value.

When "homologous" is used in reference to proteins or polypeptides, the inconsistencies in residue positions typically resulted by conservative amino acid substitutions. A "conservative amino acid substitution" refers to one in which an amino acid residue is substituted by another amino acid residue having a side chain (R group) with similar chemical properties (e.g., charge or hydrophobicity). In general, a conservative amino acid substitution will not substantially change the functional properties of a polypeptide. In cases where two or more amino acid sequences differ from each other by conservative substitutions, the percent sequence identity can be adjusted upwards to correct for the conservative nature of the substitution. Means for making this adjustment are well known to a person skilled in the art, e.g., see Pearson, 1994, Methods Mol. Biol. 24:307-31 and 25:365-89.

For example, in some cases, following six groups each contain amino acids that are conservative substitutions for one another: 1) Serine, Threonine; 2) Aspartic Acid, Glutamic Acid; 3) Asparagine, Glutamine; 4) Arginine, Lysine; 5) Isoleucine, Leucine, Methionine, Alanine, Valine, and 6) Phenylalanine, Tyrosine, Tryptophan.

The term "vector", as used herein, refers to a nucleic acid molecule capable of propagating a nucleic acid molecule to which it is linked. The term further comprises plasmids (non-viral) and viral vectors. Certain vectors are capable of directing the expression of nucleic acids or polynucleotides to which they are operatively linked. Such vectors are referred to herein as "expression vectors". Expression vectors for eukaryotic use can be constructed by inserting a polynucleotide sequence encoding at least one protein of interest (POI) into a suitable vector backbone. The vector backbone may include the necessary elements to ensure maintenance of the vector and, if desirable, to provide amplification within the host. For viral vectors, e.g., lentiviral or retroviral vectors, further virus specific elements such as structural elements or other elements may be required and are well known in the art. These elements can be for instance provided in *cis* (on the same plasmid) or in *trans* (on a separate plasmid). Viral vectors may require helper viruses or packaging lines for large-scale transfection. Vectors may contain further elements such as e.g., enhancer elements (e.g., viral, eukaryotic), introns, and viral origins of plasmid replication for replication in mammalian cells. According to the present disclosure, expression vectors typically have a promoter sequence that drives expression of the POI. Expression of the POI and/or selective marker protein may be constitutive or regulated (e.g., inducible by addition or removal of small molecule inductors). Preferred regulatory sequences for mammalian host cell expression include viral elements that direct high levels of expression of a POI in mammalian cells, such as regulatory elements, promoters and/or enhancers derived from cytomegalovirus (CMV), Simian Virus 40 (SV40), adenovirus, (e.g., the adenovirus major late promoter Ad LP) or polyoma. For further description of viral regulatory elements, and sequences thereof, see e.g., U.S. 5.168.062 by Stinski, U.S. 4,510,245 by Bell et al. and U.S. 4,968,615 by Schaffner et al.

The term "conjugate" refers to a molecule comprising at least two moieties. For example, but without limitation, the moieties can be connected via a linking group.

The term "antibody conjugate" refers to a conjugate including at least one antibody or antigen binding fragment moiety and one or more further molecules or moieties. The one or more further molecules or moieties may be selected without limitation from a drug, a chelating moiety, a radioactive element, a cytotoxic agent, a further antibody or antigen binding fragment.

The term "linker" as used herein refers to any molecule enabling a direct topological connection of different portions of a construct or conjugate. For example, a linker may connect the chelating moiety and the targeting moiety. In the alternative, a linker may connect different parts of the chelating moiety. For bispecific antibodies a linker may connect different antigen-binding moieties. Examples for linkers establishing a covalent connection between the different moieties include peptide linker and non-proteinaceous polymers, including but not limited to polyethylene glycol (PEG), polypropylene glycol, polyoxyalkylenes, or copolymers of polyethylene glycol and polypropylene glycol.

The term "GPC3" as used herein encompasses any variant or isoform of GPC3 naturally expressed by cells. GPC3 or any variant or isoform thereof may be obtained by isolating from cells or tissues naturally expressing them, or produced by using recombinant techniques as described herein and those known in the art.

The term "targeting moiety" (also referred to as "tissue-targeting group" or "tissue-targeting moiety") as used herein is any chemical structure binding to a biological target, such as GPC3 or a cell expressing GPC3. The targeting moiety localizes itself, e.g., as part of a bigger structure at a specific site to exert the intended effect. Thus, a tissue targeting group or moiety serves to provide greater localization of a molecule or conjugate to at least one desired site in the body of a subject in comparison with the concentration of an equivalent complex not comprising the targeting moiety. According to the present disclosure, the targeting moiety can be for example selected without limitation from the group consisting of nucleotides, DNA and RNA fragments, aptamers, peptides, proteins, antibodies or fragments thereof, nanoparticles, or small molecules, or any combination thereof.

The term "targeting moiety binding to GPC3" refers to a chemical structure binding to GPC3. According to preferred embodiments of the present disclosure, the targeting moiety can be an antibody or antigen binding fragment thereof, as described herein.

The terms "anti-GPC3 antibody", "GPC3 antibody" and "antibody binding to GPC3" refer to an antibody that is capable of binding to GPC3 or fragment thereof with sufficient affinity such that the antibody can be useful as a diagnostic and/or therapeutic agent in targeting GPC3. In some embodiments, the binding extent of the antibody binding to GPC3 to an unrelated protein different from GPC3 is less than about 10%, less than about 5%, or preferably less than about 2%, and most preferably less than about 1 % of the binding of the antibody to GPC3 as measured, e.g., by surface plasmon resonance (SPR) or a further standard method such as ELISA assay. In certain preferred embodiments, an antibody that binds to GPC3 has a dissociation constant (K_{D}) of ≤ 1µM, ≤500 nM, ≤200 nM, ≤ 100 nM, ≤ 10 nM, ≤ 1 nM, ≤ 0.1 nM, ≤ 0.01 nM, or ≤ 0.001 nM (e.g., 10⁻⁸ M or less, e.g. from 10⁻⁸ M to 10⁻¹³ M, e.g., from 10⁻⁹ M to 10⁻¹¹ M). In certain embodiments, an antibody that binds to GPC3 has a binding activity (EC₅₀) of ≤ 1 µM, ≤ 100 nM, ≤ 10 nM, ≤ 1 nM, ≤ 0.1 nM, ≤ 0.01 nM, or ≤ 0.001 nM (e.g., 10⁻⁸ M or less, e.g., from 10⁻⁸ M to 10⁻¹³ M, e.g., from 10⁻⁹ M to 10⁻¹³ M).

The term "antibody" includes, but is not limited to, an immunoglobulin molecule (e.g., without limitation human IgGl, IgG2, IgG3, IgG4, IgM, IgD, IgE, IgA1, IgA2, mouse IgG1, IgG2a, IgG2b, IgG2c, IgG3, IgA, IgD, IgE or IgM, rat IgG1, IgG2a, IgG2b, IgG2c, IgA, IgD, IgE or IgM, rabbit IgA1, IgA2, IgA3, IgE, IgG, IgM, goat IgA, IgE, IgG1, IgG2, IgE, IgM or chicken IgY) that binds to a particular antigen. Depending on the context, the term "antibody" may also refer to a functional fragment of a full-length antibody as disclosed elsewhere herein. The term furthermore includes any proteinaceous binding molecule with immunoglobulin-like function. According to the present disclosure, the antibodies or fragments thereof are preferably characterized by an affinity for their target corresponding to a K_{D} of less than 10⁻⁷ M, more preferably of less than 10⁻⁸ M, even more preferably in the range from 10⁻¹¹ M to 10⁻⁹ M.

Naturally occurring intact antibodies are approximately 150 kDa tetramer composed of two identical heavy chain polypeptides and two identical light chain polypeptides, which associate with each other to form a structure commonly referred to as the "Y-shaped" conformation. Each heavy chain contains at least four domains: an amino-terminal variable (VH) region (located at the apex of the Y-shaped structure), followed by three constant regions: CH1, CH2, and CH3 at a carboxy-terminal (located at the base of the Y-shaped structure). A hinge region is located between CH1 and CH2. Each light chain consists of two domains: an amino-terminal variable (VL) region and a carboxy-terminal constant (CL) region. The heavy and light chains are linked via a single disulfide bond, while two additional disulfide bonds connect the hinge regions of heavy chains to each other, thereby forming the tetramer.

The amino-terminal variable region of the antibody polypeptide chain is primarily responsible for antigen recognition. The VH and VL regions can be further subdivided into hypervariable regions (also known as complementarity-determining regions or CDRs), separated by more conserved regions called "framework regions" or "FRs." Each VH and VL typically contains three CDRs and up to four FRs arranged in the following order from amino-terminus to carboxy-terminus: FR1, CDR1, FR2, CDR2, FR3, CDR3, FR4.

The term "CDR" refers to the complementary determining region of an antibody. CDRs are crucial to the diversity of antigen specificities. A set of CDRs constitutes a paratope. There are usually three CDRs (CDR1, CDR2 and CDR3), arranged non-consecutively on the amino acid sequence of a variable domain of an antigen receptor. The CDRs are typically held together in close proximity by the FR regions and - e.g., with the CDRs from the other chain - contribute to the formation of the antigen binding site of antibodies. The CDRs of the light chain are termed LCDR1, LCDR2 and LCDR3. The CDRs of the heavy chain are termed HCDR1, HCDR2 and HCDR3. As known in the art, the amino acid position/boundary delineating a hypervariable region of an antibody can vary, depending on the context and the various definitions known in the art. As used herein, numbering of antibody amino acid residues is done according to the immunoglobulin amino acid residue numbering system of IMGT.

The term "fragment crystallizable region", also "Fc domain", "Fc region" or "Fc part" as used herein refers to a C-terminal region of an antibody heavy chain that contains at least a portion of the constant region. The Fc region may interact with Fc receptors and some proteins of the complement system, e.g., on immune effector cells. The Fc region defines the class of an antibody or isotype. For an IgG antibody, the Fc region consists of the CH2 and CH3 domains. The term includes native sequence Fc regions and variant Fc regions.

In some embodiments, an antibody may be polyclonal. In some embodiments, an antibody may be monoclonal.

The term "polyclonal antibodies" refers to heterogenous populations of antibodies, derived for example from the sera of animals immunized with an antigen or an antigenic functional derivative thereof. For the production of polyclonal immunoglobulins, one or more of various host animals may be immunized by injection with the antigen.

The term "monoclonal antibodies" are substantially homogenous populations of antibodies binding a particular antigen. Monoclonal antibodies may be obtained by methods well known to a person skilled in the art (see for example, Kohler et al., Nature (1975) 256, 495-497, and U.S. Patent No. 4,376,110). An antibody or fragment with specific binding affinity can be isolated, enriched, or purified from a prokaryotic or eukaryotic organism.

In some embodiments, an antibody with constant region sequences is an antibody exhibiting mouse, rabbit, primate, or human antibody characteristics. In some embodiments, the antibody sequence element(s) is humanized, primate-adapted, or chimeric. In some embodiments, the antibody includes an intact antibody or immunoglobulin or an antibody fragment.

The "fragment", "functional fragment" or "antigen binding fragment" of an antibody is required to retain the ability of the antibody to bind the particular antigen. The fragment of an antibody therefore typically includes a functional portion of a full-length antibody, generally the antigen binding region or variable region thereof. Preferably, a fragment of an antibody as used herein substantially retains the affinity of the full-length antibody. As such, suitable fragments of an anti-GPC3 antibody will retain the ability to bind to the target protein, e.g., to bind to GPC3. Examples of the antibody fragment include, but are not limited to, Fab, Fab', F(ab')₂, and scFv fragments, single-chain antibody molecules, diabodies and single-domain antibodies.

Fab fragment is a monovalent fragment consisted of VL, VH, CL, and CH1 domains. F(ab')₂ fragment is a bivalent fragment containing two Fab fragments linked by the disulfide bond in the hinge region. Fab' fragment is produced by cleavage of the disulfide bond at the hinge cysteines of the F(ab')₂, and is different from Fab fragment in that a few residues are added at the carboxyl terminus of the heavy chain CH1 domain. Fab and F(ab')₂ fragments lack the Fc fragment of an intact antibody, thereby being cleared more rapidly from the circulation of animals, and having less non-specific tissue binding than the intact antibody.

Fv fragment is the minimum fragment of an antibody that contains a complete target recognition and binding site. This region consists of a dimer of one heavy and one light chain variable domain in a tight, non-covalent association (VH-VL dimer). It is in this configuration that the three CDRs of each variable domain interact to define an antigen binding site on the surface of the VH-VL dimer. Generally, the six CDRs confer antigen binding specificity upon the antibody. However, in some instances even a single variable domain (or half of an Fv comprising only three CDRs specific for a target) may also have the ability to recognize and bind antigens, although the affinity is lower than the whole binding site.

Single-chain Fv or scFv antibody fragment includes VH and VL domains of the antibody in a single polypeptide chain. Generally, scFv further includes a polypeptide linker between VH and VL domains, which enables the scFv to form the desired structure for antigen binding. scFv retains the antigen specificity of the original immunoglobulin while offering advantages such as potent intratumoral penetration, rapid degradation in the blood, and minimal negative feedback in the human body due to its smaller molecular size.

Single-domain antibody or nanobody consists of a single VH domain capable of specifically binding to antigens.

Antibodies or antibody fragments can be produced synthetically or recombinantly. A number of technologies are available to produce antibodies. For example, phage-antibody technology can be used to generate antibodies (Knappik et al., J. Mol. Biol. 296:57-86, 2000). Another approach for obtaining antibodies is to screen a DNA library from B cells as described in WO 91/17271 and WO 92/01047. In these methods, libraries of phages are produced in which members display different antibodies on their outer surfaces. Antibodies are usually displayed as Fv or Fab fragments. Phage displaying antibodies are selected by affinity enrichment for binding to a selected protein. Antibodies can also be produced using trioma methodology (e.g., Oestberg et al., Hybridoma 2:361-367, 1983; U.S. Patent 4,634,664; U.S. Patent 4,634,666).

Antibodies can also be purified from any cell that expresses the antibodies, including host cells that have been transfected with antibody-encoding expression constructs. The host cells can be cultured under conditions whereby the antibodies are expressed. Purified antibody can be separated from other cellular components that can associate with the antibody in the cell, such as certain proteins, carbohydrates, or lipids, using methods well known in the art. Such methods include, but are not limited to, size exclusion chromatography, ammonium sulfate fractionation, ion exchange chromatography, affinity chromatography, and preparative gel electrophoresis. Purity of the preparations can be assessed by any means known in the art, such as SDS-polyacrylamide gel electrophoresis. A preparation of purified antibodies may contain more than one type of antibody.

Alternatively, for antibodies according to the present disclosure, the amino acid sequence thereof can be produced and synthesized by using chemical methods, such as by direct peptide synthesis using solid-phase techniques (e.g., Merrifield, J. Am. Chem. Soc. 85:2149-2154, 1963; Roberge et al., Science 269:202-204, 1995). Protein synthesis can be performed using manual techniques or by automation. Optionally, fragments of antibodies can be separately synthesized and combined using chemical methods to produce a full-length molecule.

The term "affinity" or "binding affinity" is a term of the art and describes the strength of non-covalent binding between a single binding site of a molecule and its binding partner. The affinity of an antibody or fragment thereof for a target can be determined using techniques well known in the art, for example by ELISA, isothermal titration calorimetry (ITC), surface plasmon resonance (SPR), flow cytometry or fluorescent polarization assays. Preferably the affinity is provided as dissociation constant K_{D}. In the alternative, the affinity is provided as an EC₅₀ value.

The "dissociation constant" (K_{D}) has molar units (M) and corresponds to the concentration of the binder (e.g., antibody or fragment) at which half of the target proteins or binding partners are occupied at equilibrium. The smaller the dissociation constant is, the higher is the affinity between the binder and its target. The K_{D} values can be preferably measured through surface plasmon resonance assays by using suitable devices including but not limited to Biacore instruments like Biacore T100, Biacore T200, Biacore 2000, Biacore 4000, Biacore 3000 (GE Healthcare Biacore, Inc.,) (cf. e.g., Sjolander & Urbaniczky; Anal. Chem. 63:2338-2345, 1991; Szabo, et al., Curr. Opin. Struct. Biol. 5:699-705, 1995), or a ProteOn XPR36 instrument (Bio-Rad Laboratories, Inc.). In the alternative, the affinity can be determined using any method known in the art including, for example immunoassays such as enzyme-linked immununospecific assay (ELISA) and fluorescence-activated cell sorting (FACS) for quantifying the binding of antibodies to cells that express an antigen. Where assay conditions were found to influence the determined K_{D}, the assay setup with the least standard deviation shall be used.

"Half maximal effective concentration" (EC₅₀) refers to the concentration of a drug, modulator, antibody, fragment, conjugate or molecule which induces a response halfway between the baseline and maximum after a specified incubation time. When referring to affinity, EC₅₀ reflects the concentration of binder (e.g., antibody) that is required for half-maximal binding. EC₅₀ can be determined if an inflection point can be determined by mathematical modeling (e.g., nonlinear regression) of the dose-response curve describing the relationship between the concentration of the applied drug, antibody, fragment, conjugate or molecule and signal. For example, if the dose-response curve follows a sigmoidal curve, EC₅₀ can be determined. Where the correlation response refers to inhibitory, EC₅₀ is termed "half maximal inhibitory concentration" (IC₅₀).

As used herein, a binder or antibody that is described as "specifically binds to", refers to that with respect to "specific to/for" or "specifically recognizing" an antigen of interest, e.g., GPC3, it can bind the antigen with sufficient affinity such that the binder or antibody can be useful as a therapeutic agent in targeting a cell or tissue expressing the antigen without significantly cross-reacting with proteins other than orthologs and variants (e.g. mutant forms, splice variants, or proteolytically truncated forms) of the aforementioned antigen target. The term "specifically recognizing" as used herein can be exhibited, for example, by a binder, an antibody, or antigen-binding fragment thereof, having a monovalent K_{D} for the antigen of less than about 10⁻⁴ M, or less than about 10⁻⁵ M, or less than about 10⁻⁶ M, or less than about 10⁻⁷ M, or less than about 10⁻⁸ M, or less than about 10⁻⁹ M, or less than about 10⁻¹⁰ M, or less than about 10⁻¹¹ M, or less than about 10⁻¹² M, or more less.

In its most general form, "specific binding" is referring to the ability of a binder or antibody to discriminate between the antigen of interest and an unrelated antigen, as determined, for example, by surface plasmon resonance (SPR), Western blot, ELISA-, RIA-, ECL-, IRMA-test or peptide scans. For example, a standard ELISA assay can be carried out. The scoring may be carried out by standard color development (e.g., secondary antibody with horseradish peroxidase and tetramethyl benzidine with hydrogen peroxide). The reaction in certain wells is scored by the optical density, for example, at 450 nm. Typical background (=negative reaction) may be 0.1 OD; typical positive reaction may be 1 OD. This means the difference positive/negative is more than 5-fold, 10-fold, 50-fold, and preferably more than 100-fold. Typically, determination of binding specificity is performed by using a set of about three to five unrelated antigens (such as milk powder, BSA, transferrin or the like), rather than a single reference antigen.

A "radionuclide" (also called "radioactive nuclide", "radioisotope" or "radioactive isotope") refers to an atom with unstable nuclei, characterized by excess energy that can be transferred to newly generated radiation particles or electrons within the nucleus. During this process, the radionuclide undergoes radioactive decay. The radionuclide can occur naturally or be produced artificially. The properties of radionuclides, including half-life, energy emission characteristics, and decay type, vary significantly, and thus radionuclides with specific combinations of properties suitable for diagnostic and/or therapeutic applications can be selected. For example, gamma-ray emitters are typically used for diagnosis, while beta-ray emitters are typically used for treatment. However, some radionuclides serve as both gamma-ray emitters and beta-ray emitters. Therefore, by adjusting the radioactivity employed, these radionuclides may be suitable for both diagnostic and therapeutic applications. The radionuclides herein also include paramagnetic metals used for MRI imaging.

The term "diagnostic radionuclide" refers to a radionuclide that can be conjugated to an antibody and provides a detectable signal. Diagnostic radionuclides include those detectable by radioactive decay or other imaging techniques. Diagnostic radionuclides can be detected through various imaging techniques, such as PET, SPECT, or MRI imaging. Exemplary diagnostic radionuclides include but are not limited to: ⁶⁸Ga, ¹⁸F-Al, ⁶⁴Cu, ^{99m}Tc, ⁸⁹Zr, ⁴⁴Sc, ¹¹¹In, ⁴⁵Ti, ⁵²Mn, ⁵⁹Fe, ^{94m}Tc, ¹³¹I, ⁶⁷Ga, ⁷¹As, ⁷²As, ^{82m}Rb, ⁸⁶Y, Gd, Mn, or any combination thereof.

The term "therapeutic radionuclide" refers to a radionuclide that can be conjugated to an antibody, functioning to deliver a cytotoxic dose of radiation to a target location (such as a tumor). Suitable therapeutic radionuclides include radionuclides emitting β particles, α particles, or Auger electrons. Exemplary therapeutic radionuclides include but are not limited to: ¹⁷⁷Lu, ⁹⁰Y, ¹⁵³Sm, ⁶⁷Cu, ⁸⁹Sr, ¹³¹I, ¹⁶⁶Ho, ¹⁷⁷Yb, ⁴⁷Sc, ¹⁸⁶Re, ¹⁸⁸Re, ²¹²Bi, ²¹³Bi, ¹⁴⁹Pm, ²¹²Pb, ²¹¹At, ²²³Ra, ²²⁵Ac, ²²⁷Th, or any combination thereof.

"Treating" a disease in a subject or "treating" a subject with a disease refers to subjecting the subject to a pharmaceutical treatment, e.g., administrating a drug, such that at least one symptom of the disease is decreased or prevented from worsening. When referring to a specific disease, treatment indicates: (1) improving the disease or one or more of its biological manifestations; (2) interfering with one or more points in the biological cascade that causes or contributes to the disease; (3) alleviating one or more symptoms, effects, or side effects associated with the disease; or (4) slowing the progression of the disease.

The terms "prevent", "preventing", "prevention" refer to reducing the probability of developing a disease, disorder, or condition in a subject, who does not have, but is at risk of or susceptible to developing a disease, disorder, or condition.

The terms "effective amount" and "therapeutically effective amount" are used interchangeably herein and refer to an amount sufficient to achieve a particular biological result, or to modulate or ameliorate a symptom in a subject, or the time of onset of a symptom typically by at least about 10%, usually by at least about 20%, preferably at least about 30%, or more preferably at least about 50%.

The term "pharmaceutically acceptable" refers to a substance that is not physiologically or otherwise undesirable. For example, the substance can be incorporated into a pharmaceutical composition and administered to a patient without producing any significant undesirable physiological effects, nor does it interact in a harmful manner with any other component contained in the composition.

The term "pharmaceutically acceptable salt" refers to an inorganic or organic acid addition salt of a compound disclosed herein. A suitable pharmaceutically acceptable salt of the compounds disclosed herein may be, for example and without limitation, an acid-addition salt of a compound disclosed herein bearing a nitrogen atom in a chain or in a ring with for example sufficiently basic, such as an acid-addition salt with an inorganic acid, or "mineral acid", such as hydrochloric, hydrobromic, hydroiodic, sulfuric, sulfamic, bisulfuric, phosphoric, or nitric acid, or with an organic acid, such as formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, 3-phenylpropionic, pivalic, 2-hydroxyethanesulfonic, itaconic, trifluoromethanesulfonic, dodecylsulfuric, ethanesulfonic, benzenesulfonic, para-toluenesulfonic, methanesulfonic, 2-naphthalenesulfonic, naphthalinedisulfonic, camphorsulfonic, citric, tartaric, stearic, lactic, oxalic, malonic, succinic, malic, adipic, alginic, maleic, fumaric, D-gluconic, mandelic, ascorbic, glucoheptanoic, glycerophosphoric, aspartic, sulfosalicylic, or thiocyanic acid. Further, another suitably pharmaceutically acceptable salt of a compound disclosed herein with sufficiently acidic, is an alkali metal salt, for example a sodium or potassium salt, an alkaline earth metal salt, for example a calcium, magnesium or strontium salt, or an aluminium or a zinc salt, or an ammonium salt derived from ammonia or from an organic primary, secondary or tertiary amine having 1 to 20 carbon atoms, such as ethylamine, diethylamine, triethylamine, ethyldiisopropylamine, monoethanolamine, diethanolamine, triethanolamine, dicyclohexylamine, dimethylaminoethanol, diethylaminoethanol, tris(hydroxymethyl)aminomethane, procaine, dibenzylamine, N-methylmorpholine, arginine, lysine, 1,2-ethylenediamine, N-methylpiperidine, N-methyl-glucamine, N,N-dimethyl-glucamine, N- ethyl-glucamine, 1,6-hexanediamine, glucosamine, sarcosine, serinol, 2-amino-1,3-propanediol, 3-amino-1,2-propanediol, 4-amino-1,2,3-butanetriol, or a salt with a quarternary ammonium ion having 1 to 20 carbon atoms, such as tetramethylammonium, tetraethylammonium, tetra(n-propyl)ammonium, tetra(n-butyl)ammonium, N-benzyl-N,N,N-trimethylammonium, choline or benzalkonium.

### II. Radiolabeled anti-GPC3 antibody conjugates

The present disclosure provides a radiolabeled molecular probe that binds to glypican-3 (GPC3), and includes a targeting moiety binding to GPC3, a linking group, and a radionuclide.

### 2.1 Targeting Moiety binding to GPC3

The targeting moiety binding to GPC3 of the disclosure may be selected from, but is not limited to, peptides, proteins, antibody or antigen-binding fragments, nanoparticles, polynucleotides, DNA or RNA fragments, aptamers, or small molecules that specifically bind to GPC3.

In some embodiments, the targeting moiety binding to GPC3 provided by the disclosure binds to GPC3 with a K_{D} of about 1.0 pM to 200 nM. In some embodiments, the targeting moiety binding to GPC3 provided by the disclosure inhibits cell proliferation with an IC₅₀ of about 0.01 to 250 nM.

In some embodiments, the targeting moiety binding to GPC3 provided by the disclosure is anti-GPC3 antibody or antigen binding fragment thereof. In some embodiments, anti-GPC3 antibody or antigen binding fragment thereof is a monoclonal antibody, polyclonal antibody, single-chain antibody, chimeric antibody, humanized antibody, or fully human antibody.

In some embodiments, anti-GPC3 antibody or antigen binding fragment thereof is a monoclonal antibody (mAb), Fab, Fab', F(ab')₂, Fv, scFv, scFv-Fc, or single-domain antibody.

In some embodiments, anti-GPC3 antibody or antigen binding fragment thereof is of IgG, IgM, IgA, IgE, IgD, or IgY type.

In some preferred embodiments, anti-GPC3 antibody or antigen binding fragment thereof contains at least one, two, three, four, five, or six CDR sequences. CDR sequence has at least 80%, 90%, 95%, 98%, 99%, or 100% sequence identity to at least one of SEQ ID NO: 1, SEQ ID NO: 2, SEQ ID NO: 3, SEQ ID NO: 4, SEQ ID NO: 5, or SEQ ID NO: 6.

In preferred embodiments, anti-GPC3 antibody or antigen binding fragment thereof contains a heavy chain and fragment thereof having HCDR1, HCDR2, and HCDR3. HCDR1 contains an amino acid sequence having at least 80%, 90%, 95%, 98%, 99%, or 100% sequence identity to the amino acid sequence shown as SEQ ID NO: 1. Preferably, the amino acid sequence of HCDR1 is shown as SEQ ID NO: 1. HCDR2 contains an amino acid sequence having at least 80%, 90%, 95%, 98%, 99%, or 100% sequence identity to the amino acid sequence shown as SEQ ID NO: 2. Preferably, the amino acid sequence of HCDR2 is shown as SEQ ID NO: 2. HCDR3 contains an amino acid sequence having at least 80%, 90%, 95%, 98%, 99%, or 100% sequence identity to the amino acid sequence shown as SEQ ID NO: 3. Preferably, the amino acid sequence of HCDR3 is shown as SEQ ID NO: 3.

In preferred embodiments, the heavy chain and fragment thereof contains a heavy chain variable region (VH). VH contains an amino acid sequence having at least 80%, 90%, 95%, 98%, 99%, or 100% sequence identity to the amino acid sequence shown as SEQ ID NO: 7. Preferably the amino acid sequence of VH is shown as SEQ ID NO: 7.

In more preferred embodiments, the heavy chain contains an amino acid sequence having at least 80%, 90%, 95%, 98%, 99%, or 100% sequence identity to the amino acid sequence shown as SEQ ID NO: 9. Preferably the amino acid sequence of the heavy chain is shown as SEQ ID NO: 9.

In preferred embodiments, anti-GPC3 antibody or antigen binding fragment thereof contains a light chain and fragment thereof having LCDR1, LCDR2, and LCDR3. LCDR1 contains an amino acid sequence having at least 80%, 90%, 95%, 98%, 99%, or 100% sequence identity to the amino acid sequence shown as SEQ ID NO: 4. Preferably, the amino acid sequence of LCDR1 is shown as SEQ ID NO: 4. LCDR2 contains an amino acid sequence having at least 80%, 90%, 95%, 98%, 99%, or 100% sequence identity to the amino acid sequence shown as SEQ ID NO: 5. Preferably, the amino acid sequence of LCDR2 is shown as SEQ ID NO: 5. LCDR3 contains an amino acid sequence having at least 80%, 90%, 95%, 98%, 99%, or 100% sequence identity to the amino acid sequence shown as SEQ ID NO: 6. Preferably, the amino acid sequence of LCDR3 is shown as SEQ ID NO: 6.

In preferred embodiments, the light chain and fragment thereof contains a light chain variable region (VL). VL contains an amino acid sequence having at least 80%, 90%, 95%, 98%, 99%, or 100% sequence identity to the amino acid sequence shown as SEQ ID NO: 8. Preferably the amino acid sequence of VL is shown as SEQ ID NO: 8.

In more preferred embodiments, the light chain contains an amino acid sequence having at least 80%, 90%, 95%, 98%, 99%, or 100% sequence identity to the amino acid sequence shown as SEQ ID NO: 10. Preferably the amino acid sequence of the light chain is shown as SEQ ID NO: 10.

In some embodiments, anti-GPC3 antibody or antigen binding fragment thereof is of Fab, Fab', or F(ab')₂ fragment, preferably Fab fragment.

In some preferred embodiments, Fab, Fab', or F(ab')₂ fragment contains at least one, two, three, four, five, or six CDR sequences. CDR sequence has at least 80%, 90%, 95%, 98%, 99%, or 100% sequence identity to at least one of SEQ ID NO: 1, SEQ ID NO: 2, SEQ ID NO: 3, SEQ ID NO: 4, SEQ ID NO: 5, or SEQ ID NO: 6.

In preferred embodiments, Fab, Fab', or F(ab')₂ fragment contains a heavy chain and fragment thereof having HCDR1, HCDR2, and HCDR3. HCDR1 contains an amino acid sequence having at least 80%, 90%, 95%, 98%, 99%, or 100% sequence identity to the amino acid sequence shown as SEQ ID NO: 1. Preferably, the amino acid sequence of HCDR1 is shown as SEQ ID NO: 1. HCDR2 contains an amino acid sequence having at least 80%, 90%, 95%, 98%, 99%, or 100% sequence identity to the amino acid sequence shown as SEQ ID NO: 2. Preferably, the amino acid sequence of HCDR2 is shown as SEQ ID NO: 2. HCDR3 contains an amino acid sequence having at least 80%, 90%, 95%, 98%, 99%, or 100% sequence identity to the amino acid sequence shown as SEQ ID NO: 3. Preferably, the amino acid sequence of HCDR3 is shown as SEQ ID NO: 3.

In preferred embodiments, the heavy chain and fragment thereof contains a heavy chain variable region (VH). VH contains an amino acid sequence having at least 80%, 90%, 95%, 98%, 99%, or 100% sequence identity to the amino acid sequence shown as SEQ ID NO: 7. Preferably, the amino acid sequence of VH is shown as SEQ ID NO: 7.

In more preferred embodiments, the heavy chain contains an amino acid sequence having at least 80%, 90%, 95%, 98%, 99%, or 100% sequence identity to the amino acid sequence shown as SEQ ID NO: 11. Preferably, the amino acid sequence of the heavy chain is shown as SEQ ID NO: 11.

In preferred embodiments, Fab, Fab', or F(ab')₂ fragment contains a light chain and fragment thereof having LCDR1, LCDR2, and LCDR3. LCDR1 contains an amino acid sequence having at least 80%, 90%, 95%, 98%, 99%, or 100% sequence identity to the amino acid sequence shown as SEQ ID NO: 4. Preferably, the amino acid sequence of LCDR1 is shown as SEQ ID NO: 4. LCDR2 contains an amino acid sequence having at least 80%, 90%, 95%, 98%, 99%, or 100% sequence identity to the amino acid sequence shown as SEQ ID NO: 5. Preferably, the amino acid sequence of LCDR2 is shown as SEQ ID NO: 5. LCDR3 contains an amino acid sequence having at least 80%, 90%, 95%, 98%, 99%, or 100% sequence identity to the amino acid sequence shown as SEQ ID NO: 6. Preferably, the amino acid sequence of LCDR3 is shown as SEQ ID NO: 6.

In preferred embodiments, the light chain and fragment thereof contains a light chain variable region (VL). VL contains an amino acid sequence having at least 80%, 90%, 95%, 98%, 99%, or 100% sequence identity to the amino acid sequence shown as SEQ ID NO: 8. Preferably, the amino acid sequence of VL is shown as SEQ ID NO: 8.

In more preferred embodiments, the light chain contains an amino acid sequence having at least 80%, 90%, 95%, 98%, 99%, or 100% sequence identity to the amino acid sequence shown as SEQ ID NO: 10. Preferably, the amino acid sequence of the light chain is shown as SEQ ID NO: 10.

In some embodiments, anti-GPC3 antibody or antigen binding fragment thereof is scFv.

In some preferred embodiments, scFv contains at least one, two, three, four, five, or six CDR sequences. CDR sequence has at least 80%, 90%, 95%, 98%, 99%, or 100% sequence identity to at least one of SEQ ID NO: 1, SEQ ID NO: 2, SEQ ID NO: 3, SEQ ID NO: 4, SEQ ID NO: 5, or SEQ ID NO: 6.

In preferred embodiments, scFv contains a heavy chain variable region (VH) having HCDR1, HCDR2, and HCDR3. HCDR1 contains an amino acid sequence having at least 80%, 90%, 95%, 98%, 99%, or 100% sequence identity to the amino acid sequence shown as SEQ ID NO: 1. Preferably, the amino acid sequence of HCDR1 is shown as SEQ ID NO: 1. HCDR2 contains an amino acid sequence having at least 80%, 90%, 95%, 98%, 99%, or 100% sequence identity to the amino acid sequence shown as SEQ ID NO: 2. Preferably, the amino acid sequence of HCDR2 is shown as SEQ ID NO: 2. HCDR3 contains an amino acid sequence having at least 80%, 90%, 95%, 98%, 99%, or 100% sequence identity to the amino acid sequence shown as SEQ ID NO: 3. Preferably, the amino acid sequence of HCDR3 is shown as SEQ ID NO: 3.

In preferred embodiments, the heavy chain variable region (VH) contains an amino acid sequence having at least 80%, 90%, 95%, 98%, 99%, or 100% sequence identity to the amino acid sequence shown as SEQ ID NO: 7. Preferably, the amino acid sequence of VH is shown as SEQ ID NO: 7.

In preferred embodiments, scFv contains a light chain variable region (VL) having LCDR1, LCDR2, and LCDR3. LCDR1 contains an amino acid sequence having at least 80%, 90%, 95%, 98%, 99%, or 100% sequence identity to the amino acid sequence shown as SEQ ID NO: 4. Preferably, the amino acid sequence of LCDR1 is shown as SEQ ID NO: 4. LCDR2 contains an amino acid sequence having at least 80%, 90%, 95%, 98%, 99%, or 100% sequence identity to the amino acid sequence shown as SEQ ID NO: 5. Preferably, the amino acid sequence of LCDR2 is shown as SEQ ID NO: 5. LCDR3 contains an amino acid sequence having at least 80%, 90%, 95%, 98%, 99%, or 100% sequence identity to the amino acid sequence shown as SEQ ID NO: 6. Preferably, the amino acid sequence of LCDR3 is shown as SEQ ID NO: 6.

In preferred embodiments, the light chain variable region (VL) contains an amino acid sequence having at least 80%, 90%, 95%, 98%, 99%, or 100% sequence identity to the amino acid sequence shown as SEQ ID NO: 8. Preferably the amino acid sequence of VL is shown as SEQ ID NO: 8.

In more preferred embodiments, scFv contains an amino acid sequence having at least 80%, 90%, 95%, 98%, 99%, or 100% sequence identity to the amino acid sequence shown as SEQ ID NO: 12. Preferably, the amino acid sequence of scFv is shown as SEQ ID NO: 12.

Proteins, peptides, and/or amino acid sequences mentioned in the present disclosure should also be understood to include variants or homologs that possess the same or similar functions as the proteins, peptides, and/or amino acid sequences.

In the present disclosure, variants may be proteins or peptides obtained by substituting, deleting, or inserting one or more amino acids relative to the amino acid sequence of the protein and/or peptide (e.g., antibodies or fragments thereof that specifically bind to GPC3). For example, functional variants may include proteins or peptides with a modification (e.g., a substitution, deletion, and/or insertion) of at least one amino acid (e.g., 1-30, 1-20, 1-10, or for example 1, 2, 3, 4, or 5 amino acids). Functional variants can essentially retain the biological activity of the original protein or peptide without a modification (e.g., an amino acid substitution, deletion, and/or insertion). For example, functional variants may retain at least 60%, 70%, 80%, 90%, or 100% of the biological activity of the original protein or peptide. For example, a substitution may be a conservative amino acid substitution.

### 2.2 Radionuclide and linking group

In the radiolabeled antibody conjugates of the present disclosure, a radionuclide is directly or indirectly conjugated to the GPC3 targeting moiety (e.g., anti-GPC3 antibody or antigen binding fragment thereof) to form a radiolabeled antibody conjugate.

Suitable radionuclides include, but are not limited to, those that form stable complexes with a chelating moiety and possess a physical half-life suitable for imaging and/or therapeutic purposes. Suitable radionuclides further include those that bind directly to the GPC3 targeting moiety (e.g., ⁷⁶Br and ¹³¹I) and those introduced via a repair group (e.g., ¹⁸F).

Examples of the chelating moiety include, but are not limited to, structures that form stable complexes with radionuclides, e.g., deferoxamine (DFO), 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), 6-hydrazinonicotinic acid (HYNIC), 4-[[[(1R,2R)-2-[bis(carboxymethyl)amino]cyclohexyl] (carboxymethyl)amino]methyl]-phenylacetic acid (H3RESCA), ethylenediaminetetraacetic acid (EDTA), 1,4,7,10-tetraazacyclododecane-1,4,7,10-tetrakis (methylene phosphonic acid) (DOTP), 1,4,7- triazacyclononane-1,4,7-tris (methylene phosphonic acid) (NOTP), 1,4,8,11-tetraazacyclotetradecane-1,4,8,11-tetraacetic acid (TETA), 1,4,7,10-tetrakis(carbamoylmethyl)-1,4,7,10-tetraazacyclododecane (DOTAM), triacetylfusarine C (TAFC), ferrochloroquine E (FOXE), ferrochloroquine B (FOXB), Ferrichrome A (FCHA) and the like.

In some embodiments, the chelating moiety is covalently bonded to GPC3 targeting moiety (e.g., anti-GPC3 antibody or antigen binding fragment thereof) via a linker. The linker covalently links the chelating moiety to GPC3 targeting moiety. In some embodiments, the linker is formed by the reaction between a reactive moiety (e.g., cysteine or lysine of an antibody) of GPC3 targeting moiety and a reactive moiety (e.g., p-isothiocyanate phenyl or amido group) linked to the chelating moiety and a reactive moiety provided in the following conjugation method. In addition, the linker may optionally further include chemical groups ("a spacer group") for adjusting polarity, solubility, spatial interaction, rigidity and/or the length between the chelating moiety and the GPC3 targeting moiety. For example, the linker may be a single bond, an optionally substituted C₁-C₂₀ alkylene group, an optionally substituted C₁-C₂₀ alkenylene group, an optionally substituted C₁-C₂₀ alkynylene group, an optionally substituted C₁-C₂₀ heteroalkylene group, an optionally substituted C₃-C₂₀ cycloalkylene group, an optionally substituted C₃-C₂₀ heterocycloalkylene group, an optionally substituted C₅-C₂₀ arylene group, an optionally substituted C₅-C₂₀ heteroarylene group, ketone group, -S-, -(C=S)-, -O-, -NH-, - NHCO-, polypeptides (e.g., valine-citrulline (Val-Cit), valine-alanine (Val-Ala), glycine-glycine-phenylalanine-glycine (Gly-Gly-Phe-Gly), phenylalanine-lysine (Phe-Lys), alanine-alanine-asparagine (Ala-Ala-Asn), polyglycine), polyethylene glycol (PEG), polypropylene glycol, polyoxyalkylene, or copolymer of polyethylene glycol and polypropylene glycol.

The radionuclides mentioned in the present disclosure include diagnostic radionuclides and therapeutic radionuclides. Exemplary diagnostic radionuclides include but are not limited to: ⁶⁸Ga, ¹⁸F-Al, ⁶⁴Cu, ^{99m}Tc, ⁸⁹Zr, ⁴⁴Sc, ¹¹¹In, ⁴⁵Ti, ⁵²Mn, ⁵⁹Fe, ^{94m}Tc, ⁶⁷Ga, ⁷¹As, ⁷²As, ^{82m}Rb, ⁸⁶Y, ¹³¹I, Gd, Mn, or any combination thereof. Exemplary therapeutic radionuclides include but are not limited to: ¹⁷⁷Lu, ⁹⁰Y, ¹⁵³Sm, ⁶⁷Cu, ⁸⁹Sr, ¹⁶⁶Ho, ¹⁷⁷Yb, ⁴⁷Sc, ¹⁸⁶Re, ¹⁸⁸Re, ²¹²Bi, ²¹³Bi, ¹⁴⁹Pm, ²¹²Pb, ²¹¹At, ²²³Ra, ²²⁵Ac, ²²⁷Th, or any combination thereof.

### 2.3 Preparation of radiolabeled conjugates

Radiolabeled GPC3 conjugates may be prepared by: (1) reacting GPC3 targeting moiety (e.g., an antibody or antigen binding fragment thereof) with a molecule containing a chelating moiety and a moiety capable of reacting with desired conjugation sites on GPC3 targeting moiety; and (2) loading the desired radionuclide.

The desired conjugation sites include, but are not limited to, lysine and cysteine residues, which may be either native or synthetic and may reside on, for example, the heavy chain or light chain of an antibody. Cysteine conjugation sites include, but are not limited to, structures obtained through mutation, insertion, or reduction of antibody disulfide bonds. Methods for preparing cysteine-engineered antibodies include, but are not limited to, those disclosed in WO2011/056983. Site-specific conjugation methods may also be employed to direct the conjugation reaction to specific sites on the antibody, achieving desired stoichiometry, and/or attain the desired drug-to-antibody ratio (DAR). Such conjugation methods are well known to a person skilled in the art and include, but are not limited to, cysteine-engineering and enzymatic and chemo-enzymatic methods, including but not limited to glutamine conjugation, Q295 conjugation, and transglutaminase-mediated conjugation, as well as those described in J. Clin. Immunol., 36:100 (2016), the entirety of which is incorporated herein by reference. Suitable moieties reacting with the desired conjugation site are typically capable of efficiently and readily coupling GPC3 targeting moiety (e.g., an antibody or antigen binding fragment thereof) to a chelating moiety of a radionuclide. Moieties reacting with lysine and cysteine sites include electrophilic groups that are well known to a person skilled in the art. In some aspects, when the desired conjugation site is lysine, the reactive moiety is isothiocyanate (e.g., p-isothiocyanate phenyl or reactive ester). In some aspects, when the desired conjugation site is cysteine, the reactive moiety is maleimide.

In some embodiments, suitable reactive moieties include, but are not limited p-isothiocyanate phenyl, N-hydroxysuccinimide ester, maleimidyl, 2,3,5,6-tetrafluorophenol ester, N-succinimidyl-S-acetylthioacetat and the like. In some embodiments, molecules containing a chelating moiety and a moiety capable of reacting with a conjugation site include, but are not limited to, p-isothiocyanate phenyl-NOTA (p-SCN-Bn-NOTA), p-isothiocyanate phenyl-DOTA (p-SCN-Bn-DOTA), p-isothiocyanate phenyl-deferoxamine (p-SCN-Bn-DFO) or a tetrafluorophenyl ester derivative of RESCA (H3RESCA-TFP).

The loading of the radionuclide is achieved by incubating GPC3 targeting moiety and the chelating moiety conjugates with the radionuclide for a sufficiently long period of time. The sufficiently long period of time is a period of time sufficient to allow the radionuclide to coordinate with the chelating moiety, for example by performing the method described in the embodiments provided herein, or a substantially similar method.

### 2.4 Exemplary embodiments of conjugates

In some embodiments, radiolabeled molecular probes provided by the present disclosure is a compound having a following structure, or pharmaceutically acceptable salt or ester thereof:

Abₘ-Rₙ (I)

where Ab is a targeting moiety binding to GPC3, such as anti-GPC3 antibody, including antigen binding fragments thereof, scFv, or single-domain antibody; R is selected from a group consisting of a linking group, a chelating moiety, a radionuclide, another targeting peptide or antibody, antibody fragment, single-domain antibody, and a small-molecule inhibitor; n is an integer from 0 to 4; m is not 0, preferably an integer from 1 to 4, more preferably 1 or 2.

In some embodiments, the molecular probe provided by the disclosure is a compound having a following structure, or pharmaceutically acceptable salt or ester thereof:

Abₘ-(L-M_{z})ₙ (II)

where Ab is targeting moiety binding to GPC3; L is a linking group; M is a radionuclide, z is 0 or 1; n is an integer from 1 to 4, preferably 1 or 2; m is an integer from 1 to 4, preferably 1 or 2.

In some embodiments, L is represented by following formula: Y-Lk-T (III)
where Y is a linker, Lk is a coupling group, and T is a chelating moiety.

In some embodiments, Lk is independently selected from a single bond, p-isothiocyanate phenyl, N-hydroxysuccinimide ester, maleimidyl, 2,3,5,6-tetrafluorophenol ester, N-succinimidyl-S-acetylthioacetate, amido group, or a combination thereof. In some embodiments, Lk is independently selected from a single bond, p-isothiocyanate phenyl, amido group, or maleimidyl. In some embodiments, Lk is p-isothiocyanate phenyl or amido group. In some embodiments, Lk is p-isothiocyanate phenyl.

In some embodiments, Y includes a reactive moiety and a spacer group, in which the spacer group is independently selected from a single bond, an optionally substituted C₁-C₂₀ alkylene group, an optionally substituted C₁-C₂₀ alkenylene group, an optionally substituted C₁-C₂₀ alkynylene group, an optionally substituted C₁-C₂₀ heteroalkylene group, an optionally substituted C₃-C₂₀ cycloalkylene group, an optionally substituted C₃-C₂₀ heterocycloalkylene group, an optionally substituted C₅-C₂₀ arylene group, an optionally substituted C₅-C₂₀ heteroarylene group, ketone group, -S-, -(C=S)-, -O-, -NH-, -NHCO-, polypeptides (e.g., valine-citrulline (Val-Cit), valine-alanine (Val-Ala), glycine-glycine-phenylalanine-glycine (Gly-Gly-Phe-Gly), phenylalanine-lysine (Phe-Lys), alanine-alanine-asparagine (Ala-Ala-Asn), polyglycine), polyethylene glycol (PEG), polypropylene glycol, polyoxyalkylene, copolymerization groups of polyethylene glycol and polypropylene glycol, or a combination thereof; the reactive moiety is linked to Ab and is independently selected from a single bond, p-isothiocyanate phenyl, N-hydroxysuccinimide ester, maleimidyl, 2,3,5,6-tetrafluorophenol ester, N-succinimidyl-S-acetylthioacetate, amido group, or a combination thereof.

In some embodiments, Y includes a reactive moiety and a spacer group, in which the spacer group is independently selected from a single bond, an optionally substituted C₁-C₁₀ alkylene group, an optionally substituted C₁-C₁₀ alkenylene group, an optionally substituted C₁-C₁₀ alkynylene group, an optionally substituted C₁-C₁₀ heteroalkylene group, an optionally substituted C₃-C₁₀ cycloalkylene group, an optionally substituted C₃-C₁₀ heterocycloalkylene group, an optionally substituted C₅-C₁₀ arylene group, an optionally substituted C₅-C₁₀ heteroarylene group, ketone group, -S-, -(C=S)-, -O-, -NH-, -NHCO-, -(CH₂CH₂O)ᵣ- (also referred to as -(PEG)ᵣ-), -(CH₂CH₂CH₂O)ᵣ-, valine-citrulline (Val-Cit), valine-alanine (Val-Ala), glycine-glycine-phenylalanine-glycine (Gly-Gly-Phe-Gly), phenylalanine-lysine (Phe-Lys), alanine-alanine-asparagine (Ala-Ala-Asn), -(Gly)ᵣ-, or a combination thereof, in which r is an integer from 1 to 10; the reactive moiety is linked to Ab and is independently selected from a single bond, p-isothiocyanate phenyl, N-hydroxysuccinimide ester, maleimidyl, 2,3,5,6-tetrafluorophenol ester, N-succinimidyl-S-acetylthioacetate, amido group, or a combination thereof.

In some embodiments, Y includes a reactive moiety and a spacer group, in which the spacer group is independently selected from a single bond, -(PEG)ᵣ-, -(Gly)ᵣ-, or a combination thereof, in which r is an integer from 1 to 10; the reactive moiety is linked to Ab and is independently selected from a single bond, p-isothiocyanate phenyl, maleimidyl, amido group, or a combination thereof.

In some embodiments, T is independently selected from a single bond or a ligand derived from any of following structures: deferoxamine (DFO), 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), 6-hydrazinonicotinic acid (HYNIC), 4-[[[(1R,2R)-2-[bis(carboxymethyl)amino]cyclohexyl] (carboxymethyl)amino]methyl]-phenylacetic acid (H3RESCA), ethylenediaminetetraacetic acid (EDTA), 1,4,7,10-tetraazacyclododecane-1,4,7,10-tetrakis (methylene phosphonic acid) (DOTP), 1,4,7-triazacyclononane-1,4,7-tris (methylene phosphonic acid) (NOTP), 1,4,8,11-tetraazacyclotetradecane-1,4,8,11-tetraacetic acid (TETA), 1,4,7,10-tetrakis(carbamoylmethyl)-1,4,7,10-tetraazacyclododecane (DOTAM), triacetylfusarine C (TAFC), ferrochloroquine E (FOXE), ferrochloroquine B (FOXB), Ferrichrome A (FCHA), or a combination thereof.

In some embodiments, T is independently selected from a single bond or a ligand derived from any of following structures: deferoxamine (DFO), 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), 4-[[[(1R,2R)-2-[bis(carboxymethyl)amino]cyclohexyl] (carboxymethyl)amino]methyl]-phenylacetic acid (H3RESCA), 1,4,7,10-tetraazacyclododecane-1,4,7,10-tetrakis (methylene phosphonic acid) (DOTP), 1,4,7- triazacyclononane-1,4,7-tris (methylene phosphonic acid) (NOTP), or a combination thereof.

In some embodiments, T is independently selected from a single bond or a ligand derived from any of following structures: 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), 4-[[[(1R,2R)-2-[bis(carboxymethyl)amino]cyclohexyl] (carboxymethyl)amino]methyl]-phenylacetic acid (H3RESCA), or a combination thereof.

In some embodiments, T is independently selected from a single bond or a ligand derived from any of following structures: 1,4,7,10-tetraazacyclododecane-1,4,7,10-tetracarboxylic acid (DOTA), 1,4,7-triazacyclononane-1,4,7-tricarboxylic acid (NOTA), 4-[[[(1R,2R)-2-[bis(carboxymethyl)amino]cyclohexyl] (carboxymethyl)amino]methyl]-phenylacetic acid (H3RESCA), or a combination thereof.

In some embodiments, a spacer group is further included between Lk and T, in which the spacer group is independently selected from a single bond, an optionally substituted C₁-C₂₀ alkylene group, an optionally substituted C₁-C₂₀ alkenylene group, an optionally substituted C₁-C₂₀ alkynylene group, an optionally substituted C₁-C₂₀ heteroalkylene group, an optionally substituted C₃-C₂₀ cycloalkylene group, an optionally substituted C₃-C₂₀ heterocycloalkylene group, an optionally substituted C₅-C₂₀ arylene group, an optionally substituted C₅-C₂₀ heteroarylene group, ketone group, -S-, -(C=S)-, -O-, -NH-, -NHCO-, polypeptides (e.g., valine-citrulline (Val-Cit), valine-alanine (Val-Ala), glycine-glycine-phenylalanine-glycine (Gly-Gly-Phe-Gly), phenylalanine-lysine (Phe-Lys), alanine-alanine-asparagine (Ala-Ala-Asn), polyglycine (Gly)ᵣ, r being an integer from 1 to 10), polyethylene glycol ((PEG)ᵣ, in which r is an integer from 1 to 10), polypropylene glycol, polyoxyalkylene, copolymerization groups of polyethylene glycol and polypropylene glycol, or a combination thereof. Preferably, the spacer group is independently selected from a single bond, an optionally substituted C₁-C₁₀ alkylene group, an optionally substituted C₁-C₁₀ alkenylene group, an optionally substituted C₁-C₁₀ alkynylene group, an optionally substituted C₁-C₁₀ heteroalkylene group, an optionally substituted C₃-C₁₀ cycloalkylene group, an optionally substituted C₃-C₁₀ heterocycloalkylene group, an optionally substituted C₅-C₁₀ arylene group, an optionally substituted C₅-C₁₀ heteroarylene group, ketone group, -S-, -(C=S)-, -O-, -NH-, -NHCO-, -(CH₂CH₂O)ᵣ-(also referred to as -(PEG)ᵣ-), -(CH₂CH₂CH₂O)ᵣ-, valine-citrulline (Val-Cit), valine-alanine (Val-Ala), glycine-glycine-phenylalanine-glycine (Gly-Gly-Phe-Gly), phenylalanine-lysine (Phe-Lys), alanine-alanine-asparagine (Ala-Ala-Asn), -(Gly)ᵣ-, or a combination thereof, in which r is an integer from 1 to 10. More preferably, the spacer group is independently selected from a single bond, -(PEG)ᵣ-, -(Gly)ᵣ-, or a combination thereof, in which r is an integer from 1 to 10.

In some embodiments, Lk is independently selected from a single bond, p-isothiocyanate phenyl, amido group or maleimidyl; Y includes a reactive moiety and a spacer group, in which the spacer group is independently selected from a single bond, an optionally substituted C₁-C₁₀ alkylene group, an optionally substituted C₁-C₁₀ alkenylene group, an optionally substituted C₁-C₁₀ alkynylene group, an optionally substituted C₁-C₁₀ heteroalkylene group, an optionally substituted C₃-C₁₀ cycloalkylene group, an optionally substituted C₃-C₁₀ heterocycloalkylene group, an optionally substituted C₅-C₁₀ arylene group, an optionally substituted C₅-C₁₀ heteroarylene group, ketone group, -S-, -(C=S)-, -O-, -NH-, -NHCO-, -(PEG)ᵣ-, valine-citrulline (Val-Cit), valine-alanine (Val-Ala), glycine-glycine-phenylalanine-glycine (Gly-Gly-Phe-Gly), phenylalanine-lysine (Phe-Lys), alanine-alanine-asparagine (Ala-Ala-Asn), -(Gly)ᵣ-, or a combination thereof, in which r is an integer from 1 to 10; the reactive moiety is linked to Ab and is independently selected from a single bond, p-isothiocyanate phenyl, maleimidyl, amido group, or a combination thereof; and T is independently selected from a single bond or a ligand derived from any of following structures: deferoxamine (DFO), 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), 4-[[[(1R,2R)-2-[bis(carboxymethyl)amino]cyclohexyl] (carboxymethyl)amino]methyl]-phenylacetic acid (H3RESCA), 1,4,7,10-tetraazacyclododecane-1,4,7,10-tetrakis (methylene phosphonic acid) (DOTP), or 1,4,7- triazacyclononane-1,4,7-tris (methylene phosphonic acid) (NOTP).

In some embodiments, Lk is independently selected from a single bond, p-isothiocyanate phenyl, or amido group; Y includes a reactive moiety and a spacer group, in which the spacer group is independently selected from a single bond, -(PEG)ᵣ-, -(Gly)ᵣ-, or a combination thereof, in which r is an integer from 1 to 10; the reactive moiety is linked to Ab and is independently selected from a single bond, p-isothiocyanate phenyl, maleimidyl, amido group, or a combination thereof; and T is independently selected from a single bond or a ligand derived from any of following structures: 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), 6-hydrazinonicotinic acid (HYNIC), or 4-[[[(1R,2R)-2-[bis(carboxymethyl)amino]cyclohexyl] (carboxymethyl)amino]methyl]-phenylacetic acid (H3RESCA).

In some embodiments, Lk is independently selected from p-isothiocyanate phenyl, or amido group; Y is a single bond; and T is independently selected from a ligand derived from any of following structures: 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), 6-hydrazinonicotinic acid (HYNIC), or 4-[[[(1R,2R)-2-[bis(carboxymethyl)amino]cyclohexyl] (carboxymethyl)amino]methyl]-phenylacetic acid (H3RESCA).

In some embodiments, Lk is p-isothiocyanate phenyl; Y includes a reactive moiety and a spacer group, in which the spacer group is independently selected from a single bond, -(PEG)ᵣ-, -(Gly)ᵣ-, or a combination thereof, in which r is an integer from 1 to 10; the reactive moiety is linked to Ab and is independently selected from a single bond, p-isothiocyanate phenyl, maleimidyl, amido group, or a combination thereof; and T is independently selected from or derived from a ligand with any of following structures: 1,4,7,10-tetraazacyclododecane-1,4,7,10-tetracarboxylic acid (DOTA), 1,4,7-triazacyclononane-1,4,7-tricarboxylic acid (NOTA), or diethylenetriamine pentaacetic acid (DTPA).

In some embodiments, Lk is p-isothiocyanate phenyl; Y is a single bond, and T is independently selected from or derived from a ligand with any of following structures: 1,4,7,10-tetraazacyclododecane-1,4,7,10-tetracarboxylic acid (DOTA), 1,4,7-triazacyclononane-1,4,7-tricarboxylic acid (NOTA), or diethylenetriamine pentaacetic acid (DTPA).

In some embodiments, Lk is amido group, Y includes a reactive moiety and a spacer group, in which the spacer group is independently selected from a single bond, -(PEG)ᵣ-, -(Gly)ᵣ-, or a combination thereof, in which r is an integer from 1 to 10; the reactive moiety is linked to Ab and is independently selected from a single bond, p-isothiocyanate phenyl, maleimidyl, amido group, or a combination thereof; and T is independently selected from or derived from a ligand with any of following structures: mercaptoacetyl triglycine (MAG3), or 4-[[[(1R,2R)-2-[bis(carboxymethyl)amino]cyclohexyl] (carboxymethyl)amino]methyl]-phenylacetic acid (H3RESCA).

In some embodiments, Lk is amido group; Y is a single bond, and T is independently selected from a ligand derived from any one of the following structures: mercaptoacetyl triglycine (MAG3), or 4-[[[(1R,2R)-2-[bis(carboxymethyl)amino]cyclohexyl] (carboxymethyl)amino]methyl]-phenylacetic acid (H3RESCA).

In some embodiments, L is independently selected from a ligand derived from any one of the following structures: 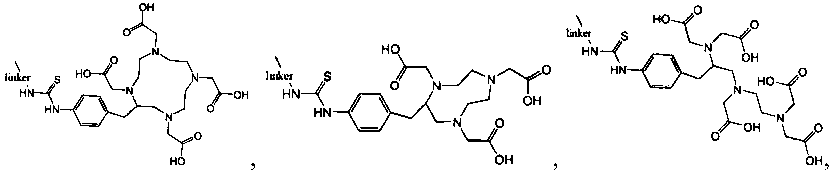 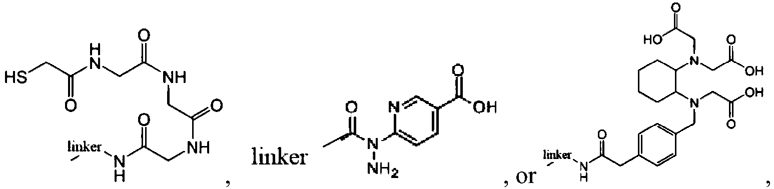 where the linker includes a reactive moiety and a spacer group, in which the spacer group is independently selected from a single bond, -(PEG)ᵣ-, -(Gly)ᵣ-, or a combination thereof, in which r is an integer from 1 to 10; the reactive moiety is linked to Ab and is independently selected from a single bond, p-isothiocyanate phenyl, maleimidyl, amido group, or a combination thereof. Preferably, the linker is a single bond.

In some embodiments, M is selected from a diagnostic radionuclide or a therapeutic radionuclide. In some embodiments, the diagnostic radionuclide is selected from ⁶⁸Ga, ¹⁸F-Al, ⁶⁴Cu, ^{99m}Tc, ⁸⁹Zr, ⁴⁴Sc, ¹¹¹In, ⁴⁵Ti, ⁵²Mn, ⁵⁹Fe, ^{94m}Tc, ⁶⁷Ga, ⁷¹As, ⁷²As, ^{82m}Rb, ⁸⁶Y, ¹³¹I, Gd, Mn, or a combination thereof. In some embodiments, the therapeutic radionuclide is selected from¹⁷⁷Lu, ⁹⁰Y, ¹⁵³Sm, ⁶⁷Cu, ⁸⁹Sr, ¹⁶⁶Ho, ¹⁷⁷Yb, ⁴⁷Sc, ¹⁸⁶Re, ¹⁸⁸Re, ²¹²Bi, ²¹³Bi, ¹⁴⁹Pm, ²¹²Pb, ²¹¹At, ²²³Ra, ²²⁵Ac, ²²⁷Th, or a combination thereof.

In some embodiments, M is selected from ⁶⁸Ga, ¹⁸F-Al, ^{99m}Tc, ¹⁷⁷Lu, ¹³¹I, or a combination thereof.

In some embodiments, L-M is selected from following structures: 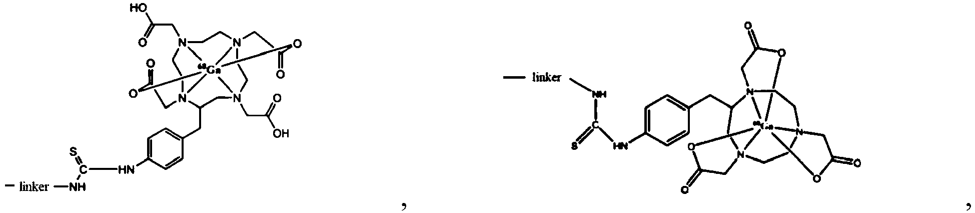 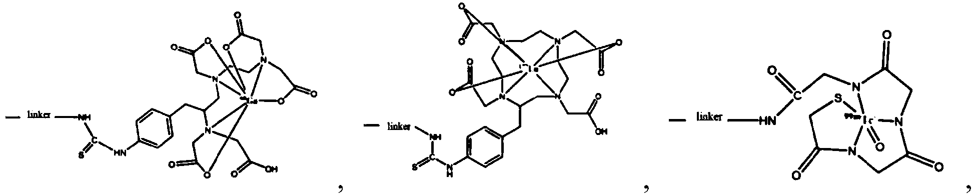 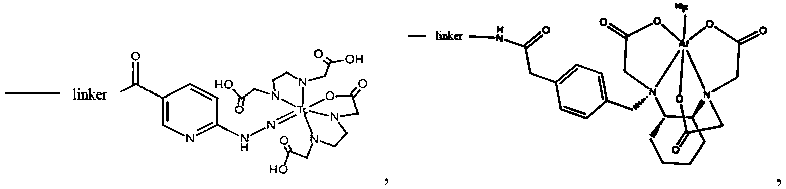 or -linker-131I,
where the linker includes a reactive moiety and a spacer group, in which the spacer group is independently selected from a single bond, -(PEG)ᵣ-, -(Gly)ᵣ-, or a combination thereof, in which r is an integer from 1 to 10; the reactive moiety is linked to Ab and is independently selected from a single bond, p-isothiocyanate phenyl, maleimidyl, amido group, or a combination thereof. Preferably, the linker is a single bond.

In some embodiments, Ab is anti-GPC3 antibody or antigen binding fragment thereof. In some embodiments, anti-GPC3 antibody or antigen binding fragment thereof is a monoclonal antibody, polyclonal antibody, single-chain antibody, chimeric antibody, humanized antibody, or fully human antibody. In some embodiments, anti-GPC3 antibody or antigen binding fragment thereof is selected from a monoclonal antibody (mAb), Fab, Fab', F(ab')₂, Fv, scFv, scFv-Fc, or single-domain antibody.

In some embodiments, Ab is a monoclonal antibody, Fab fragment, Fab' fragment, or F(ab')₂ fragment, or scFv fragment.

In some embodiments, Ab contains at least one, two, three, four, five, or six CDR sequences. CDR sequence has at least 80%, 90%, 95%, 98%, 99%, or 100% sequence identity to at least one of SEQ ID NO: 1, SEQ ID NO: 2, SEQ ID NO: 3, SEQ ID NO: 4, SEQ ID NO: 5, or SEQ ID NO: 6.

In some embodiments, Ab contains a heavy chain and fragment thereof having HCDR1, HCDR2, and HCDR3. HCDR1 contains an amino acid sequence having at least 80%, 90%, 95%, 98%, 99%, or 100% sequence identity to the amino acid sequence shown as SEQ ID NO: 1. Preferably, the amino acid sequence of HCDR1 is shown as SEQ ID NO: 1. HCDR2 contains an amino acid sequence having at least 80%, 90%, 95%, 98%, 99%, or 100% sequence identity to the amino acid sequence shown as SEQ ID NO: 2. Preferably, the amino acid sequence of HCDR2 is shown as SEQ ID NO: 2. HCDR3 contains an amino acid sequence having at least 80%, 90%, 95%, 98%, 99%, or 100% sequence identity to the amino acid sequence shown as SEQ ID NO: 3. Preferably, the amino acid sequence of HCDR3 is shown as SEQ ID NO: 3.

In some embodiments, Ab contains a heavy chain variable region (VH). VH contains an amino acid sequence having at least 80%, 90%, 95%, 98%, 99%, or 100% sequence identity to the amino acid sequence shown as SEQ ID NO: 7. Preferably, the amino acid sequence of VH is shown as SEQ ID NO: 7.

In some embodiments, Ab contains a heavy chain amino acid sequence having at least 80%, 90%, 95%, 98%, 99%, or 100% sequence identity to the amino acid sequence shown as SEQ ID NO: 9. Preferably, Ab contains a heavy chain amino acid sequence shown as SEQ ID NO: 9.

In some embodiments, Ab contains a light chain and fragment thereof having LCDR1, LCDR2, and LCDR3. LCDR1 contains an amino acid sequence having at least 80%, 90%, 95%, 98%, 99%, or 100% sequence identity to the amino acid sequence shown as SEQ ID NO: 4. Preferably, the amino acid sequence of LCDR1 is shown as SEQ ID NO: 4. LCDR2 contains an amino acid sequence having at least 80%, 90%, 95%, 98%, 99%, or 100% sequence identity to the amino acid sequence shown as SEQ ID NO: 5. Preferably, the amino acid sequence of LCDR2 is shown as SEQ ID NO: 5. LCDR3 contains an amino acid sequence having at least 80%, 90%, 95%, 98%, 99%, or 100% sequence identity to the amino acid sequence shown as SEQ ID NO: 6.

Preferably, the amino acid sequence of LCDR3 is shown as SEQ ID NO: 6.

In some embodiments, Ab contains a light chain variable region (VL). VL contains an amino acid sequence having at least 80%, 90%, 95%, 98%, 99%, or 100% sequence identity to the amino acid sequence shown as SEQ ID NO: 8. Preferably, the amino acid sequence of VL is shown as SEQ ID NO: 8.

In some embodiments, Ab contains a light chain amino acid sequence having at least 80%, 90%, 95%, 98%, 99%, or 100% sequence identity to the amino acid sequence shown as SEQ ID NO: 10. Preferably, Ab contains a light chain amino acid sequence shown as SEQ ID NO: 10.

In some embodiments, Ab is Fab fragment. The Fab fragment contains a heavy chain amino acid sequence having at least 80%, 90%, 95%, 98%, 99%, or 100% sequence identity to the amino acid sequence shown as SEQ ID NO: 11, preferably, a heavy chain amino acid sequence shown as SEQ ID NO: 11, and contains a light chain amino acid sequence having at least 80%, 90%, 95%, 98%, 99%, or 100% sequence identity to the amino acid sequence shown as SEQ ID NO: 10, preferably a light chain amino acid sequence shown as SEQ ID NO: 10.

In some embodiments, Ab is scFv and contains an amino acid sequence having at least 80%, 90%, 95%, 98%, 99%, or 100% sequence identity to the amino acid sequence shown as SEQ ID NO: 12. Preferably Ab contains an amino acid sequence of SEQ ID NO: 12.

In some embodiments, the radiolabeled molecular probe provided in the disclosure is selected from following structures:

| | | |
|---|---|---|
| 1. ⁶⁸Ga-DOTA-aGPC3-mAb | 2. ⁶⁸Ga-DOTA-aGPC3-Fab | 3. ⁶⁸Ga-DOTA-aGPC3-scFv |
| 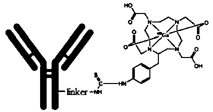 | 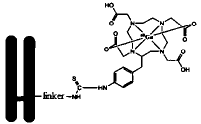 | 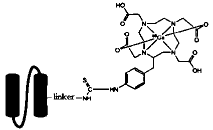 |
| 4. ⁶⁸Ga-NOTA-aGPC3-mAb | 5. ⁶⁸Ga-NOTA-aGPC3-Fab | 6. ⁶⁸Ga-NOTA-aGPC3-scFv |
| 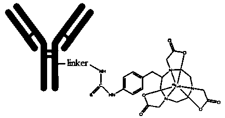 | 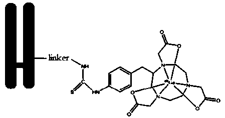 | 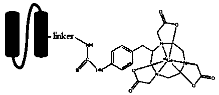 |
| 7. ⁶⁸Ga-DOTA-aGPC3-mAb | 8. ⁶⁸Ga-DOTA-aGPC3-Fab | 9. ⁸⁸Ga-DOTA-aGPC3-scFv |
| 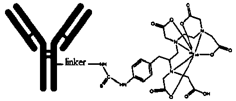 | 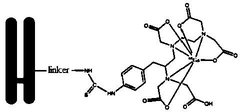 | 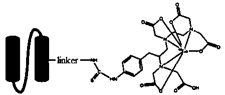 |
| 10. ¹⁷⁷Lu-DOTA-aGPC3-mAb | 11. ¹⁷⁷Lu-DOTA-aGPC3-Fab | 12. ¹⁷⁷Lu-DOTA-aGPC3-scFv |
| 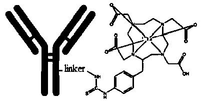 | 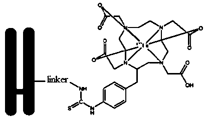 | 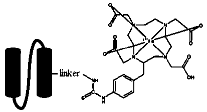 |
| 13. ^{99m}TC-MAG3-aGPC3-mAb | 14. ^{99m}TC-MAG3-aGPC3-Fab | 15. ^{99m}TC-MAG3-aGPC3-scFv |
| 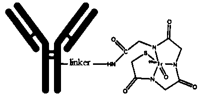 | 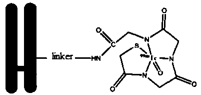 | 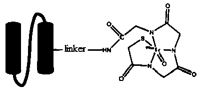 |
| 16. Al¹⁸F-RESCA-aGPC3-mAb | 17. Al¹⁸F-RESCA-aGPC3-Fab | 18. Al¹⁸F-RESCA-aGPC3-scFv |
| 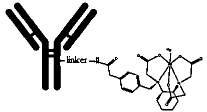 | 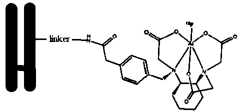 | 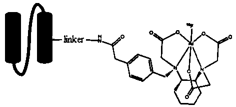 |
| 19. ¹³¹I-aGPC3-mAb | 20. ¹³¹I-aGPC3-Fab | 21. ¹³¹I-aGPC3-scFv |
| 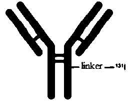 | 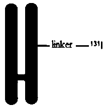 | 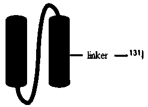 |
| 22. ^{99m}TC-HYNIC-aGPC3-mAb | 23. ⁹⁹mTC-HYNIC-aGPC3-Fab | 24. ^{99m}TC-HYNIC-aGPC3-scFv |
| 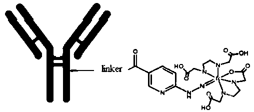 | 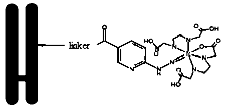 | 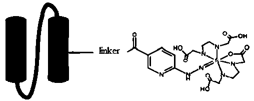 |

where the linker includes a reactive moiety and a spacer group, in which the spacer group is independently selected from a single bond, -(PEG)ᵣ-, -(Gly)ᵣ-, or a combination thereof, in which r is an integer from 1 to 10; the reactive moiety is linked to Ab and is independently selected from a single bond, p-isothiocyanate phenyl, maleimidyl, amido group, or a combination thereof.

Preferably, the linker is a single bond.

### III. Methods for using radiolabeled GPC3 molecular probes

In some aspects, the present disclosure provides diagnostic and therapeutic methods using the radiolabeled molecular probes of the disclosure.

According to one aspect, the present disclosure provides a method for detecting GPC3 in tissue, including: administering a radiolabeled molecular probe provided by the disclosure to the tissue; and visualizing GPC3 expression via single photon emission computed tomography (SPECT), positron emission tomography (PET) imaging, or magnetic resonance imaging (MRI). In some embodiments, the tissue includes cells or cell lines. In some embodiments, the tissue is present in a subject, and the subject is a mammal. In some embodiments, the subject is human. In some embodiments, the subject has a disease or disorder selected from a group consisting of: cancer, infectious diseases, and inflammatory diseases. In one embodiment, the subject has cancer.

According to one aspect, the present disclosure provides a method for measuring response to treatment, in which the response to treatment is measured by measuring inflammation. According to this aspect, the method includes: administering a radiolabeled molecular probe provided herein to a subject in need thereof; and visualizing GPC3 expression via SPECT, PET, or MRI. In some embodiments, inflammation is present in the subject's tumor. In some embodiments, increased GPC3 expression is correlated with increased inflammation in the tumor. According to one aspect, the present disclosure provides a method for determining whether a patient is suitable for antitumor therapy including a GPC3 inhibitor, including: selecting a patient with a solid tumor; administering a radiolabeled molecular probe of the present disclosure; and localizing the administered radiolabeled molecular probe in the tumor via SPECT, PET, or MRI; in which the presence of the radiolabeled molecular probe in the tumor indicates that the patient is suitable for antitumor therapy including a GPC3 inhibitor.

According to one aspect, the present disclosure provides a method for detecting GPC3-positive tumors in subjects. According to this aspect, the method includes: selecting a subject with a solid tumor; administering a radiolabeled molecular probe of the disclosure to the subject; and localizing the radiolabeled molecular probe via SPECT, PET, or MRI, in which the presence of the radiolabeled molecular probe in the tumor indicates that the tumor a GPC3-positive.

According to one aspect, the present disclosure provides a therapeutic method including: administering a therapeutically effective amount of the radiolabeled molecular probe of the disclosure to a subject in need thereof. The molecular probe may be administered by any suitable routes known to a person skilled in the art, including intravenous, intra-arterial, intraperitoneal, intramuscular, subcutaneous, intrapleural, or intratumoral injection. In some embodiments, the therapeutic method of the present disclosure includes: administering a therapeutically effective amount of a pharmaceutical composition to a subject in need thereof; in which the pharmaceutical composition includes the radiolabeled molecular probe of the present disclosure and a pharmaceutically acceptable carrier.

As used herein, the expression "a subject in need thereof" means a human or non-human mammal that exhibits one or more symptoms or indications of cancer, and/or who has been diagnosed with cancer, including a solid tumor and wneeds treatment for the same. In many embodiments, the term "subject" may be interchangeably used with the term "patient". For example, a human subject may be diagnosed with a primary or a metastatic tumor and/or with one or more symptoms or indications including, but not limited to, unexplained weight loss, general weakness, persistent fatigue, loss of appetite, fever, night sweats, bone pain, shortness of breath, swollen abdomen, chest pain/pressure, enlargement of spleen, and elevation in the level of a cancer-related biomarker. The expression includes subjects with primary or established tumors. In specific embodiments, the expression includes human subjects that have and/or need treatment for a solid tumor, e.g., colon cancer, breast cancer, lung cancer, prostate cancer, skin cancer, liver cancer, bone cancer, ovarian cancer, cervical cancer, pancreatic cancer, head and neck cancer, and brain cancer. The term includes subjects with primary or metastatic tumors (advanced malignancies). In some embodiments, the expression "a subject in need thereof" includes patients with a solid tumor that is resistant to or refractory to or is inadequately controlled by prior therapy (e.g., treatment with an anti-cancer agent). For example, the expression includes subjects who have been treated with one or more lines of prior therapy such as treatment with chemotherapy (e.g., carboplatin or docetaxel). In some embodiments, the expression "a subject in need thereof" includes patients with a solid tumor which has been treated with one or more lines of prior therapy but which has subsequently relapsed or metastasized.

In some embodiments, the methods of the disclosure are used in a subject with a solid tumor. The terms "tumor", "cancer" and "malignancy" are interchangeably used herein. As used herein, the term "solid tumor" refers to an abnormal mass of tissue that usually does not contain cysts or liquid areas. Solid tumors may be benign (not cancer) or malignant (cancer). For the purposes of the present disclosure, the term "solid tumor" means malignant solid tumors. The term includes different types of solid tumors named for the cell types that form them, viz. sarcomas, carcinomas and lymphomas. In some embodiments, the term "solid tumor" includes cancers including, but not limited to, colorectal cancer, ovarian cancer, prostate cancer, breast cancer, brain cancer, cervical cancer, bladder cancer, anal cancer, uterine cancer, colon cancer, liver cancer, pancreatic cancer, lung cancer, endometrial cancer, bone cancer, testicular cancer, skin cancer, kidney cancer, stomach cancer, esophageal cancer, head and neck cancer, salivary gland cancer, and myeloma.

### IV. Examples

The following description will elaborate on the present disclosure in detail through specific embodiments and implementation, thereby clearly illustrating its advantages and various effects. A person skilled in the art should understand that these specific embodiments and implementation are provided to illustrate the disclosure and are not intended to limit it.

### Example 1. Preparation of mAb, Fab, and scFv targeting GPC3

Variable region VH and VL sequences of mAb, Fab, and scFv antibodies targeting GPC3 are derived from the variable regions VH and VL of P17-8E12 of patent application PCT/US2022/077807. The entire of which are hereby incorporated by reference.

The variable regions VH and VL of P17-8E12 were cloned into mammalian IgG1 expression vector, in which the expression vector provides the constant regions of human IgG1, including CL, CH1-CH2-CH3. The final sequences of the light chain and heavy chain of mAb targeting GPC3 are shown in SEQ ID NO:10 and SEQ ID NO:9, respectively. The mAb antibody targeting GPC3 (aGPC3-mAb) was expressed in Expi293F cells using standard transient transfection protocols purified from cell culture supernatants using MabSelect Prism A.

The variable region sequences VH and VL of P17-8E12 were linked via a linker (G4S)₃ and six histidine residues were introduced at the C-terminus to obtain the complete sequence of scFv targeting GPC3 (SEQ ID NO:12). This sequence was cloned into a mammalian expression vector. The svFc antibody targeting GPC3 (aGPC3-scFv) was expressed in Expi293F cells using standard transient transfection protocols and purified from cell culture supernatants using Ni Sepharose High Performance.

The variable region sequences VH and VL of P17-8E12 were cloned into mammalian Fab expression vector, the expression vector provides the constant regions of human Fab, including CL, CH1. The final sequences of the light chain and heavy chain of Fab targeting GPC3 are shown in SEQ ID NO:10 and SEQ ID NO:11, respectively. The Fab antibody targeting GPC3 (aGPC3-Fab) was expressed in ExpiCHO-S cells using standard transient transfection protocols and purified from cell culture supernatants using MabSelect Prism A.

### Example 2. Preparation of ⁶⁸Ga-NOTA-aGPC3-Fab

### 2.1 Preparation of NOTA-aGPC3-Fab

Antibody aGPC3-Fab was purified via PD-10 desalting chromatography column. The purity of antibody aGPC3-Fab was determined by size exclusion chromatography (SEC-HPLC). 20 nmol of purified antibody aGPC3-Fab was taken and the pH was adjusted to 8.5-9.0 using Na₂CO₃ (0.1 M, pH 9.0). Then, p-SCN-Bn-NOTA (dissolved in a small volume of DMSO) was added at a molar ratio of 20-30 times relative to the antibody, then reacted overnight at 4°C in the dark to synthesize NOTA-aGPC3-Fab. The excess unreacted NOTA was removed by purification via PD-10 desalting chromatography column. The purified antibody was concentrated using an ultrafiltration centrifuge tube (Millipore 0.5 mL/3 Kd) under conditions of 4°C, 13,000 x g, for 30 min, and then stored in a refrigerator at 4°C. The concentrated antibody solution remained stable for at least 3 months when stored at 4°C.

### 2.2 Radiolabeling with ⁶⁸Ga

The ⁶⁸Ge/⁶⁸Ga generator was eluted with 4 mL of 0.05 M HCl. The pH of ⁶⁸GaCl₃ was adjusted to 4.0-4.5 using sodium acetate (0.25 M, pH 6.8). Purified NOTA-aGPC3-Fab (3 nmol) was thoroughly mixed with ⁶⁸GaCl₃ (111 MBq, 3 mCi) and reacted at 37°C for 20 min. The labeling mixture was purified via PD-10 desalting chromatography column. The radiochemical purity of the product was determined via radio-instant thin-layer chromatography (ITLC) scanner, with a citrate-citric acid buffer (pH 4.0) used as the developing solvent.

### 2.3 Radiochemical Characterization of ⁶⁸Ga-NOTA-aGPC3-Fab

A sample of 3.70 MBq ⁶⁸Ga-NOTA-aGPC3-Fab was added to 100 µL of PBS and incubated at room temperature for 1 h. The PBS sample was then directly subjected to SEC-HPLC analysis under the same elution conditions as described in Example 2.1 to determine the radiochemical purity.

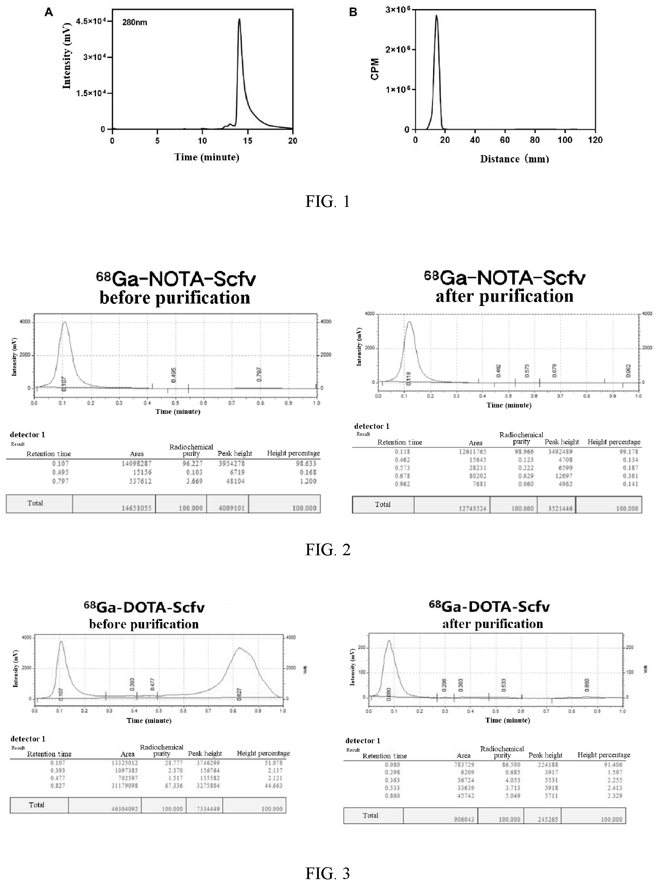

The prepared ⁶⁸Ga-NOTA-aGPC3-Fab was determined and characterized by analytical SEC-HPLC. The results from SEC-HPLC indicated that the purity of the antibody aGPC3-Fab was greater than 99% (FIG. 1A). The radiochemical purity of ⁶⁸Ga-NOTA-aGPC3-Fab, as determined by ITLC, was greater than 99% (FIG. 1B).

### Example 3. Preparation of ⁶⁸Ga-NOTA-aGPC3-scFv

### 3.1 Preparation of NOTA-aGPC3-scFv

Antibody aGPC3-scFv was purified via PD-10 desalting chromatography column. The purity of antibody aGPC3-scFv was determined via size exclusion chromatography (SEC-HPLC). 20 nmol of purified antibody aGPC3-scFv was taken and the pH was adjusted to 8.5-9.0 using Na₂CO₃ (0.1 M, pH 9.0). Then, p-SCN-Bn-NOTA (dissolved in a small volume of DMSO) was added at a molar ratio of 15 times relative to the antibody, and then reacted overnight at 4°C in the dark to synthesize NOTA-aGPC3-scFv. The excess unreacted NOTA was removed by purification via PD-10 desalting chromatography column. The purified antibody was concentrated using an ultrafiltration centrifuge tube (Millipore 0.5 mL/3 kd) under conditions of 4°C, 13,000 x g, for 30 min, and then stored in a refrigerator at 4°C.

### 3.2 Radiolabeling with ⁶⁸Ga

⁶⁸GaCl₃ was eluted from the ⁶⁸Ge/⁶⁸Ga radionuclide generator (ITM Medical Isotopes GmbH, Germany) using 4 mL of 0.05 M HCl solution for subsequent radiolabeling. For the radiolabeling reaction, 740 MBq of ⁶⁸GaCl₃ was adjusted to pH 4.0 with 0.25 M sodium acetate solution, followed by the addition of 5 nmol of anti-GPC3 antibody NOTA-aGPC3-scFv. The reaction mixture was stirred at 35°C for 30 minutes and subsequently purified via PD-10 desalting column (GE, USA). The labeling efficiency before PD-10 purification and the radiochemical purity after purification were assessed via radio-instant thin-layer chromatography (ITLC) with 0.5 M EDTA as the mobile phase.

### 3.3 Radiochemical Characterization of ⁶⁸Ga-NOTA-aGPC3-scFv

A sample of 3.70 MBq ⁶⁸Ga-NOTA-aGPC3-scFv was added to 100 µL of FBS and incubated at room temperature for 2 h. The FBS sample was then directly subjected to SEC-HPLC analysis to determine the radiochemical purity, in which the elution conditions were the same as those described in Example 2.

The labeling efficiency before PD-10 purification and the radiochemical purity after purification of the prepared ⁶⁸Ga-NOTA-aGPC3-scFv were assessed via radio-instant thin-layer chromatography (ITLC) using 0.5 M EDTA as the mobile phase. The ITLC results (FIG. 2) indicated the successful preparation of the PET probe ⁶⁸Ga-NOTA-aGPC3-scFv, with a labeling efficiency of approximately 67.2%. SEC-HPLC results indicated that the radiochemical purity is greater than 98% after purification via PD-10 desalting column.

### Example 4. Preparation of ⁶⁸Ga-DOTA-aGPC3-scFv

### 4.1 Preparation of DOTA-aGPC3-scFv

50 nmol of aGPC3-scFv antibody was taken and the pH was adjusted to 8.5-9.0 using Na₂CO₃ (0.1 M, pH 9.0). Then, p-SCN-Bn-DOTA (dissolved in a small volume of DMSO) was added at a molar ratio of 20 times relative to the antibody, then reacted at 25°C in the dark for 4 h to synthesize DOTA-aGPC3-scFv. The excess unreacted DOTA was removed by purification via PD-10 desalting chromatography column. The purified antibody was concentrated using an ultrafiltration centrifuge tube (Millipore 0.5 mL/3 Kd) under conditions of 4°C, 13,000 x g, for 30 min, and then stored in a refrigerator at 4°C. The concentrated antibody solution remained stable for at least 3 months when stored at 4°C.

### 4.2 Radiolabeling with ⁶⁸Ga

⁶⁸GaCl₃ was eluted from the ⁶⁸Ge/⁶⁸Ga radionuclide generator (ITM Medical Isotopes GmbH, Germany) using 4 mL of 0.05 M HCl solution for subsequent radiolabeling. For the radiolabeling reaction, 740 MBq of ⁶⁸GaCl₃ was adjusted to pH 4.0 with 0.25 M sodium acetate solution, followed by the addition of 30 nmol of the anti-GPC3 antibody DOTA-aGPC3-scFv. The mixture was shaken and reacted at 35°C for 30 minutes and subsequently purified via PD-10 desalting column (GE, USA). The labeling efficiency before PD-10 purification and the radiochemical purity after purification were assessed via radio-instant thin-layer chromatography (ITLC) with 0.5 M EDTA as the mobile phase.

### 4.3 Radiochemical Characterization of ⁶⁸Ga-DOTA-aGPC3-scFv

The labeling efficiency before PD-10 purification and the radiochemical purity after purification of the prepared ⁶⁸Ga-DOTA-aGPC3-scFv were assessed via radio-instant thin-layer chromatography (ITLC) with 0.5 M EDTA as the mobile phase.

The prepared ⁶⁸Ga-NOTA-aGPC3-Fab was determined and characterized by analytical SEC-HPLC. The results showed the successful preparation of the PET probe ⁶⁸Ga-DOTA-aGPC3-scFv. ITLC showed a labeling efficiency of approximately 28.78% (FIG. 3). SEC-HPLC showed that the radiochemical purity was greater than 90% after purification of the labeling mixture via PD-10 desalting column.

### Example 5. Preparation of ⁶⁸Ga-DOTA-aGPC3-Fab

### 5.1 Preparation of DOTA-aGPC3-Fab

50 nmol of aGPC3-Fab antibody was taken and the pH was adjusted to 8.5-9.0 using Na₂CO₃ (0.1 M, pH 9.0). Then, p-SCN-Bn-DOTA (dissolved in a small volume of DMSO) was added at a molar ratio of 20 times relative to the antibody, then reacted at 25°C in the dark for 4 h to synthesize NOTA-aGPC3-Fab. The excess unreacted NOTA was removed by purification via PD-10 desalting chromatography column. The purified antibody was concentrated using an ultrafiltration centrifuge tube (Millipore 0.5 mL/3 Kd) under conditions of 4°C, 13,000 x g, for 30 min, and then stored in a refrigerator at 4°C. The concentrated antibody solution remained stable for at least 3 months when stored at 4°C.

### 5.2 Radiolabeling with ⁶⁸Ga

⁶⁸GaCl₃ was eluted from the ⁶⁸Ge/⁶⁸Ga radionuclide generator (ITM Medical Isotopes GmbH, Germany) using 4 mL of 0.05 M HCl solution for subsequent radiolabeling. For the radiolabeling reaction, 740 MBq of ⁶⁸GaCl₃ was adjusted to pH 4.0 with 0.25 M sodium acetate solution, followed by the addition of 30 nmol of the anti-GPC3 antibody DOTA-aGPC3-Fab. The reaction mixture was shaken and reacted at 35°C for 30 minutes and subsequently purified via PD-10 desalting column (GE, USA). The labeling efficiency before PD-10 purification and the radiochemical purity after purification were assessed via radio-instant thin-layer chromatography (ITLC) with 0.5 M EDTA as the mobile phase.

### 5.3 Radiochemical Characterization of ⁶⁸Ga-DOTA-aGPC3-Fab

The labeling efficiency before PD-10 purification and the radiochemical purity after purification of the prepared ⁶⁸Ga-DOTA-aGPC3-Fab were assessed via thin-layer chromatography (TLC) with 0.5 M EDTA as the mobile phase.

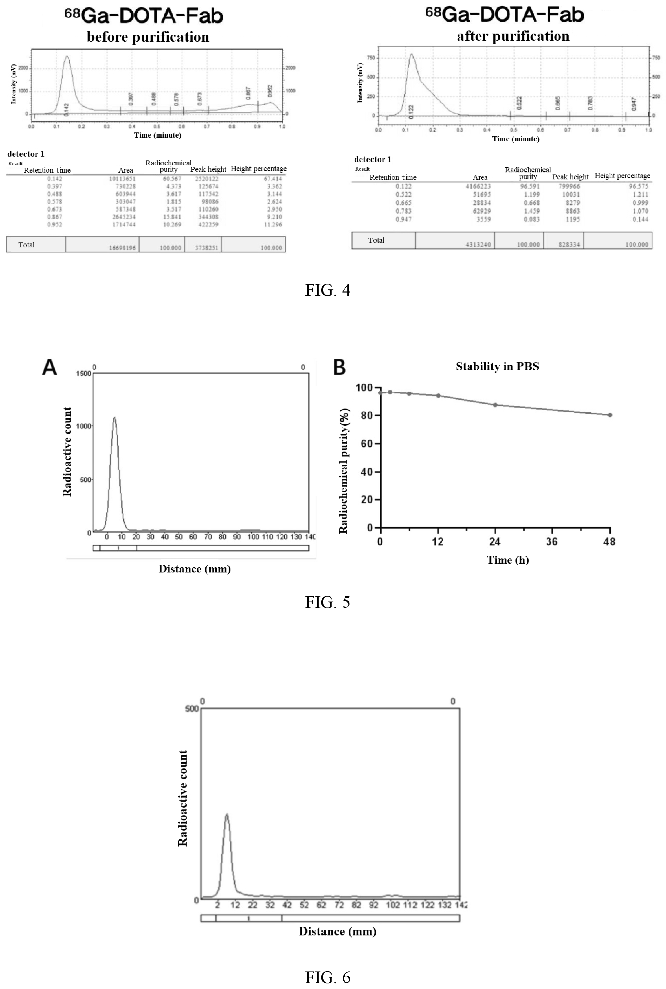

The results showed the successful preparation of the PET probe ⁶⁸Ga-DOTA-aGPC3-Fab. ITLC showed a labeling efficiency of approximately 67.41% (FIG. 4). SEC-HPLC results showed that the radiochemical purity was greater than 99%.

### Example 6. Preparation of¹⁸FAl-H₃RESCA-aGPC3-scFv

### 6.1 Preparation of H₃RESCA-aGPC3-scFv

Antibody aGPC3-scFv was purified via PD-10 desalting chromatography column. The purity of antibody aGPC3-scFv was determined via size exclusion chromatography (SEC-HPLC). 20 nmol of purified antibody aGPC3-scFv was taken, and (±)-H3RESCA-TFP (dissolved in a small volume of DMSO) was added at a molar ratio of 12 times relative to the antibody. The pH of the reaction solution was adjusted to 8.5-9.0 using a small volume of Na₂CO₃ solution (0.1 M), and then reacted at room temperature in the dark with shaking for 2 h to synthesize H₃RESCA-aGPC3-scFv. Excess unreacted (±)-H₃RESCA-TFP was removed by purification via PD-10 desalting chromatography column, with 0.1 M ammonium acetate buffer (pH 4.4-4.6) used as both the equilibration solution and elution solution. The purified antibody was concentrated using an ultrafiltration centrifuge tube (Millipore 0.5 mL/3 KD) under conditions of 4°C, 13,000 x g, for 30 min, and stored in a refrigerator at 4°C.

### 6.2 Radiolabeling with ¹⁸F

A batch of high-activity fluoride water (> 37 MBq) was processed via activated QMA cartridge. The ¹⁸F ions were eluted with metal-free saline. To the resulting solution, an appropriate volume of 2 mM AlCl₃ solution was added (20 nmol AlCl₃ per 1.1-2.2 GBq of ¹⁸F⁻) and incubated at room temperature for 5 min. Subsequently, 20 nmol of H₃RESCA-aGPC3-scFv was added, and then reacted under shaking at room temperature for 15 min. The product was purified via PD-10 desalting column with saline used as both the equilibration solution and elution solution, to obtain ¹⁸F Al-H₃RESCA-aGPC3-scFv. The labeling efficiency and radiochemical purity were determined via radio-instant thin-layer chromatography (ITLC) with saline as the mobile phase.

### 6.3 Radiochemical characterization of ¹⁸FAl-H₃RESCA-aGPC3-scFv

The prepared ¹⁸FAl-H₃RESCA-aGPC3-scFv was determined and characterized by analytical SEC-HPLC. The SEC-HPLC results indicated that the purity of the antibody aGPC3-scFv was greater than 99%. The radiochemical purity of ¹⁸FA1-H₃RESCA-aGPC3-scFv, as determined by ITLC, was greater than 99%. The radiochemical purity was greater than 95% after purification of the labeling mixture via PD-10 desalting column.

### Example 7. Preparation of ¹⁷⁷Lu-DOTA-aGPC3-Fab

### 7.1 Preparation of DOTA-aGPC3-Fab

Antibody aGPC3-Fab was purified via PD-10 desalting chromatography column. The purity of antibody aGPC3-Fab was determined via size exclusion chromatography (SEC-HPLC). 20 nmol of purified antibody aGPC3-Fab was taken and the pH was adjusted to 8.5-9.0 using 0.1M of Na₂CO₃-NaHCO₃ (pH 9.0). Then, p-SCN-Bn-DOTA (dissolved in a small volume of DMSO) was added at a molar ratio of 20-30 times relative to the antibody, and then reacted overnight at 4°C in the dark to synthesize DOTA-aGPC3-Fab. The excess unreacted DOTA was removed by purification via PD-10 desalting chromatography column. The purified antibody was concentrated using an ultrafiltration centrifuge tube (Millipore, 0.5 mL/30 kDa) under the conditions of 4°C, 8,000 x g for 10 min, and stored in a refrigerator at 4°C for short-term, or aliquoted and stored in a freezer at -80°C for long-term.

### 7.2 Radiolabeling with ¹⁷⁷Lu

A solution of ¹⁷⁷LuCl₃ (185 MBq) was taken and added to 500 µL of ammonium acetate solution (0.1 M, pH 5.5). The pH was adjusted to 5.5, followed by the addition of approximately 200 µg of DOTA-aGPC3-Fab. After thorough mixing, the reaction was proceeded at 37°C for 1 h. The reaction product was purified via PD-10 desalting chromatography column. The radiochemical purity of the product was determined via radio-instant thin-layer chromatography (ITLC) scanner, with a citrate-citric acid buffer (pH 4.0) as the developing solvent.

### 7.3 Radiochemical Characterization of ¹⁷⁷Lu-DOTA-aGPC3-Fab

The prepared ¹⁷⁷Lu-DOTA-aGPC3-Fab was determined and characterized via analytical Radio-ITLC. A novel immuno-SPECT probe, ¹⁷⁷Lu-DOTA-aGPC3-Fab, was successfully prepared. Radio-ITLC results showed a radiolabeling efficiency of approximately 82.5% (FIG. 5A). SEC-HPLC results showed that the radiochemical purity of ¹⁷⁷Lu-DOTA-aGPC3-Fab was greater than 97%, and a 24-hour stability in PBS was greater than 85% (FIG. 5B).

### Example 8. Preparation of ¹⁷⁷Lu-DOTA-aGPC3-scFv

### 8.1 Preparation of DOTA-aGPC3-scFv

Antibody aGPC3-scFv was purified via PD-10 desalting chromatography column. The purity of antibody aGPC3-scFv was determined via size exclusion chromatography (SEC-HPLC). 20 nmol of purified antibody aGPC3-scFv was taken and the pH was adjusted to 8.5-9.0 using 0.1M of Na₂CO₃-NaHCO₃ (pH 9.0). Then, p-SCN-Bn-DOTA (dissolved in a small volume of DMSO) was added at a molar ratio of 20 times relative to the antibody, and then reacted overnight at 4°C in the dark to synthesize DOTA-aGPC3-scFv. The excess unreacted DOTA was removed by purification via PD-10 desalting chromatography column. The purified antibody was concentrated using an ultrafiltration centrifuge tube (Millipore, 0.5 mL/10 kDa) under the conditions of 4°C, 10,000 x g for 15 min, and stored in a refrigerator at 4°C for short-term, or aliquoted and stored in a freezer at -80°C for long-term.

### 8.2 Radiolabeling with ¹⁷⁷Lu

A solution of ¹⁷⁷LuCl₃ (185 MBq) was taken and added to 500 µL of ammonium acetate solution (0.1 M, pH 5.5). The pH was adjusted to 5.5, followed by the addition of approximately 400 µg of DOTA-aGPC3-scFv. After thorough mixing, the reaction was proceeded at 37°C for 1 h. The reaction product was purified via PD-10 desalting chromatography column. The radiochemical purity of the product was determined via radio-instant thin-layer chromatography (ITLC) scanner, with a citrate-citric acid buffer (pH 4.0) as the developing solvent.

### 8.3 Radiochemical Characterization of ¹⁷⁷Lu-DOTA-aGPC3-scFv

The prepared ¹⁷⁷Lu-DOTA-aGPC3-scFv was determined and characterized via analytical SEC-ITLC. A novel immuno-SPECT probe, ¹⁷⁷Lu-DOTA-aGPC3-scFv, was successfully prepared. Radio-iTLC results showed a radiolabeling efficiency of approximately 73.1% (FIG. 6). SEC-HPLC results showed that the radiochemical purity of ¹⁷⁷Lu-DOTA-aGPC3-scFv was greater than 95%.

### Example 9. Preparation of ¹⁷⁷Lu-DOTA-aGPC3-mAb

### 9.1 Preparation of DOTA-aGPC3-mAb

Antibody aGPC3-mAb was purified via PD-10 desalting chromatography column. The purity of antibody aGPC3-mAb was determined via size exclusion chromatography (SEC-HPLC). 20 nmol of purified antibody aGPC3-mAb was taken and the pH was adjusted to 8.5-9.0 using 0.1M of Na₂CO₃-NaHCO₃ (pH 9.0). Then, p-SCN-Bn-DOTA (dissolved in a small volume of DMSO) was added at a molar ratio of 60 times relative to the antibody, and then reacted overnight at 4°C in the dark to synthesize DOTA-aGPC3-mAb. The excess unreacted DOTA was removed by purification via PD-10 desalting chromatography column. The purified antibody was concentrated using an ultrafiltration centrifuge tube (Millipore, 0.5 mL/30 Kd) under the conditions of 4°C, 10,000 x g for 10 min, and stored in a refrigerator at 4°C for short-term, or aliquoted and stored in a freezer at -80°C for long-term.

### 9.2 Radiolabeling with ¹⁷⁷Lu

A solution of ¹⁷⁷LuCl₃ (185 MBq) was taken and added to 500 µL of ammonium acetate solution (0.1 M, pH 5.5). The pH was adjusted to 5.5, followed by the addition of approximately 1 mg of DOTA-aGPC3-mAb. After thorough mixing, the reaction was proceeded at 37°C for 1 h. The reaction product was purified via PD-10 desalting chromatography column. The radiochemical purity of the product was determined via radio-instant thin-layer chromatography (ITLC) scanner, with a citrate-citric acid buffer (pH 4.0) as the developing solvent.

### 9.3 Radiochemical characterization of ¹⁷⁷Lu-DOTA-aGPC3-mAb

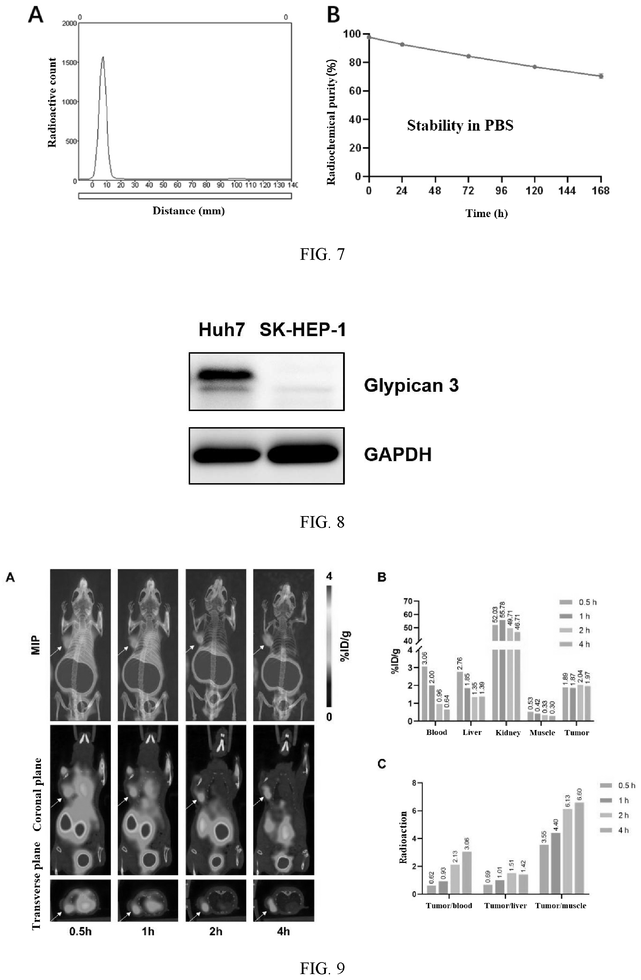

The prepared ¹⁷⁷Lu-DOTA-aGPC3-mAb was determined and characterized via analytical SEC-ITLC. A novel immuno-SPECT probe, ¹⁷⁷Lu-DOTA-aGPC3-mAb, was successfully prepared. Radio-ITLC results showed a radiolabeling efficiency of approximately 80.4% (FIG. 7A). SEC-HPLC results showed that the radiochemical purity of ¹⁷⁷Lu-DOTA-aGPC3-mAb was greater than 97%, and a 72-hour stability in PBS was greater than 80% (FIG. 7B).

### Example 10. Analysis of GPC3 expression in cells

The human-derived liver cancer cell lines Huh7 (GPC3-positive) and SK-HEP-1 (GPC3-negative) were cultured in complete MEM or DMEM medium (containing 10% FBS and 1% penicillin-streptomycin). Cells were maintained in a cell culture incubator at 37°C with 5% CO₂. Huh7 and SK-HEP-1 cells were cultured in dishes until reaching 75%-80% confluence, after which total protein was extracted. Protein concentration was determined using a BCA assay kit. SDS-PAGE stacking and resolving gels were prepared using standard formulations. The prepared protein samples and a protein marker were loaded into the sample wells, with a total protein loading amount of 40 µg per sample, followed by SDS-PAGE electrophoresis. After electrophoresis, proteins were transferred onto a PVDF membrane, which was then blocked. A primary antibody, rabbit anti-Glypican 3 monoclonal antibody, was diluted at 1:1000 and incubated with the PVDF membrane at 4°C overnight. Subsequently, the membrane was incubated with a secondary antibody at room temperature on a shaker for 1 hour, followed by development. Band intensities were measured using Image J software. GPC3 expression in the human liver cancer cell line was evaluated by Western blot. As shown in FIG. 8, the Huh7 cell line exhibited positive GPC3 expression, while the SK-HEP-1 cell line showed negative expression.

### Example 11. PET/CT Imaging of subcutaneous tumor models with the ⁶⁸Ga-NOTA-aGPC3-Fab probe

All animal studies were conducted in accordance with the guidelines of the Laboratory Animal Use and Management Committee of Tongji Medical College, Huazhong University of Science and Technology. Female Balb/c nude mice (4-5 weeks old) were purchased from Beijing Huafukang Biological Technology Co. Ltd and housed under SPF conditions at the Experimental Animal Center of Tongji Medical College, Huazhong University of Science and Technology.

Huh7 and SK-HEP-1 cells in the logarithmic growth phase were collected, routinely digested with trypsin, and then cell pellets were collected and counted. Subsequently, washing was performed twice with pre-cooled PBS, and cell pellets were collected by centrifugation. A cell resuspension solution was prepared at a volume ratio of PBS : Matrigel = 1:1. The cells were blown thoroughly and then inoculated subcutaneously near the armpit of the right forelimb of each mouse, with each mouse receiving 1 x 10⁷ cells (in a volume of 125 µL per mouse). The mice were used for subsequent experiments when the diameter of tumor reached 8-10 mm.

Experimental group Huh7 tumor-bearing nude mice, control group SK-HEP-1 tumor-bearing nude mice, and liver cancer PDX models were intravenously injected via tail vein with ⁶⁸Ga-NOTA-aGPC3-Fab (3.7-5.55 MBq, 100-150 µCi). PET/CT scans were performed under isoflurane inhalation anesthesia at 0.5, 1, 2, and 4 h post-injection. Each scan acquisition lasted approximately 15 min. After scanning, PET and CT images were reconstructed following attenuation correction. Regions of interest (ROI) were delineated using Amide software to calculate the uptake values (%ID/g).

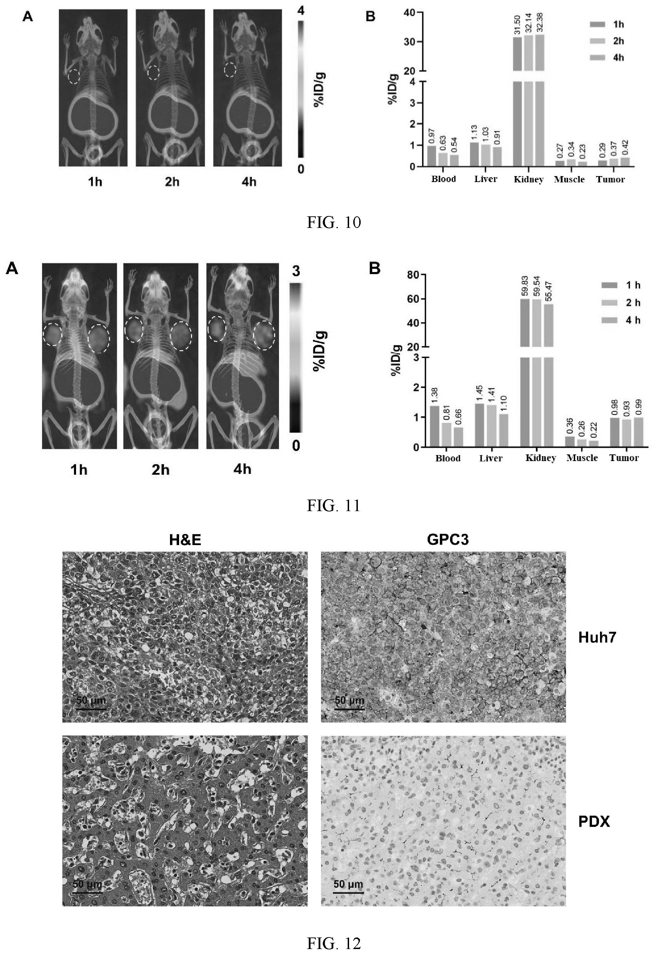

To evaluate the *in vivo* targeting specificity of ⁶⁸Ga-NOTA-aGPC3-Fab, static PET/CT scans were performed on Huh7 tumor-bearing mice at 30 min, 1 h, 2 h, and 4 h post-intravenous injection via tail vein of the probe. As shown in FIG. 9, the tumor was visualized as early as 30 minutes after injection of ⁶⁸Ga-NOTA-aGPC3-Fab, with a tumor uptake of 1.89 %ID/g. Thereafter, the tumor uptake remained at a stable level, reaching 1.87 %ID/g at 1 h and 2.04 %ID/g at 2 h. At 4 h, the tumor outline remained clearly defined with an uptake value of 1.97 %ID/g, indicating high affinity of the probe for the target and favorable retention within the tumor. The probe was rapidly cleared from the circulation and liver. At 2 h, the images exhibited a high tumor-to-background ratio. ROI quantitative analysis revealed that cardiac uptake decreased from 3.06 %ID/g at 30 minutes to 0.96 %ID/g, and liver uptake decreased from 2.76 %ID/g to 1.35 %ID/g. At 4 h, the tumor-to-heart ratio was 3.06, the tumor-to-liver ratio was 1.42, and the tumor-to-muscle ratio was 6.60. In contrast, tumors in the control group SK-HEP-1 tumor-bearing mice showed no tumor visualization at any time point (FIG. 10). Tumor uptake values at 1 h, 2 h, and 4 h were 0.29 %ID/g, 0.37 %ID/g, and 0.42 %ID/g, respectively, all significantly lower than those in the Huh7 tumor-bearing mice. In the imaging of both tumor-bearing groups, significant radioactive accumulation was observed in the non-target organs kidneys and bladder, indicating that the probe is primarily metabolized via the renal pathway.

### Example 12. PET/CT imaging of PDX models

Collection of patient baseline data: the collection of all patient information and tumor samples was approved by the Clinical Ethics Committee of Union Hospital, Tongji Medical College, Huazhong University of Science and Technology. The inclusion criteria for sampling were preoperative suspicion of liver cancer and the assessed ability to obtain more than 200 mm³ of tumor tissue, with informed consent obtained from the patients prior to surgery. Basic clinical information collected from patients included general patient information, clinical data, tumor markers, biochemical indicators, imaging examination data, and postoperative pathological data.

Acquisition and transport of fresh tumor tissue: Immediately after resection, the surgical liver tumor sample was placed in a sterile tray. A sterile scalpel was used to dissect 200-500 mm³ of fresh tumor tissue, which was then placed into a 15 mL sample tube containing sterile PBS. The tumor sample was stored in a 4°C refrigerator, and a cooling box was prepared for sampling. To preserve tissue viability, the sample must be surrounded by an ice-water mixture or ice packs throughout transport. The time from sample collection to delivery to the laboratory for PDX model construction must not exceed 2 hours.

Tumor implantation: All tumor implantation procedures were performed on a sterile operating table. First, the tumor tissue was poured into a cell culture dish. Sterile ophthalmic forceps were used to transfer the tissue into PBS containing a penicillin-streptomycin mixture for washing. Then, necrotic tumor tissue was carefully removed with sterile ophthalmic scissors, leaving viable tumor tissue blocks as much as possible. The washed tumor tissue was transferred to a cell culture dish containing sterile DMEM medium. The tissue was trimmed into approximately 2 mm³ tumor fragments for subsequent use. Additionally, 500,100 mm³ of tumor tissue was collected for paraffin embedding, frozen storage, and viable tissue cryopreservation. Healthy NSG mice were fasted for 10 hours and deprived of water for 4 hours prior to surgery. The mice were anesthetized via isoflurane inhalation. The surgical area was shaved and routinely disinfected with povidone-iodine solution. A midline incision of approximately 2-3 mm was made at the level of the shoulder blades. Using a trocar, the tumor tissue was gently advanced and placed into the bilateral axillary regions of the mice. The skin was then sutured with absorbable suture material. The health status of the mice was monitored daily for 5 consecutive days postoperatively, with attention to signs of wound dehiscence or infection. The mice were used for imaging studies when the tumor volume reached 1,000-1,500 mm³.

Liver tumor tissue samples obtained from surgical resection of patients were used to establish PDX models. The clinical data and tumor histopathological characteristics of the patients are listed in Table 1. Bilateral PDX models were successfully established. After intravenous injection of ⁶⁸Ga-NOTA-aGPC3-Fab via the tail vein into the PDX model mice, static small-animal PET/CT imaging was performed at 1, 2, and 4 h post-injection (FIG. 11). Following the injection of ⁶⁸Ga-NOTA-aGPC3-Fab, the tumors were clearly visualized. The average uptake values in bilateral tumors at 1, 2, and 4 h were 0.98 %ID/g, 0.93 %ID/g, and 0.99 %ID/g, respectively, indicating stable retention of the probe within the tumors. Similar to the subcutaneous tumor models, the uptake of the probe in the heart and liver decreased over time. The observed radioactive accumulation in the kidneys and bladder suggested that the probe is primarily metabolized via the renal pathway.

**Table 1. Clinical information and tumor histopathological characteristics of patients**

| **Gender** | **Age** | **TNM stage** | **Histological classification** |
|---|---|---|---|
| Female | 42 | pT1bN0M1 | Moderately differentiated cholangiocarcinoma |

### Example 13. Histological analysis

Tumor tissues were fixed in 4% paraformaldehyde, dehydrated through a graded ethanol series, cleared in xylene, infiltrated with paraffin, and embedded to form paraffin blocks. Sections (5 µm thickness) were prepared using a microtome.

H&E staining: Sections were stained with Harris's hematoxylin for 4 min, rinsed with tap water for 2 min until no excess staining solution is leached out. Differentiation was performed in 0.8% hydrochloric acid alcohol for 2 seconds, followed by rinsing with tap water. The sections may also be blued in lithium carbonate solution, then rinsed with water for 2 min. Subsequently, sections were stained with eosin (alcohol-soluble) for 20 seconds. Without water rinsing, they were directly immersed in 95% ethanol for color adjustment for 5 seconds, followed by dehydration in absolute ethanol with two changes, 2 minutes each. Finally, sections were cleared in an eco-friendly clearing agent, mounted, and examined under a microscope.

GPC3 immunohistochemical staining: Following antigen retrieval, endogenous peroxidase activity was blocked. The sections were then incubated with 10% serum that is from the same species as the secondary antibody at 37°C for 30 min to reduce non-specific staining. The serum was removed. The primary antibody stock solution was diluted with 10% serum to prepare the primary antibody working solution. The diluted primary antibody working solution (anti-Glypican 3 monoclonal antibody, 1:500 dilution) was then added dropwise to the sections, which were incubated at 4°C overnight. Next day, the sections were allowed to equilibrate to room temperature for 15 minutes, rinsed 3 times with TBST, and then subjected to three 3-minute immersion washings in TBST. The secondary antibody stock solution was diluted with TBST to prepare the secondary antibody working solution, which was added dropwise to each section followed by incubation at 37°C for 45 minutes. The sections were rinsed three times with TBST and then subjected to three 3-minute immersion washings in TBST. After removing the TBST, a tyramide working solution was added dropwise to each section and incubated at room temperature in the dark for 10 minutes. The sections were rinsed three times with TBST and then subjected to three 3-minute immersion washes in TBST. Subsequently, a freshly prepared tyramide working solution was added dropwise to each section for chromogenic development. Following counterstaining with hematoxylin and bluing, the sections were mounted and examined under a microscope.

Immunohistochemical analysis of the tumor tissues was performed to assess GPC3 expression in the Huh7 subcutaneous tumor model and the PDX model. As shown in FIG. 12, Huh7 tumor tissues exhibited extensive and strong GPC3 expression, whereas the PDX tumor tissue (cholangiocarcinoma) showed relatively low GPC3 expression. These findings are consistent with the imaging results, indicating that this probe can effectively image tumors with varying levels of GPC3 expression.

### Example 14. PET/CT Imaging of subcutaneous tumor models with the ⁶⁸Ga-NOTA-aGPC3-scFv probe

The human-derived liver cancer cell line Hep3B (GPC3-positive) was cultured in complete MEM medium (containing 10% FBS and 1% penicillin-streptomycin). Cells were maintained in a cell culture incubator at 37°C with 5% CO₂.

Hep3B cells in the logarithmic growth phase were collected, routinely digested with trypsin, and then cell pellets were collected and counted. Subsequently, washing was performed twice with pre-cooled PBS, and cell pellets were collected by centrifugation. A cell resuspension solution was prepared at a volume ratio of PBS : Matrigel = 1:1. The cells were blown thoroughly and then inoculated subcutaneously near the armpit of the right forelimb of each mouse, with each mouse receiving 5 x 10⁶ cells (in a volume of 100 µL per mouse). The mice were used for subsequent experiments when the diameter of tumor reached 8-10 mm.

Hep3B cells in the logarithmic growth phase were collected, routinely digested with trypsin, and then cell pellets were collected and counted. Subsequently, washing was performed twice with pre-cooled PBS, and cell pellets were collected by centrifugation. A cell suspension was prepared at a volume ratio of PBS : Matrigel = 1:1 for later use. Healthy BALB/c-nu mice were fasted for 10 hours and deprived of water for 4 hours prior to surgery. Within a biological safety cabinet, mice were placed under continuous anesthesia using a small-animal gas anesthesia system (2% isoflurane). Each mouse was positioned supine and secured. The skin was routinely disinfected with povidone-iodine solution. A midline incision approximately 1 cm in length was made below the xiphoid process in the upper abdomen. The skin and peritoneum were incised layer by layer. A gauze pad moistened with saline was placed beneath the incision. Both sides of the costal arch were gently pressed to expose the left liver lobe onto the moist gauze. A needle was inserted at a beveled angle, approximately 20° to the horizontal plane, and advanced about 1 cm into the liver. The syringe plunger was gently depressed to slowly inject 0.05 mL of cell suspension containing approximately 5×10⁵ Hep3B cells. The needle was slowly withdrawn, and light pressure was applied to the puncture site with a sterile cotton swab for a moment. The liver was then gently repositioned into the abdominal cavity, and the abdomen was closed layer by layer. The skin was disinfected again, completing the procedure. Postoperatively, the mice were deprived of both food and water for 4 hours.

Hep3B tumor-bearing nude mice and the Hep3B liver orthotopic cancer model were intravenously injected via tail vein with ⁶⁸Ga-NOTA-aGPC3-scFv (3.7-5.55 MBq, 100-150 µCi). PET/CT scans were performed under isoflurane inhalation anesthesia at 1, 2, and 4 h post-injection. Each scan acquisition lasted approximately 15 min. After scanning, PET and CT images were reconstructed following attenuation correction. Regions of interest (ROI) were delineated using Amide software to calculate the uptake values (%ID/g).

Static PET/CT scans were performed on Hep3B subcutaneous tumor-bearing mice at 1 h, 2 h, and 4 h post-intravenous injection of ⁶⁸Ga-NOTA-aGPC3-scFv via the tail vein. As shown in FIG. 13A, the tumor was clearly visualized in Hep3B tumor-bearing mice at 1 hour post-injection of ⁶⁸Ga-NOTA-aGPC3-scFv, with the images exhibiting a high tumor-to-background ratio. Results from ROI delineation and semiquantitative analysis of the PET images (FIG. 13B, FIG. 13C) showed that tumor uptake increased continuously over time, with uptake values of 3.00 ± 1.37 %ID/g, 3.13 ± 1.54 %ID/g, and 3.22 ± 1.64 %ID/g at 1 h, 2 h, and 4 h, respectively. The probe was rapidly cleared from the circulation. At 1 h, the tumor-to-blood pool ratio was 4.91 ± 1.84, the tumor-to-liver ratio was 2.25 ± 0.78, and the tumor-to-muscle ratio was 8.92 ± 5.72. Both the tumor-to-blood pool and tumor-to-liver ratios continued to increase over time. Significant radioactive accumulation was observed in the non-target organs like kidneys and bladder, indicating that the probe is primarily metabolized via the renal pathway. The prolonged retention of the probe at the tumor site and its rapid clearance from the background demonstrated its high affinity for the target and its promising potential for early post-injection imaging.

In a comparative analysis of PET imaging for the Hep3B subcutaneous tumor model with ⁶⁸Ga-NOTA-aGPC3-scFv versus ⁶⁸Ga-NOTA-aGPC3-Fab (FIG. 14A, FIG. 14B), both probes showed high uptake in the tumor at 1 h post-injection. The tumor uptake gradually increased over time for both. However, ⁶⁸Ga-NOTA-aGPC3-scFv exhibited a shorter blood half-life, enabling faster clearance from the blood and non-target organs such as the liver, and consequently demonstrated superior tumor-to-background contrast at all time points. Semi-quantitative analysis of the PET images (FIG. 14C-FIG. 14E) revealed that the tumor-to-blood pool and tumor-to-liver ratios of ⁶⁸Ga-NOTA-aGPC3-scFv were significantly higher than those of ⁶⁸Ga-NOTA-aGPC3-Fab at all time points, while its tumor-to-muscle ratio was slightly higher than that of ⁶⁸Ga-NOTA-aGPC3-Fab.

### Example 15. Comparison of PET/CT imaging of Hep3B Orthotopic Liver Tumor Model Mice with ⁶⁸Ga-NOTA-aGPC3-scFv, ⁶⁸Ga-NOTA-aGPC3-Fab, and ¹⁸F-FDG

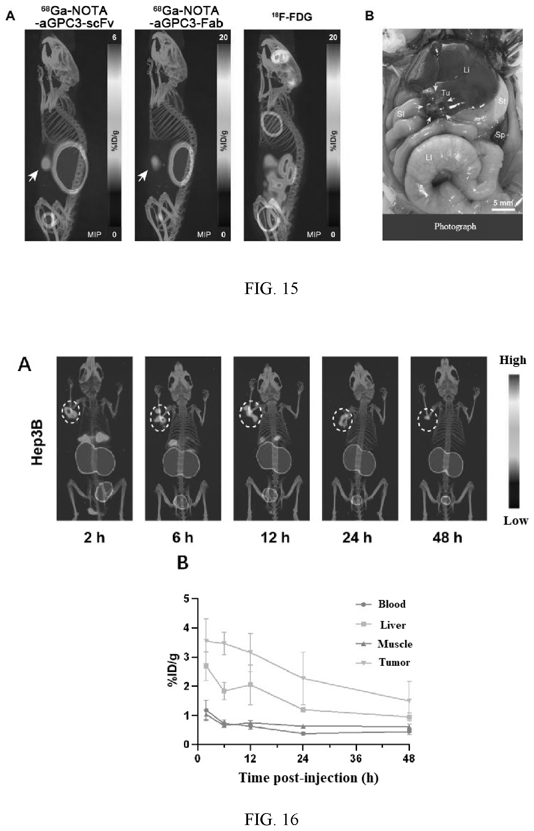

The same group of Hep3B orthotopic tumor model was imaged with ⁶⁸Ga-NOTA-aGPC3-scFv, ⁶⁸Ga-NOTA-aGPC3-Fab, and ¹⁸F-FDG, respectively, to compare detection capabilities for orthotopic liver tumors among them. As shown in FIG. 15A, both ⁶⁸Ga-NOTA-aGPC3-scFv and ⁶⁸Ga-NOTA-aGPC3-Fab clearly delineated the location of the orthotopic liver tumors at 1 h post-injection. In contrast, ¹⁸F-FDG imaging showed high abdominal and intestinal uptake, making it difficult to distinguish the tumor from normal organs. Anatomic exploration for liver orthotopic tumors (FIG. 15B) revealed tumor growth in the left lobe of the mouse liver, with a diameter of approximately 5 mm. The tumor location was consistent with the PET imaging findings from both ⁶⁸Ga-NOTA-aGPC3-scFv and ⁶⁸Ga-NOTA-aGPC3-Fab, indicating that both probes were capable of clearly detecting 5 mm lesions.

### Example 16. SPECT/CT Imaging of subcutaneous tumor models with ¹⁷⁷Lu-DOTA-aGPC3-Fab probe

The human-derived liver cancer cell line Hep3B (GPC3-positive) was cultured in complete MEM medium (containing 10% FBS and 1% penicillin-streptomycin). Cells were maintained in a cell culture incubator at 37°C with 5% CO₂. Hep3B cells in the logarithmic growth phase were collected, routinely digested with trypsin, and then cell pellets were collected and counted. Subsequently, washing was performed twice with pre-cooled PBS, and cell pellets were collected by centrifugation. A cell resuspension solution was prepared at a volume ratio of PBS : Matrigel = 1:1. The cells were blown thoroughly and then inoculated subcutaneously near the armpit of the right forelimb of each mouse, with each mouse receiving 5 x 10⁶ cells (in a volume of 100 µL per mouse). The mice were used for subsequent experiments when the diameter of tumor reached about 8-10 mm.

Following intravenous injection of ¹⁷⁷Lu-DOTA-aGPC3-Fab (18.5 MBq, 500 µCi) via the tail vein into Hep3B subcutaneous tumor-bearing nude mice, SPECT/CT scans were performed at 2, 6, 12, 24, and 48 h post-injection. Each scan acquisition lasted approximately 20-30 minutes. Anesthesia was maintained throughout the scanning procedure using 1.0-1.5% isoflurane. Attenuation and scatter correction were performed on SPECT data. Image reconstruction was performed using the three-dimensional ordered subsets expectation maximization (OSEM) algorithm. Regions of interest (ROI) were delineated for quantitative analysis of the SPECT images. Tissue radioactive uptake was expressed as the percentage of injected dose per gram of tissue (%ID/g).

Static SPECT/CT scans were performed on Hep3B subcutaneous tumor-bearing mice at 2, 6, 12, 24 and 48 h post-intravenous injection of ¹⁷⁷Lu-DOTA-aGPC3-Fab via the tail vein. As shown in FIG. 16A, the tumor was clearly visualized in Hep3B tumor-bearing mice at 2 h post-injection of ¹⁷⁷Lu-DOTA-aGPC3-Fab, with the images exhibiting a high tumor-to-background ratio. Results from ROI delineation and semiquantitative analysis of the SPECT images (FIG. 16B) showed that tumor uptake remained relatively stable at 2-6 h (2 h: 3.55 ± 0.76 %ID/g; 6 h: 3.46 ± 0.37 %ID/g). After 6 h, tumor uptake gradually declined, yet remained at 1.50 ± 0.67 %ID/g at 48 h. The probe was rapidly cleared from the blood. At 2 h, the tumor-to-blood pool ratio was 3.34 ± 1.66, which subsequently increased and peaked at 6.08 ± 2.44 at 24 h. Both the tumor-to-liver ratio and the tumor-to-muscle ratio reached their peak values at 6 h, measuring 1.95 ± 0.55 and 5.37 ± 1.17, respectively. Significant radioactive accumulation was observed in the non-target organs like kidneys and bladder, indicating that kidneys serve as the primary metabolic organ for the probe ROI delineation and semi-quantitative analysis of the kidneys revealed a rapid decrease in renal radioactive uptake within 48 h. The probe's rapid uptake and prolonged retention at the tumor site, along with its rapid clearance from non-target organs, suggested its potential for further application in radioimmunotherapy.

### Example 17. SPECT/CT Imaging of subcutaneous tumor models with ¹⁷⁷Lu-DOTA-aGPC3-scFv probe

The human-derived liver cancer cell lines Hep3B (GPC3-positive) was cultured in complete MEM medium (containing 10% FBS and 1% penicillin-streptomycin). Cells were maintained in a cell culture incubator at 37°C with 5% CO₂.Hep3B cells in the logarithmic growth phase were collected, routinely digested with trypsin, and then cell pellets were collected and counted. Subsequently, washing was performed twice with pre-cooled PBS, and cell pellets were collected by centrifugation. A cell resuspension solution was prepared at a volume ratio of PBS : Matrigel = 1:1. The cells were blown thoroughly and then inoculated subcutaneously near the armpit of the right forelimb of each mouse, with each mouse receiving 7 x 10⁶ cells (in a volume of 100 µL per mouse). The mice were used for subsequent experiments when the diameter of tumor reached about 8-10 mm.

Following intravenous injection of ¹⁷⁷Lu-DOTA-aGPC3-scFv (18.5 MBq, 500 µCi) via the tail vein into Hep3B subcutaneous tumor-bearing nude mice, SPECT/CT scans were performed at 2, 6, 12, 24, and 48 h post-injection. Each scan acquisition lasted approximately 20-30 minutes. Anesthesia was maintained throughout the scanning procedure using 1.0-1.5% isoflurane. Attenuation and scatter correction were performed on SPECT data. Image reconstruction was performed using the three-dimensional ordered subsets expectation maximization (OSEM) algorithm. Regions of interest (ROI) were delineated for quantitative analysis of the SPECT images. Tissue radioactive uptake was expressed as the percentage of injected dose per gram of tissue (%ID/g).

Static SPECT/CT scans were performed on Hep3B subcutaneous tumor-bearing mice at 2, 6, 12, 24 and 48 h post-intravenous injection of ¹⁷⁷Lu-DOTA-aGPC3-scFv via the tail vein. As shown in FIG. 17A, the tumor was clearly visualized in Hep3B tumor-bearing mice at 2 h post-injection of ¹⁷⁷Lu-DOTA-aGPC3-scFv, with the images exhibiting a high tumor-to-background ratio. Results from ROI delineation and semiquantitative analysis of the SPECT images (FIG. 17B) showed that tumor uptake remained relatively stable at 2-6 h (2 h: 3.99 ± 0.15 %ID/g; 6 h: 3.77 ± 0.32 %ID/g). After 6 h, tumor uptake gradually declined, reaching 0.94 ± 0.61 %ID/g at 48 h. The probe was rapidly cleared from the circulation. From 2 to 24 h, high tumor-to-background ratios were maintained. Among these, the tumor-to-blood pool, tumor-to-liver, and tumor-to-muscle ratios reached their peak values at 6 h, measuring 10.72 ± 3.93, 2.89 ± 0.80, and 7.36 ± 2.08, respectively. Significant radioactive accumulation was observed in the non-target organs like kidneys and bladder, indicating that kidneys serve as the primary metabolic organ for the probe. ROI delineation and semi-quantitative analysis of the kidneys revealed a rapid decrease in renal radioactive uptake within 24 h. The probe's rapid uptake and prolonged retention at the tumor site, along with its rapid clearance from non-target organs, suggested its potential for further application in radioimmunotherapy.

### Example 18. SPECT/CT Imaging of subcutaneous tumor models with ¹⁷⁷Lu-DOTA-aGPC3-mAb probe

The human-derived liver cancer cell line Hep3B (GPC3-positive) was cultured in complete MEM medium (containing 10% FBS and 1% penicillin-streptomycin). Cells were maintained in a cell culture incubator at 37°C with 5% CO₂. Hep3B cells in the logarithmic growth phase were collected, routinely digested with trypsin, and then cell pellets were collected and counted. Subsequently, washing was performed twice with pre-cooled PBS, and cell pellets were collected by centrifugation. A cell resuspension solution was prepared at a volume ratio of PBS : Matrigel = 1:1. The cells were blown thoroughly and then inoculated subcutaneously near the armpit of the right forelimb of each mouse, with each mouse receiving 5 x 10⁶ cells (in a volume of 100 µL per mouse). The mice were used for subsequent experiments when the diameter of tumor reached about 8-10 mm.

Following intravenous injection of ¹⁷⁷Lu-DOTA-aGPC3-mAb (18.5 MBq, 500 µCi) via the tail vein into Hep3B subcutaneous tumor-bearing nude mice, SPECT/CT scans were performed at 2, 6, 12h and day 3, 5, and 7 post-injection. Each scan acquisition lasted approximately 20-30 minutes. Anesthesia was maintained throughout the scanning procedure using 1.0-1.5% isoflurane. Attenuation and scatter correction were performed on SPECT data. Image reconstruction was performed using the three-dimensional ordered subsets expectation maximization (OSEM) algorithm. Regions of interest (ROI) were delineated for quantitative analysis of the SPECT images. Tissue radioactive uptake was expressed as the percentage of injected dose per gram of tissue (%ID/g).

Static SPECT/CT scans were performed on Hep3B subcutaneous tumor-bearing mice at 2, 6, 12h and day 3, 5, and 7 post-intravenous injection of ¹⁷⁷Lu-DOTA-aGPC3-mAb via the tail vein. As shown in FIG. 18A, after injection of ¹⁷⁷Lu-DOTA-aGPC3-mAb into Hep3B tumor-bearing mice, the probe was primarily distributed in the circulation from 2 to 6 h, began to be uptaken by tumor at 6 h post-injection which is gradually increased thereafter, and progressively cleared from the circulation. At 24 h, the images exhibited favorable tumor-to-background contrast. Results from ROI delineation and semi-quantitative analysis of the SPECT images (FIG. 18B) showed that the probe maintained sustained and stable retention within the tumor from 24 to 72 h (24 h: 11.34 ± 0.98 %ID/g; 72 h: 10.40 ± 1.13 %ID/g). Subsequently, the uptake declined slowly, remaining at 4.55 ± 0.64 %ID/g by day 7. The probe was gradually cleared from the circulation. The tumor-to-blood pool and tumor-to-liver ratios continued to increase (FIG. 18C to FIG. 18D), reaching their peak values on day 7 at 10.08 ± 2.42 and 4.11 ± 1.37, respectively. The tumor-to-muscle ratio peaked at 9.13 ± 1.53 on day 5 (FIG. 18E). The probe's prolonged retention at the tumor site suggested its potential for further application in radioimmunotherapy.

### Example 19. Radioimmunotherapy of subcutaneous tumor models with ¹⁷⁷Lu-DOTA-aGPC3-Fab Probe

The human-derived liver cancer cell line Hep3B (GPC3-positive) was cultured in complete MEM medium (containing 10% FBS and 1% penicillin-streptomycin). Cells were maintained in a cell culture incubator at 37°C with 5% CO₂. Hep3B cells in the logarithmic growth phase were collected, routinely digested with trypsin, and then cell pellets were collected and counted. Subsequently, washing was performed twice with pre-cooled PBS, and cell pellets were collected by centrifugation. A cell resuspension solution was prepared at a volume ratio of PBS : Matrigel = 1:1. The cells were blown thoroughly and then inoculated subcutaneously near the armpit of the right forelimb of each mouse, with each mouse receiving 7 x 10⁶ cells (in a volume of 100 µL per mouse).

When the tumor volume reached approximately 150-200 mm³, the tumor-bearing mice were randomly divided into two groups: a saline group and ¹⁷⁷Lu-DOTA-aGPC3-Fab group (n=6). The general conditions of the mice were observed every three days. Body weight was measured (normalized body weight: body weight on day n / body weight on day 0 × 100%). Tumor length and width were measured using a vernier caliper, and tumor volume was calculated (Tumor volume = length x width x width / 2).

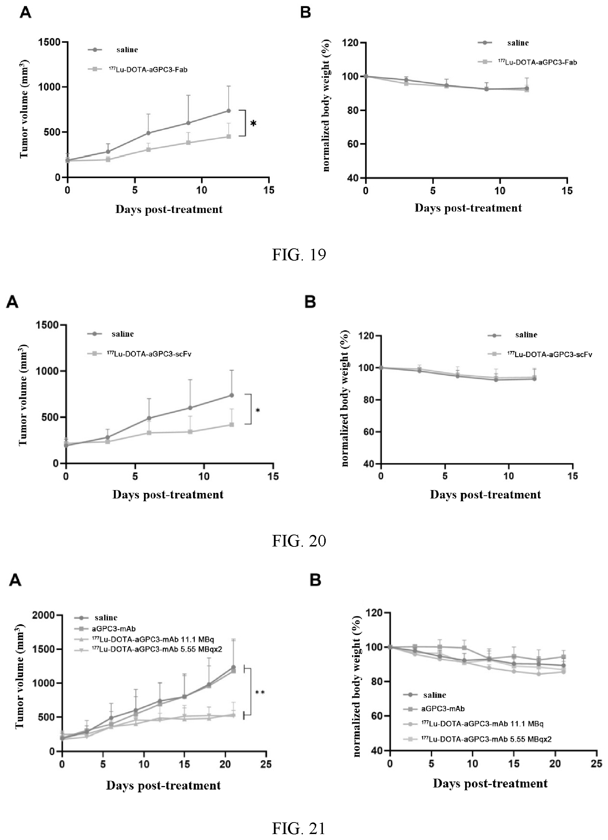

On day 0, mice in the two groups were intravenously injected via the tail vein with 150 µL of saline and the equal volume of 11.1 MBq ¹⁷⁷Lu-DOTA-aGPC3-Fab, respectively. FIG. 19 shows the changes in tumor volume for each group over 12-day treatment period. The tumor volume in ¹⁷⁷Lu-DOTA-aGPC3-Fab treatment group was significantly reduced compared to the saline group (P < 0.05). Both groups exhibited some degree of body weight loss after treatment, with no statistical difference between them. These results demonstrated that radioimmunotherapy with an injection of 11.1 MBq ¹⁷⁷Lu-DOTA-aGPC3-Fab can significantly slow down the tumor growth rate in tumor-bearing mice.

### Example 20. Radioimmunotherapy of subcutaneous tumor models with ¹⁷⁷Lu-DOTA-aGPC3-scFv Probe

The human-derived liver cancer cell line Hep3B (GPC3-positive) was cultured in complete MEM medium (containing 10% FBS and 1% penicillin-streptomycin). Cells were maintained in a cell culture incubator at 37°C with 5% CO₂. Hep3B cells in the logarithmic growth phase were collected, routinely digested with trypsin, and then cell pellets were collected and counted. Subsequently, washing was performed twice with pre-cooled PBS, and cell pellets were collected by centrifugation. A cell resuspension solution was prepared at a volume ratio of PBS : Matrigel = 1:1. The cells were blown thoroughly and then inoculated subcutaneously near the armpit of the right forelimb of each mouse, with each mouse receiving 7 x 10⁶ cells (in a volume of 100 µL per mouse).

When tumor volume reached approximately 150-200 mm³, tumor-bearing mice were randomly divided into two groups: saline group and ¹⁷⁷Lu-DOTA-aGPC3-scFv group (n=6). The general conditions of the mice were observed every three days. Body weight was measured (normalized body weight: body weight on day n / body weight on day 0 × 100%). Tumor length and width were measured using a vernier caliper, and tumor volume was calculated (Tumor volume = length x width x width / 2).

On day 0, mice in the two groups were intravenously injected via the tail vein with 150 µL of saline and the equal volume of 11.1 MBq ¹⁷⁷Lu-DOTA-aGPC3-scFv, respectively. FIG. 20 shows the changes in tumor volume for each group over 12-day treatment period. On day 12, the tumor volume in ¹⁷⁷Lu-DOTA-aGPC3-scFv treatment group was significantly reduced compared to the saline group (P < 0.05). Both groups exhibited some degree of body weight loss after treatment, with no statistical difference between them. These results demonstrated that radioimmunotherapy with an injection of 11.1 MBq ¹⁷⁷Lu-DOTA-aGPC3-scFv can significantly slow down the tumor growth rate in tumor-bearing mice.

### Example 21. Radioimmunotherapy of subcutaneous tumor models with ¹⁷⁷Lu-aGPC3-mAb Probe

The human-derived liver cancer cell line Hep3B (GPC3-positive) was cultured in complete MEM medium (containing 10% FBS and 1% penicillin-streptomycin). Cells were maintained in a cell culture incubator at 37°C with 5% CO₂. Hep3B cells in the logarithmic growth phase were collected, routinely digested with trypsin, and then cell pellets were collected and counted. Subsequently, washing was performed twice with pre-cooled PBS, and cell pellets were collected by centrifugation. A cell resuspension solution was prepared at a volume ratio of PBS : Matrigel = 1:1. The cells were blown thoroughly and then inoculated subcutaneously near the armpit of the right forelimb of each mouse, with each mouse receiving 7 x 10⁶ cells (in a volume of 100 µL per mouse).

When tumor volume reached approximately 150-200 mm³, tumor-bearing mice were randomly divided into four groups: saline group, aGPC3-mAb group, 11.1 MBq ¹⁷⁷Lu-DOTA-aGPC3-mAb group, and 5.55 MBq x 2 ¹⁷⁷Lu-DOTA-aGPC3-mAb group (n=6). The general conditions of the mice were observed every three days. Body weight was measured (normalized body weight: body weight on day n / body weight on day 0 × 100%). Tumor length and width were measured using a vernier caliper, and tumor volume was calculated (Tumor volume = length x width x width / 2).

On day 0, mice in the four groups were intravenously injected via the tail vein with 150 µL of saline, the equal volume of aGPC3-mAb (100 µg), 11.1 MBq of ¹⁷⁷Lu-DOTA-aGPC3-mAb, respectively. For the 5.55 MBq x 2 ¹⁷⁷Lu-DOTA-aGPC3-mAb group, equal volumes of 5.55 MBq ¹⁷⁷Lu-DOTA-aGPC3-mAb were administered on day 0 and day 7, respectively. FIG. 21 shows the changes in tumor volume for each group over 21-day treatment period. On day 21, the tumor volumes in both 11.1 MBq ¹⁷⁷Lu-DOTA-aGPC3-mAb group and 5.55 MBq x 2 ¹⁷⁷Lu-DOTA-aGPC3-mAb group were significantly smaller than those in the saline group and the aGPC3-mAb group (P < 0.01). All four groups of mice exhibited a certain degree of body weight loss following treatment. Compared to the saline control group, the body weight loss in the other groups showed no statistically significant difference. The therapeutic effect of twice administration of 5.55 MBq ¹⁷⁷Lu-DOTA-aGPC3-mAb is similar to that of once administration of 11.1 MBq ¹⁷⁷Lu-DOTA-aGPC3-mAb. However, the weight loss in the group of once administration of 11.1 MBq ¹⁷⁷Lu-DOTA-aGPC3-mAb is more significant than that in the group of divided administration, in which significant differences were observed specifically on day 6 and day 11. However, on day 21, no statistically significant difference in body weight loss was found between the two groups. These results demonstrated that radioimmunotherapy with an injection of ¹⁷⁷Lu-DOTA-aGPC3-mAb can significantly slow down the tumor growth rate in tumor-bearing mice.

The information of the antibodies used in the present disclosure are listed in Table 2 below.

**Table 2**

| No. | Name | Sequence |
|---|---|---|
| SEQ ID NO: 1 | HCDR1 | GFTFRSYA |
| SEQ ID NO: 2 | HCDR2 | ISGSGGRT |
| SEQ ID NO: 3 | HCDR3 | AKEYDFKTGWMHYYYGMDV |
| SEQ ID NO: 4 | LCDR1 | SSGYD |
| SEQ ID NO: 5 | LCDR2 | GSN |
| SEQ ID NO: 6 | LCDR3 | QSYDSSLNAI |
| SEQ ID NO: 7 | Heavy chain variable region | EVQLVESGGGLVQPGGSLRLSCAASGFTFRSYAMSWVRQAPG KGLEWVSAISGSGGRTYYADSVKGRFTISRDNSKNTLYLQMN SLRAEDTAVYYCAKEYDFKTGWMHYYYGMDVWGQGTTVT VSS |
| SEQ ID NO: 8 | Light chain variable region | QSVLTQPPSVSGAPGQRVTISCTGSSSGYDVHWYQQLPGRAP KLLIYGSNNRPSGVPDRFSGSNSGTSASLAITGLQAEDEADYY CQSYDSSLNAIFGGGTKLTVL |
| SEQ ID NO: 9 | Heavy chain | EVQLVESGGGLVQPGGSLRLSCAASGFTFRSYAMSWVRQAPG KGLEWVSAISGSGGRTYYADSVKGRFTISRDNSKNTLYLQMN SLRAEDTAVYYCAKEYDFKTGWMHYYYGMDVWGQGTTVT VSSASTKGPSVFPLAPSSKSTSGGTAALGCLVKDYFPEPVTVS WNSGALTSGVHTFPAVLQSSGLYSLSSVVTVPSSSLGTQTYIC NVNHKPSNTKVDKKVEPKSCDKTHTCPPCPAPELLGGPSVFLF PPKPKDTLMISRTPEVTCVVVDVSHEDPEVKFNWYVDGVEVH NAKTKPREEQYNSTYRVVSVLTVLHQDWLNGKEYKCKVSNK ALPAPIEKTISKAKGQPREPQVYTLPPSRDELTKNQVSLTCLVK GFYPSDIAVEWESNGQPENNYKTTPPVLDSDGSFFLYSKLTVD KSRWQQGNVFSCSVMHEALHNHYTQKSLSLSPGK |
| SEQ ID NO: 10 | Light chain | QSVLTQPPSVSGAPGQRVTISCTGSSSGYDVHWYQQLPGRAP KLLIYGSNNRPSGVPDRFSGSNSGTSASLAITGLQAEDEADYY CQSYDSSLNAIFGGGTKLTVLGQPKAAPSVTLFPPSSEELQAN KATLVCLISDFYPGAVTVAWKADSSPVKAGVETTTPSKQSNN KYAASSYLSLTPEQWKSHRSYSCQVTHEGSTVEKTVAPTECS |
| SEQ ID NO: 11 | Fab heavy chain | EVQLVESGGGLVQPGGSLRLSCAASGFTFRSYAMSWVRQAPG KGLEWVSAISGSGGRTYYADSVKGRFTISRDNSKNTLYLQMN SLRAEDTAVYYCAKEYDFKTGWMHYYYGMDVWGQGTTVT VSSASTKGPSVFPLAPSSKSTSGGTAALGCLVKDYFPEPVTVS WNSGALTSGVHTFPAVLQSSGLYSLSSVVTVPSSSLGTQTYIC NVNHKPSNTKVDKKVEPKSCDKTHL |
| SEQ ID NO: 12 | scFv | EVQLVESGGGLVQPGGSLRLSCAASGFTFRSYAMSWVRQAPG KGLEWVSAISGSGGRTYYADSVKGRFTISRDNSKNTLYLQMN SLRAEDTAVYYCAKEYDFKTGWMHYYYGMDVWGQGTTVT VSSGGGGSGGGGSGGGGSQSVLTQPPSVSGAPGQRVTISCTGS SSGYDVHWYQQLPGRAPKLLIYGSNNRPSGVPDRFSGSNSGT SASLAITGLQAEDEADYYCQSYDSSLNAIFGGGTKLTVLGHH HHHH |

## Claims

1. A molecular probe targeting GPC3, being a compound with a following structure, or a pharmaceutically acceptable salt or ester thereof:
(anti-GPC3 antibody, comprising a fragment of the anti-GPC3 antibody, or a single-domain antibody thereof)ₘ-Rₙ,
wherein R is selected from a group consisting of a linking group, a chelating agent, a radionuclide, another targeting peptide or monoclonal antibody, a monoclonal antibody fragment, a single-domain antibody, and a small-molecule inhibitor;
wherein n represents a number of R, and n=0-4;
wherein m is not 0.

2. The molecular probe targeting GPC3 of claim 1, wherein the molecular probe is a compound with a structure represented by following general formula (I), or a pharmaceutically acceptable salt or ester thereof:
Abₘ-(L-M_{z})ₙ (I)
wherein Ab is the anti-GPC3 antibody or antigen binding fragment thereof; L is the linking group; M is the radionuclide; z is 0 or 1; n is an integer from 1 to 4, preferably 1 or 2; m is an integer from 1 to 4, preferably 1 or 2.

3. The molecular probe targeting GPC3 of claim 2, wherein L is represented by following general formula (II):
Y-Lk-T (II)
wherein Y is a linker, Lk is a coupling group, and T is a chelating moiety;
preferably, Y comprises a reactive moiety and a spacer group, wherein the spacer group is independently selected from a single bond, an optionally substituted C₁-C₂₀ alkylene group, an optionally substituted C₁-C₂₀ alkenylene group, an optionally substituted C₁-C₂₀ alkynylene group, an optionally substituted C₁-C₂₀ heteroalkylene group, an optionally substituted C₃-C₂₀ cycloalkylene group, an optionally substituted C₃-C₂₀ heterocycloalkylene group, an optionally substituted C₅-C₂₀ arylene group, an optionally substituted C₅-C₂₀ heteroarylene group, ketone group, -S-, -(C=S)-, -O-, -NH-, -NHCO-, polypeptides (e.g., valine-citrulline (Val-Cit), valine-alanine (Val-Ala), glycine-glycine-phenylalanine-glycine (Gly-Gly-Phe-Gly), phenylalanine-lysine (Phe-Lys), alanine-alanine-asparagine (Ala-Ala-Asn), polyglycine (Gly)ᵣ, r being an integer from 1 to 10), polyethylene glycol (such as (PEG)ᵣ, r being an integer from 1 to 10), polypropylene glycol, polyoxyalkylene, copolymerization groups of polyethylene glycol and polypropylene glycol, or a combination thereof; the reactive moiety is linked to Ab and is independently selected from a single bond, p-isothiocyanate phenyl, N-hydroxysuccinimide ester, maleimidyl, 2,3,5,6-tetrafluorophenol ester, N-succinimidyl-S-acetylthioacetate, amido group, or a combination thereof;
preferably, Lk is independently selected from a single bond, p-isothiocyanate phenyl, N-hydroxysuccinimide ester, maleimidyl, 2,3,5,6-tetrafluorophenol ester, N-succinimidyl-S-acetylthioacetate, amido group, or a combination thereof;
preferably, T is independently selected from a single bond or a ligand derived from any of following structures: deferoxamine (DFO), 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), 6-hydrazinonicotinic acid (HYNIC), 4-[[[(1R,2R)-2-[bis(carboxymethyl)amino]cyclohexyl] (carboxymethyl)amino]methyl]-phenylacetic acid (H3RESCA), ethylenediaminetetraacetic acid (EDTA), 1,4,7,10-tetraazacyclododecane-1,4,7,10-tetrakis (methylene phosphonic acid) (DOTP), 1,4,7- triazacyclononane-1,4,7-tris (methylene phosphonic acid) (NOTP), 1,4,8,11-tetraazacyclotetradecane-1,4,8,11-tetraacetic acid (TETA), 1,4,7,10-tetrakis(carbamoylmethyl)-1,4,7,10-tetraazacyclododecane (DOTAM), triacetylfusarine C (TAFC), ferrochloroquine E (FOXE), ferrochloroquine B (FOXB), Ferrichrome A (FCHA), or a combination thereof;
optionally, a spacer group is further comprised between Lk and T, wherein the spacer group is independently selected from a single bond, an optionally substituted C₁-C₂₀ alkylene group, an optionally substituted C₁-C₂₀ alkenylene group, an optionally substituted C₁-C₂₀ alkynylene group, an optionally substituted C₁-C₂₀ heteroalkylene group, an optionally substituted C₃-C₂₀ cycloalkylene group, an optionally substituted C₃-C₂₀ heterocycloalkylene group, an optionally substituted C₅-C₂₀ arylene group, an optionally substituted C₅-C₂₀ heteroarylene group, ketone group, -S-, -(C=S)-, -O-, -NH-, -NHCO-, polypeptides (e.g., valine-citrulline (Val-Cit), valine-alanine (Val-Ala), glycine-glycine-phenylalanine-glycine (Gly-Gly-Phe-Gly), phenylalanine-lysine (Phe-Lys), alanine-alanine-asparagine (Ala-Ala-Asn), polyglycine (Gly)ᵣ, r being an integer from 1 to 10), polyethylene glycol ((PEG)ᵣ, r being an integer from 1 to 10), polypropylene glycol, polyoxyalkylene, copolymerization groups of polyethylene glycol and polypropylene glycol, or a combination thereof.

4. The molecular probe targeting GPC3 of claim 3, wherein
Y comprises a reactive moiety and a spacer group, wherein the spacer group is independently selected from a single bond, an optionally substituted C₁-C₁₀ alkylene group, an optionally substituted C₁-C₁₀ alkenylene group, an optionally substituted C₁-C₁₀ alkynylene group, an optionally substituted C₁-C₁₀ heteroalkylene group, an optionally substituted C₃-C₁₀ cycloalkylene group, an optionally substituted C₃-C₁₀ heterocycloalkylene group, an optionally substituted C₃-C₁₀ arylene group, an optionally substituted C₃-C₁₀ heteroarylene group, ketone group, -S-, -(C=S)-, -O-, -NH-, -NHCO-, -(PEG)ᵣ-, valine-citrulline (Val-Cit), valine-alanine (Val-Ala), glycine-glycine-phenylalanine-glycine (Gly-Gly-Phe-Gly), phenylalanine-lysine (Phe-Lys), alanine-alanine-asparagine (Ala-Ala-Asn), -(Gly)ₙ-, or a combination thereof, n being an integer from 1 to 10; the reactive moiety is linked to Ab and is independently selected from a single bond, p-isothiocyanate phenyl, maleimidyl, amido group, or a combination thereof;
Lk is independently selected from a single bond, p-isothiocyanate phenyl, amido group or maleimidyl; and
T is independently selected from a single bond or a ligand derived from any of following structures: deferoxamine (DFO), 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), 6-hydrazinonicotinic acid (HYNIC), 4-[[[(1R,2R)-2-[bis(carboxymethyl)amino]cyclohexyl] (carboxymethyl)amino]methyl]-phenylacetic acid (H3RESCA), 1,4,7,10-tetraazacyclododecane-1,4,7,10-tetrakis (methylene phosphonic acid) (DOTP), or 1,4,7- triazacyclononane-1,4,7-tris (methylene phosphonic acid) (NOTP);
optionally, a spacer group is further comprised between Lk and T, wherein the spacer group is independently selected from a single bond, an optionally substituted C₁-C₁₀ alkylene group, an optionally substituted C₁-C₁₀ alkenylene group, an optionally substituted C₁-C₁₀ alkynylene group, an optionally substituted C₁-C₁₀ heteroalkylene group, an optionally substituted C₃-C₁₀ cycloalkylene group, an optionally substituted C₃-C₁₀ heterocycloalkylene group, an optionally substituted C₃-C₁₀ arylene group, an optionally substituted C₃-C₁₀ heteroarylene group, ketone group, -S-, -(C=S)-, -O-, -NH-, -NHCO-, -(PEG)ᵣ-, valine-citrulline (Val-Cit), valine-alanine (Val-Ala), glycine-glycine-phenylalanine-glycine (Gly-Gly-Phe-Gly), phenylalanine-lysine (Phe-Lys), alanine-alanine-asparagine (Ala-Ala-Asn), -(Gly)n, or a combination thereof, wherein n is an integer from 1 to 10.

5. The molecular probe targeting GPC3 of claim 3 or 4, wherein
Y comprises the reactive moiety and the spacer group, wherein the spacer group is independently selected from a single bond, -(PEG)ᵣ-, -(Gly)ᵣ-, or a combination thereof, wherein r is an integer from 1 to 10; the reactive moiety is linked to Ab and is independently selected from a single bond, p-isothiocyanate phenyl, maleimidyl, amido group, or a combination thereof;
Lk is independently selected from a single bond, p-isothiocyanate phenyl, or amido group, and
T is independently selected from a single bond or a ligand derived from any of following structures: 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), 6-hydrazinonicotinic acid (HYNIC), or 4-[[[(1R,2R)-2-[bis(carboxymethyl)amino]cyclohexyl] (carboxymethyl)amino]methyl]-phenylacetic acid (H3RESCA);
optionally, a spacer group is further comprised between Lk and T, wherein the spacer group is independently selected from a single bond, -(PEG)ᵣ-, -(Gly)ᵣ-, or combinations thereof, wherein r is an integer from 1 to 10.

6. The molecular probe targeting GPC3 of any one of claims 3-5, wherein
Lk is independently selected from p-isothiocyanate phenyl, or amido group,
Y is a single bond, and
T is independently selected from a ligand derived from any of following structures: 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), 6-hydrazinonicotinic acid (HYNIC), or 4-[[[(1R,2R)-2-[bis(carboxymethyl)amino]cyclohexyl] (carboxymethyl)amino]methyl]-phenylacetic acid (H3RESCA).

7. The molecular probe targeting GPC3 of any one of claims 3-6, wherein
Lk is p-isothiocyanate phenyl;
Y comprises the reactive moiety and the spacer group, wherein the spacer group is independently selected from a single bond, -(PEG)ᵣ-, -(Gly)ᵣ-, or a combination thereof, wherein r is an integer from 1 to 10; the reactive moiety is linked to Ab and is independently selected from a single bond, p-isothiocyanate phenyl, maleimidyl, amido group, or a combination thereof; and
T is independently selected from a ligand derived from any of following structures: 1,4,7,10-tetraazacyclododecane-1,4,7,10-tetracarboxylic acid (DOTA), 1,4,7-triazacyclononane-1,4,7-tricarboxylic acid (NOTA), or diethylenetriamine pentaacetic acid (DTPA).

8. The molecular probe targeting GPC3 of any one of claims 3-6, wherein
Lk is p-isothiocyanate phenyl,
Y is a single bond, and
T is independently selected from a ligand derived from any of following structures: 1,4,7,10-tetraazacyclododecane-1,4,7,10-tetracarboxylic acid (DOTA), 1,4,7-triazacyclononane-1,4,7-tricarboxylic acid (NOTA), or diethylenetriamine pentaacetic acid (DTPA).

9. The molecular probe targeting GPC3 of any one of claims 3-6, wherein
Lk is amido group;
Y comprises the reactive moiety and the spacer group, wherein the spacer group is independently selected from a single bond, -(PEG)ᵣ-, -(Gly)ᵣ-, or a combination thereof, wherein r is an integer from 1 to 10; the reactive moiety is linked to Ab and is independently selected from a single bond, p-isothiocyanate phenyl, maleimidyl, amido group, or a combination thereof;
T is independently selected from a ligand derived from any of following structures: mercaptoacetyl triglycine (MAG3), or 4-[[[(1R,2R)-2-[bis(carboxymethyl)amino]cyclohexyl] (carboxymethyl)amino]methyl]-phenylacetic acid (H3RESCA).

10. The molecular probe targeting GPC3 of any one of claims 3-6, wherein
Lk is amido group;
Y is a single bond; and
T is independently selected from a ligand derived from any of following structures: mercaptoacetyl triglycine (MAG3), or 4-[[[(1R,2R)-2-[bis(carboxymethyl)amino]cyclohexyl] (carboxymethyl)amino]methyl]-phenylacetic acid (H3RESCA).

11. The molecular probe targeting GPC3 of any one of claims 2-10, wherein L is independently selected from a ligand derived from any one of following structures: 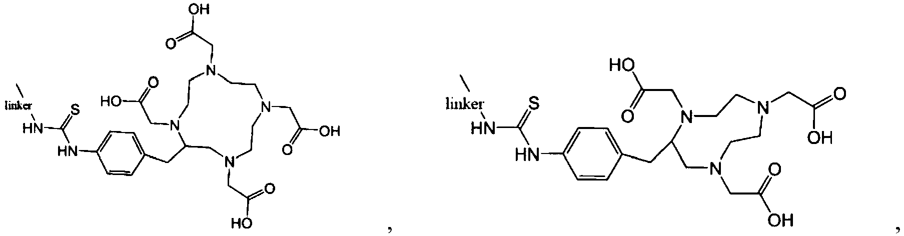 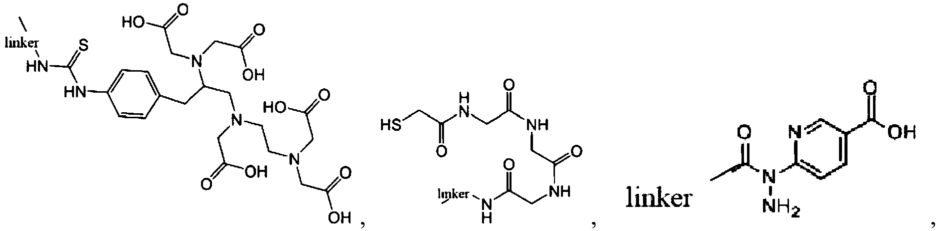 or 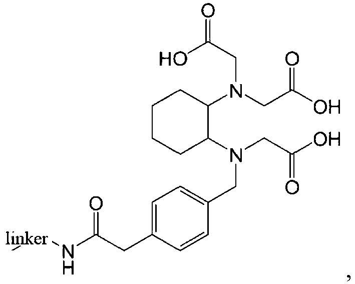 wherein the linker comprises a reactive moiety and a spacer group, and the spacer group is independently selected from a single bond, -(PEG)ᵣ-, -(Gly)ᵣ-, or a combination thereof, r being an integer from 1 to 10; the reactive moiety is linked to Ab and is independently selected from a single bond, p-isothiocyanate phenyl, maleimidyl, amido group, or a combination thereof; preferably, the linker is a single bond.

12. The molecular probe targeting GPC3 of any one of preceding claims, wherein M is a nuclide that emits β or α particles, a positron-emitting nuclide, a single-photon-emitting nuclide, or a paramagnetic metal; preferably, M is selected from a diagnostic radionuclide or a therapeutic radionuclide;
preferably, the diagnostic radionuclide is selected from ⁶⁸Ga, ¹⁸F-Al, ¹⁴Cu, ^{99m}Tc, ⁸⁹Zr, ⁴⁴Sc, ¹¹¹In, ⁴⁵Ti, ⁵²Mn, ⁵⁹Fe, ^{94m}Tc, ⁶⁷Ga, ⁷¹As, ⁷²As, ^{82m}Rb, ⁸⁶Y, ¹³¹I, Gd, Mn, or a combination thereof;
preferably, the therapeutic radionuclide is selected from ¹⁷⁷Lu, ⁹⁰Y, ¹³¹I, ¹⁵³Sm, ⁶⁷Cu, ⁸⁹Sr, ¹⁶⁶Ho, ¹⁷⁷Yb, ⁴⁷Sc, ¹⁸⁶Re, ¹⁸⁸Re, ²¹²Bi, ²¹³Bi, ¹⁴⁹Pm, ²¹²Pb, ²¹¹At, ²²³Ra, ²²⁵Ac, ²²⁷Th, or a combination thereof;
more preferably, M is selected from ⁶⁸Ga, ¹⁸F-Al, ^{99m}Tc, ¹⁷⁷Lu, ¹³¹I, or a combination thereof.

13. The molecular probe targeting GPC3 of any one of preceding claims, wherein L-M is selected from following structures: 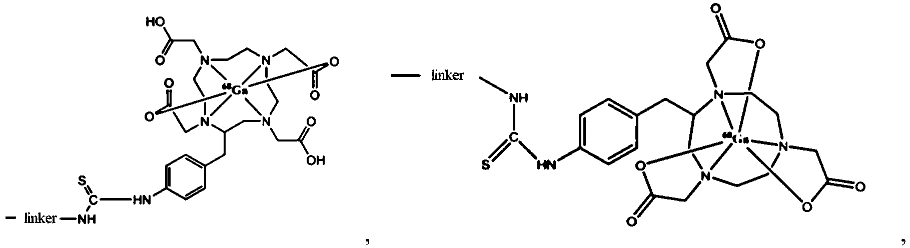 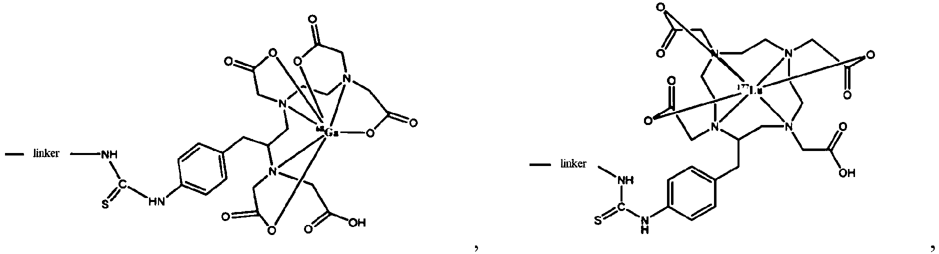 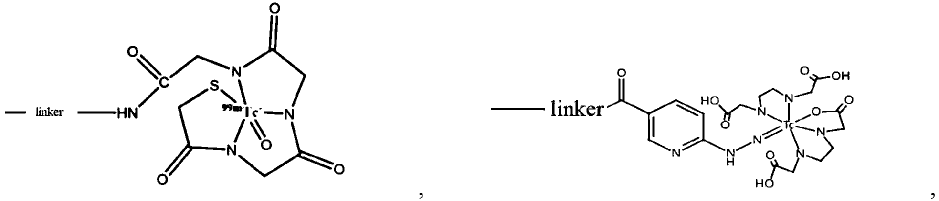 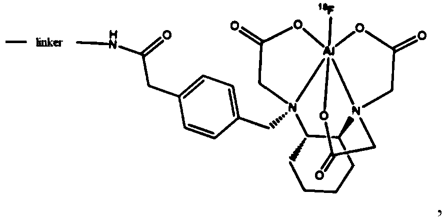 or -linker-¹³¹I,
wherein the linker comprises a reactive moiety and a spacer group, and the spacer group is independently selected from a single bond, -(PEG)ᵣ-, -(Gly)ᵣ-, or a combination thereof, r being an integer from 1 to 10; the reactive moiety is linked to Ab and is independently selected from a single bond, p-isothiocyanate phenyl, maleimidyl, amido group, or a combination thereof;
preferably, the linker is a single bond.

14. The molecular probe targeting GPC3 of any one of preceding claims, wherein Ab is the anti-GPC3 antibody or antigen binding fragment thereof; preferably, Ab is a monoclonal antibody, polyclonal antibody, single-chain antibody, chimeric antibody, humanized antibody, or fully human antibody; preferably, Ab is selected from a monoclonal antibody (mAb), Fab, Fab', F(ab')₂, Fv, scFv, scFv-Fc, or single-domain antibody.

15. The molecular probe targeting GPC3 of any one of preceding claims, wherein Ab contains a heavy chain or fragment thereof, and the heavy chain or fragment thereof contains HCDR1, HCDR2, and HCDR3,
HCDR1 contains an amino acid sequence having at least 80% sequence identity to an amino acid sequence shown as SEQ ID NO: 1, preferably the amino acid sequence of HCDR1 is an amino acid sequence having at least 90% sequence identity to the amino acid sequence shown as SEQ ID NO: 1, more preferably at least 95% sequence identity to the amino acid sequence shown as SEQ ID NO: 1, most preferably the amino acid sequence of HCDR1 is shown as SEQ ID NO: 1;
HCDR2 contains an amino acid sequence having at least 80% sequence identity to an amino acid sequence shown as SEQ ID NO: 2, preferably the amino acid sequence of HCDR2 is an amino acid sequence having at least 90% sequence identity to the amino acid sequence shown as SEQ ID NO: 2, more preferably at least 95% sequence identity to the amino acid sequence shown as SEQ ID NO: 2, most preferably the amino acid sequence of HCDR2 is shown as SEQ ID NO: 2;
HCDR3 contains an amino acid sequence having at least 80% sequence identity to an amino acid sequence shown as SEQ ID NO: 3, preferably the amino acid sequence of HCDR3 is an amino acid sequence having at least 90% sequence identity to the amino acid sequence shown as SEQ ID NO: 3, more preferably at least 95% sequence identity to the amino acid sequence shown as SEQ ID NO: 3, most preferably the amino acid sequence of HCDR3 is shown as SEQ ID NO: 3.

16. The molecular probe targeting GPC3 of claim 15, wherein Ab contains a heavy chain variable region (VH), and the VH contains an amino acid sequence having at least 80%, preferably at least 90%, more preferably at least 95% sequence identity to an amino acid sequence shown as SEQ ID NO: 7, most preferably the amino acid sequence of the VH is shown as SEQ ID NO: 7.

17. The molecular probe targeting GPC3 of claim 16, wherein Ab contains the heavy chain, and the heavy chain contains an amino acid sequence having at least 80%, preferably at least 90%, more preferably at least 95% sequence identity to an amino acid sequence shown as SEQ ID NO: 9, most preferably the amino acid sequence of the heavy chain is shown as SEQ ID NO: 9.

18. The molecular probe targeting GPC3 of any one of preceding claims, wherein Ab contains a light chain or fragment thereof, and the light chain or fragment thereof contains LCDR1, LCDR2, and LCDR3,
LCDR1 contains an amino acid sequence having at least 80% sequence identity to an amino acid sequence shown as SEQ ID NO: 4, preferably the amino acid sequence of LCDR1 is an amino acid sequence having at least 90% sequence identity to the amino acid sequence shown as SEQ ID NO: 4, more preferably at least 95% sequence identity to the amino acid sequence shown as SEQ ID NO: 4, most preferably the amino acid sequence of LCDR1 is shown as SEQ ID NO: 4;
LCDR2 contains an amino acid sequence having at least 80% sequence identity to an amino acid sequence shown as SEQ ID NO: 5, preferably the amino acid sequence of LCDR2 is an amino acid sequence having at least 90% sequence identity to the amino acid sequence shown as SEQ ID NO: 5, more preferably at least 95% sequence identity to the amino acid sequence shown as SEQ ID NO: 5, most preferably the amino acid sequence of LCDR2 is shown as SEQ ID NO: 5;
LCDR3 contains an amino acid sequence having at least 80% sequence identity to an amino acid sequence shown as SEQ ID NO: 6, preferably the amino acid sequence of LCDR3 is an amino acid sequence having at least 90% sequence identity to the amino acid sequence shown as SEQ ID NO: 6, more preferably at least 95% sequence identity to the amino acid sequence shown as SEQ ID NO: 6, most preferably the amino acid sequence of LCDR3 is shown as SEQ ID NO: 6.

19. The molecular probe targeting GPC3 of claim 18, wherein Ab contains a light chain variable region (VL), and the VL contains an amino acid sequence having at least 80%, preferably at least 90%, more preferably at least 95% sequence identity to an amino acid sequence shown as SEQ ID NO: 8, most preferably, the amino acid sequence of VL is shown as SEQ ID NO: 8.

20. The molecular probe targeting GPC3 of claim 19, wherein Ab contains a light chain, and the light chain contains an amino acid sequence having at least 80%, preferably at least 90%, more preferably at least 95% sequence identity to an amino acid sequence shown as SEQ ID NO: 10, most preferably, the amino acid sequence of the light chain is shown as SEQ ID NO: 10.

21. The molecular probe targeting GPC3 of any one of preceding claims, wherein Ab is Fab, Fab', or F(ab')₂ fragment, preferably Fab fragment,
preferably, Ab contains a heavy chain with an amino acid sequence having at least 80%, preferably at least 90%, more preferably at least 95% to an amino acid sequence shown as SEQ ID NO: 11, most preferably the amino acid sequence shown as SEQ ID NO: 11; and
preferably, Ab contains a light chain with an amino acid sequence having at least 80%, preferably at least 90%, more preferably at least 95% to the amino acid sequence shown as SEQ ID NO: 10, most preferably the amino acid sequence shown as SEQ ID NO: 10.

22. The molecular probe targeting GPC3 of any one of claims 1-20, wherein Ab is scFv fragment,
preferably, Ab contains an amino acid sequence having at least 80%, preferably at least 90%, more preferably at least 95% sequence identity to an amino acid sequence shown as SEQ ID NO: 12, most preferably the amino acid sequence shown as SEQ ID NO: 12.

23. The molecular probe targeting GPC3 of any one of preceding claims, wherein the molecular probe is selected from following structures:
| | | |
|---|---|---|
| 1. ⁶⁸Ga-DOTA-aGPC3-mAb | 2. ⁶⁸Ga-DOTA-aGPC3-Fab | 3. ⁶⁸Ga-DOTA-aGPC3-scFv |
| 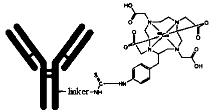 | 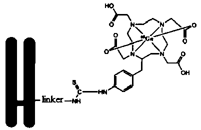 | 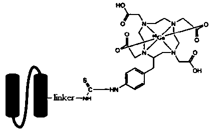 |
| 4. ⁶⁸Ga-NOTA-aGPC3-mAb | 5. ⁶⁸Ga-NOTA-aGPC3-Fab | 6. ⁶⁸Ga-NOTA-aGPC3-scFv |
| 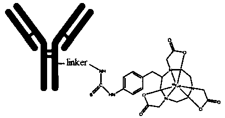 | 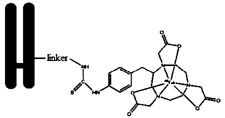 | 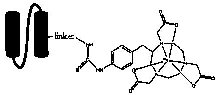 |
| 7. ⁶⁸Ga-DOTA-aGPC3-mAb | 8. ⁶⁸Ga-DOTA-aGPC3-Fab | 9. ⁶⁸Ga-DOTA-aGPC3-scFv |
| 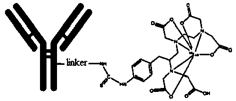 | 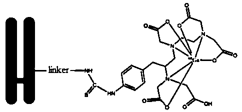 | 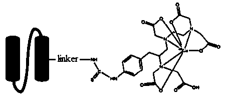 |
| 10. ¹⁷⁷Lu-DOTA-aGPC3-mAb | 11. ¹⁷⁷Lu-DOTA-aGPC3-Fab | 12. ¹⁷⁷Lu-DOTA-aGPC3-scFv |
| 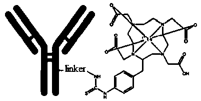 | 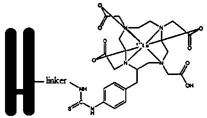 | 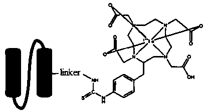 |
| 13. ^{99m}TC-MAG3-aGPC3-mAb | 14. ^{99m}TC-MAG3-aGPC3-Fab | 15. ^{99m}TC-MAG3-aGPC3-scFv |
| 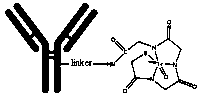 | 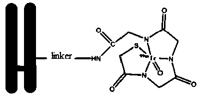 | 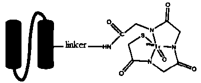 |
| 16. Al¹⁸F-RESCA-aGPC3-mAb | 17. Al¹⁸F-RESCA-aGPC3-Fab | 18. Al¹⁸F-RESCA-aGPC3-scFv |
| 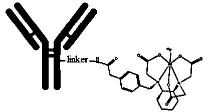 | 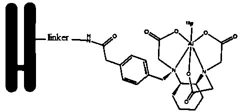 | 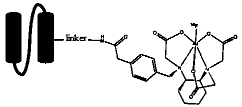 |
| 19. ¹³¹I-aGPC3-mAb | 20. ¹³¹I-aGPC3-Fab | 21. ¹³¹I-aGPC3-scFv |
| 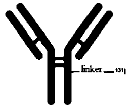 | 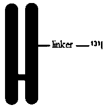 | 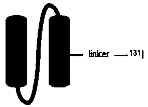 |
| 22. ^{99m}TC-HYNIC-aGPC3-mAb | 23. ^{99m}TC-HYNIC-aGPC3-Fab | 24. ^{99m}TC-HYNIC-aGPC3-scFv |
| 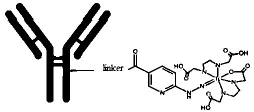 | 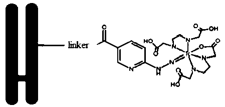 | 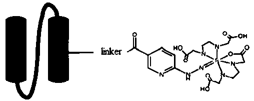 |
wherein the linker comprises a reactive moiety and a spacer group, and the spacer group is independently selected from a single bond, -(PEG)ᵣ-, -(Gly)ᵣ-, or a combination thereof, r being an integer from 1 to 10; the reactive moiety is linked to Ab and is independently selected from a single bond, p-isothiocyanate phenyl, maleimidyl, amido group, or a combination thereof; preferably, the linker is a single bond.

24. An antibody conjugate having a structure represented by following general formula (III):
Abₘ- L'ₙ (III)
wherein L' is represented by following general formula (IV):
Y-Lk-T' (IV)
wherein optionally, a spacer group is further comprised between Lk and T',
wherein Ab, Y, Lk, the spacer group, m, and n are defined in accordance with any one of claims 1-21, and
T' is independently selected from a single bond, deferoxamine (DFO), 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), 4-[[[(1R,2R)-2-[bis(carboxymethyl)amino]cyclohexyl] (carboxymethyl)amino]methyl]-phenylacetic acid (H3RESCA), ethylenediaminetetraacetic acid (EDTA), 1,4,7,10-tetraazacyclododecane-1,4,7,10-tetrakis (methylene phosphonic acid) (DOTP), 1,4,7- triazacyclononane-1,4,7-tris (methylene phosphonic acid) (NOTP), 1,4,8,11-tetraazacyclotetradecane-1,4,8,11-tetraacetic acid (TETA), 1,4,7,10-tetrakis(carbamoylmethyl)-1,4,7,10-tetraazacyclododecane (DOTAM), triacetylfusarine C (TAFC), ferrochloroquine E (FOXE), ferrochloroquine B (FOXB), Ferrichrome A (FCHA), or a combination thereof;
preferably, T' is independently selected from a single bond, deferoxamine (DFO), 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), 6-hydrazinonicotinic acid (HYNIC), 4-[[[(1R,2R)-2-[bis(carboxymethyl)amino]cyclohexyl] (carboxymethyl)amino]methyl]-phenylacetic acid (H3RESCA), 1,4,7,10-tetraazacyclododecane-1,4,7,10-tetrakis (methylene phosphonic acid) (DOTP), or 1,4,7- triazacyclononane-1,4,7-tris (methylene phosphonic acid) (NOTP);
more preferably, T' is independently selected from a single bond, 1,4,7,10-tetraazacyclododecane-1,4,7,10-tetracarboxylic acid (DOTA), 1,4,7-triazacyclononane-1,4,7-tricarboxylic acid (NOTA), diethylenetriamine pentaacetic acid (DTPA), mercaptoacetyl triglycine (MAG3), or 4-[[[(1R,2R)-2-[bis(carboxymethyl)amino]cyclohexyl] (carboxymethyl)amino]methyl]-phenylacetic acid (H3RESCA).

25. A pharmaceutical composition comprising: the molecular probe targeting GPC3 of any one of claims 1-23 or the antibody conjugate of claim 24; and a pharmaceutically acceptable carrier.

26. A kit comprising: the antibody conjugate of claim 24; and radionuclide M, wherein M is defined in accordance with any one of claims 1-23.

27. A method for preparing the molecular probe targeting GPC3 of anyone of claims 1-23, comprising: reacting the antibody conjugate of claim 24 with radionuclide M.

28. Use of the molecular probe targeting GPC3 of anyone of claims 1-23, the antibody conjugate of claim 24, the pharmaceutical composition of claim 25, or the kit of claim 26 in the manufacture of a reagent for detecting of GPC3.

29. Use of the molecular probe targeting GPC3 of anyone of claims 1-23, the antibody conjugate of claim 24, the pharmaceutical composition of claim 25, or the kit of claim 26 in the manufacture of a kit for molecular imaging.

30. A method for imaging, comprising:
administering to a subject in need thereof the molecular probe targeting GPC3 of any one of claims 1-23 or the pharmaceutical composition of claim 24; and
detecting presence of the molecular probe targeting GPC3 *in vivo* through imaging.

31. The method of claim 30, wherein the presence of the molecular probe targeting GPC3 is detected through single photon emission computed tomography (SPECT), positron emission tomography (PET) imaging, or magnetic resonance imaging (MRI).

32. Use of the molecular probe targeting GPC3 of anyone of claims 1-23, the antibody conjugate of claim 24, the pharmaceutical composition of claim 25, or the kit of claim 26 in the manufacture of a kit for diagnosing a cancer.

33. Use of the molecular probe targeting GPC3 of anyone of claims 1-23, the antibody conjugate of claim 24, the pharmaceutical composition of claim 25, or the kit of claim 26 in the manufacture of a reagent for treating a cancer.

34. A method for treating a cancer, comprising: administering to a subject in need thereof the molecular probe targeting GPC3 of any one of claims 1-23 or the pharmaceutical composition of claim 25.

35. The method of claim 34, wherein the subject is GPC3-positive.

36. The method of any one of claims 32-34, wherein the cancer is a cancer with high GPC3 expression, preferably a solid tumor, more preferably selected from liver cancer, non-small cell lung cancer, melanoma, ovarian yolk sac tumor, ovarian clear cell carcinoma, and colorectal cancer, and most preferably liver cancer.
